# EUROPEAN PATENT SPECIFICATION

(11) **EP 1 687 443 B1**
(45) Date of publication and mention of the grant of the patent: **03.11.2010**
(21) Application number: 04765664.0
(22) Date of filing: 28.09.2004
(51) Int. Cl.: C12Q 1/68

(54) **FKBP51: A NOVEL TARGET FOR ANTIDEPRESSANT THERAPY**
FKBP51: EIN NEUES ZIEL FÜR DIE THERAPIE MIT ANTIDEPRESSIVA
FKBP51: CIBLE DESTINEE A UN TRAITEMENT ANTIDEPRESSEUR

(30) Priority: 25.11.2003 EP 03026953
(43) Date of publication of application: 09.08.2006
(73) Proprietor: Max-Planck-Gesellschaft zur Förderung der Wissenschaften e.V., Berlin (DE)
(72) Inventor: BINDER, Elisabeth, 80469 München (DE); HOLSBOER, Florian, 80805 München (DE); REIN, Theo, 80995 München (DE); WOCHNIK, Gabriele, 86926 Greifenberg (DE); MÜLLER-MYHSOK, Bertram, 80689 München (DE); UHR, Manfred, 82131 Stockdork (DE)
(74) Representative: Vossius & Partner
(86) International application number: PCT/EP2004/010857
(87) International publication number: WO 2005/054500

(56) References cited:
- WO-A-03/082210
- WO-A-2004/039837
- BAUGHMAN GAIL ET AL: "Tissue distribution and abundance of human FKBP51, an FK506-binding protein that can mediate calcineurin inhibition" BIOCHEMICAL AND BIOPHYSICAL RESEARCH COMMUNICATIONS, vol. 232, no. 2, 1997, pages 437-443, XP002312737 ISSN: 0006-291X
- DATABASE GENEBANK 10 July 2001 (2001-07-10), ASHWELL R: "Homo sapiens chromosome 6 clone RP11-282I23, 2 unordered pieces" XP002312801 Database accession no. AL590962
- DATABASE GENEBANK 10 July 2001 (2001-07-10), CLARK: "Homo sapiens chromosome 6 clone RP11-238F24, 3 unordered pieces" XP002312802 Database accession no. AL512371
- YU Y W -Y ET AL: "Association study of the serotonin transporter promoter polymorphism and symptomatology and antidepressant response in major depressive disorders." MOLECULAR PSYCHIATRY, vol. 7, no. 10, 2002, pages 1115-1119, XP008041266 ISSN: 1359-4184
- TEARE J M ET AL: "Measurement of nucleic acid concentrations using the DyNA Quant and the GeneQuant" BIOTECHNIQUES, vol. 22, no. 6, 1997, pages 1170-1174, XP008041123 ISSN: 0736-6205

### SEQUENCE LISTING

<110> Max-Planck-Gesellschaft zur Forderung der Wissenschaften e.V.
<120> FKBP51 : A Novel Target for Antidepressant Therapy
<130> H 25.89 PCT
<141> 2004-09-28
<150> EP 03 02 6953.4
   <151> 2003-11-25
<160> 155
<170> PatentIn version 3.2
<210> 1
   <211> 30
   <212> DNA
   <213> Artificial
<220>
   <221> source
   <223> /note="Description of artificial sequence: oligonucleotide"
<400> 1
   acgttggatg tatctggcaa ccctaacctc 30
<210> 2
   <211> 30
   <212> DNA
   <213> Artificial
<220>
   <221> source
   <223> /note="Description of artificial sequence: oligonucleotide"
<400> 2
   acgttggatg cctaacgaga tagtgaggag 30
<210> 3
   <211> 30
   <212> DNA
   <213> Artificial
<220>
   <221> source
   <223> /note="Description of artificial sequence: oligonucleotide"
<400> 3
   acgttggatg aacaccaaga gaagcagctc 30
<210> 4
   <211> 30
   <212> DNA
   <213> Artificial
<220>
   <221> source
   <223> /note="Description of artificial sequence: oligonucleotide"
<400> 4
   acgttggatg ctatccacct cctccataag 30
<210> 5
   <211> 30
   <212> DNA
   <213> Artificial
<220>
   <221> source
   <223> /note="Description of artificial sequence: oligonucleotide"
<400> 5
   acgttggatg aagagatcca ggcacagaag 30
<210> 6 <211> 30
   <212> DNA
   <213> Artificial
<220>
   <221> source
   <223> /note="Description of artificial sequence: oligonucleotide"
<400> 6
   acgttggatg tgccagcagt agcaagtaag 30
<210> 7
   <211> 30
   <212> DNA
   <213> Artificial
<220>
   <221> source
   <223> /note="Description of artificial sequence: oligonucleotide"
<400> 7
   acgttggatg aaacccctag tgtagaagag 30
<210> 8
   <211> 30
   <212> DNA
   <213> Artificial
<220>
   <221> source
   <223> /note="Description of artificial sequence: oligonucleotide"
<400> 8
   acgttggatg tttacactcc tctatcatgc 30
<210> 9
   <211> 19
   <212> DNA
   <213> Artificial
<220>
   <221> source
   <223> /note="Description of arti'ficial sequence: oligonucleotide"
<400> 9
   gactcctaca ttttcctct 19
<210> 10
   <211> 18
   <212> DNA
   <213> Artificial
<220>
   <221> source
   <223> /note="Description of artificial sequence: oligonucleotide"
<400> 10
   gaagcagctc cctttaga 18
<210> 11
   <211> 22
   <212> DNA
   <213> Artificial
<220>
   <221> source
   <223> /note="Description of artificial sequence: oligonucleotide"
<400> 11
   ggctttcaca taagcaaagt ta 22
<210> 12
   <211> 24
   <212> DNA
   <213> Artificial
<220>
   <221> source
   <223> /note="Description of artificial sequence: oligonucleotide"
<400> 12
   aagagcaact atttatttgt caac 24
<210> 13
   <211> 21
   <212> DNA
   <213> Artificial
<220>
   <221> source
   <223> /note="Description of artificial sequence: oligonucleotide"
<400> 13
   attttcctct atcttggtcc a 21
<210> 14
   <211> 21
   <212> DNA
   <213> Artificial
<220>
   <221> source
   <223> /note="Description of artificial sequence: oligonucleotide"
<400> 14
   attttcctct gtcttggtcc a 21
<210> 15
   <211> 21
   <212> DNA
   <213> Artificial
<220>
   <221> source
   <223> /note="Description of artificial sequence: oligonucleotide"
<400> 15
   taaagtaatt ctctaaaggg a 21
<210> 16
   <211> 21
   <212> DNA
   <213> Artificial
<220>
   <221> source
   <223> /note="Description of artificial sequence: oligonucleotide"
<400> 16
   taaagtaatt ttctaaaggg a 21
<210> 17
   <211> 21
   <212> DNA
   <213> Artificial
<220>
   <221> source
   <223> /note="Description of artificial sequence: oligonucleotide"
<400> 17
   agcaaagtta tacaaaacaa a 21
<210> 18
   <211> 21
   <212> DNA
   <213> Artificial
<220>
   <221> source
   <223> /note="Description of artificial sequence: oligonucleotide"
<400> 18
   agcaaagtta cacaaaacaa a 21
<210> 19
   <211> 21
   <212> DNA
   <213> Artificial
<220>
   <221> source
   <223> /note="Description of artificial sequence: oligonucleotide"
<400> 19
   atttgtcaac cctacagatt t 21
<210> 20
   <211> 21
   <212> DNA
   <213> Artificial
<220>
   <221> source
   <223> /note="Description of artificial sequence: oligonucleotide"
<400> 20
   atttgtcaac actacagatt t 21
<210> 21
   <211> 30
   <212> DNA
   <213> Artificial
<220>
   <221> source
   <223> /note="Description of artificial sequence: oligonucleotide"
<400> 21
   acgttggatg tcgaagggac ttattcctcc 30
<210> 22
   <211> 30
   <212> DNA
   <213> Artificial
<220>
   <221> source
   <223> /note="Description of artificial sequence: oligonucleotide"
<400> 22
   acgttggatg cagcagaagg aagacatcag 30
<210> 23
   <211> 30
   <212> DNA
   <213> Artificial
<220>
   <221> source
   <223> /note="Description of artificial sequence: oligonucleotide"
<400> 23
   acgttggatg acacacggct catctgtaac 30
<210> 24
   <211> 30
   <212> DNA
   <213> Artificial
<220>
   <221> source
   <223> /note="Description of artificial sequence: oligonucleotide"
<400> 24
   acgttggatg atgccaggca tttgggtttc 30
<210> 25
   <211> 30
   <212> DNA
   <213> Artificial
<220>
   <221> source
   <223> /note="Description of artificial sequence: oligonucleotide"
<400> 25
   acgttggatg aagccctgtg gttttatgcc 30
<210> 26
   <211> 30
   <212> DNA
   <213> Artificial
<220>
   <221> source
   <223> /note="Description of artificial sequence: oligonucleotide"
<400> 26
   acgttggatg tggaacaatt ctgtccccag 30
<210> 27
   <211> 30
   <212> DNA
   <213> Artificial
<220>
   <221> source
   <223> /note="Description of artificial sequence: oligonucleotide"
<400> 27
   acgttggatg tttcctccct agattcccag 30
<210> 28
   <211> 30
   <212> DNA
   <213> Artificial
<220>
   <221> source
   <223> /note="Description of artificial sequence: oligonucleotide"
<400> 28
   acgttggatg gtcccagtgg aactttatgg 30
<210> 29
   <211> 30
   <212> DNA
   <213> Artificial
<220>
   <221> source
   <223> /note="Description of artificial sequence: oligonucleotide"
<400> 29
   acgttggatg acaagctatc gggctttctc 30
<210> 30
   <211> 30
   <212> DNA
   <213> Artificial
<220>
   <221> source
   <223> /note="Description of artificial sequence: oligonucleotide"
<400> 30
   acgttggatg agacaatctc ccagttgcag 30
<210> 31
   <211> 30
   <212> DNA
   <213> Artificial
<220>
   <221> source
   <223> /note="Description of artificial sequence: oligonucleotide"
<400> 31
   acgttggatg tctgaaatcc agctggacac 30
<210> 32
   <211> 30
   <212> DNA
   <213> Artificial
<220>
   <221> source
   <223> /note="Description of artificial sequence: oligonucleotide"
<400> 32
   acgttggatg agtgagactc tgtccaaaag 30
<210> 33
   <211> 30
   <212> DNA
   <213> Artificial
<220>
   <221> source
   <223> /note="Description of artificial sequence: oligonucleotide"
<400> 33
   acgttggatg ttgagaccag atgttggagg 30
<210> 34
   <211> 30
   <212> DNA
   <213> Artificial
<220>
   <221> source
   <223> /note="Description of artificial sequence: oligonucleotide"
<400> 34
   acgttggatg gacagagtct ggctctgttg 30
<210> 35
   <211> 30
   <212> DNA
   <213> Artificial
<220>
   <221> source
   <223> /note="Description of artificial sequence: oligonucleotide"
<400> 35
   acgttggatg tacagaccct gcaaagaacc 30
<210> 36
   <211> 30
   <212> DNA
   <213> Artificial
<220>
   <221> source
   <223> /note="Description.of artificial sequence: oligonucleotide"
<400> 36
   acgttggatg tgcgctatgg ctgcagatac 30
<210> 37
   <211> 30
   <212> DNA
   <213> Artificial
<220>
   <221> source
   <223> /note="Description of artificial sequence: oligonucleotide"
<400> 37
   acgttggatg tacagaccct gcaaagaacc 30
<210> 38
   <211> 30
   <212> DNA
   <213> Artificial
<220>
   <221> source
   <223> /note="Description of artificial sequence: oligonucleotide"
<400> 38
   acgttggatg tgcgctatgg ctgcagatac 30
<210> 39
   <211> 30
   <212> DNA
   <213> Artificial
<220>
   <221> source
   <223> /note="Description of artificial sequence: oligonucleotide"
<400> 39
   acgttggatg aaacagggaa gcttctaggc 30
<210> 40
   <211> 30
   <212> DNA
   <213> Artificial
<220>
   <221> source
   <223> /note="Description of artificial sequence: oligonucleotide"
<400> 40
   acgttggatg aagccaagac tgaaaccgcc 30
<210> 41
   <211> 30
   <212> DNA
   <213> Artificial
<220>
   <221> source
   <223> /note="Description of artificial sequence: oligonucleotide"
<400> 41
   acgttggatg tattacaggc gtgagccatc 30
<210> 42
   <211> 30
   <212> DNA
   <213> Artificial
<220>
   <221> source
   <223> /note="Description of artificial sequence: oligonucleotide"
<400> 42
   acgttggatg atggtcttta gaggaattct 30
<210> 43.
   <211> 30
   <212> DNA
   <213> Artificial
<220>
   <221> source
   <223> /note="Description of artificial sequence: oligonucleotide"
<400> 43
   acgttggatg gacagagtga gactcctcta 30
<210> 44
   <211> 30
   <212> DNA
   <213> Artificial
<220>
   <221> source
   <223> /note="Description of artificial sequence: oligonucleotide"
<400> 44
   acgttggatg ttctcaaacc agcactcagg 30
<210> 45
   <211> 30
   <212> DNA
   <213> Artificial
<220>
   <221> source
   <223> /note="Description of artificial sequence: oligonucleotide"
<400> 45
   acgttggatg gggaaaagaa tcaagggaag 30
<210> 46
   <211> 30
   <212> DNA
   <213> Artificial
<220>
   <221> source
   <223> /note="Description of artificial sequence: oligonucleotide"
<400> 46
   acgttggatg ctggaactaa gtgatctgtg 30
<210> 47
   <211> 30
   <212> DNA
   <213> Artificial
<220>
   <221> source
   <223> /note="Description of artificial sequence: oligonucleotide"
<400> 47
   acgttggatg ggttaggtag agctcagttc 30
<210> 48
   <211> 30
   <212> DNA
   <213> Artificial
<220>
   <221> source
   <223> /note="Description of artificial sequence: oligonucleotide"
<400> 48
   acgttggatg gtagagaacc tggtaagaag 30
<210> 49 <211> 30
   <212> DNA
   <213> Artificial
<220>
   <221> source
   <223> /note="Description of artificial sequence: oligonucleotide"
<400> 49
   acgttggatg gtggcaaata ggagttctcc 30
<210> 50
   <211> 30
   <212> DNA
   <213> Artificial
<220>
   <221> source
   <223> /note="Description of artificial sequence: oligonucleotide"
<400> 50
   acgttggatg ttggcaggtg tttttctgag 30
<210> 51
   <211> 30
   <212> DNA
   <213> Artificial
<220>
   <221> source
   <223> /note="Description of artificial sequence: oligonucleotide"
<400> 51
   acgttggatg agaccacacc acagaacaag 30
<210> 52
   <211> 30
   <212> DNA
   <213> Artificial
<220>
   <221> source
   <223> /note="Description of artificial sequence: oligonucleotide"
<400> 52
   acgttggatg ttgcttaacc ctctccagac 30
<210> 53
   <211> 30
   <212> DNA
   <213> Artificial
<220>
   <221> source
   <223> /note="Description of artificial sequence: oligonucleotide"
<400> 53
   acgttggatg atatacatac aggctggccg 30
<210> 54
   <211> 30
   <212> DNA
   <213> Artificial
<220>
   <221> source
   <223> /note="Description of artificial sequence: oligonucleotide"
<400> 54
   acgttggatg actcctgacc tcgtgatctg 30
<210> 55.
   <211> 30
   <212> DNA
   <213> Artificial
<220>
   <221> source
   <223> /note="Description of artificial sequence: oligonucleotide"
<400> 55
   acgttggatg gatgactgat acttcagtct 30
<210> 56
   <211> 31
   <212> DNA
   <213> Artificial
<220>
   <221> source
   <223> /note="Description of artificial sequence: oligonucleotide"
<400> 56

   acgttggatg gaaagcaaag ctgaaaagta g 31
<210> 57
   <211> 30
   <212> DNA
   <213> Artificial
<220>
   <221> source
   <223> /note="Description of artificial sequence: oligonucleotide"
<400> 57
   acgttggatg ccactcttac attcctctcc 30
<210> 58
   <211> 30
   <212> DNA
   <213> Artificial
<220>
   <221> source
   <223> /note="Description of artificial sequence: oligonucleotide"
<400> 58
   acgttggatg tccctctcca aatctcactg 30
<210> 59
   <211> 30
   <212> DNA
   <213> Artificial
<220>
   <221> source
   <223> /note="Description of artificial sequence: oligonucleotide"
<400> 59
   acgttggatg agtggagcta taggagctag 30
<210> 60
   <211> 30
   <212> DNA
   <213> Artificial
<220>
   <221> source
   <223> /note="Description of artificial sequence: oligonucleotide"
<400> 60
   acgttggatg acggaaagac tgctgattgc 30
<210> 61
   <211> 30
   <212> DNA
   <213> Artificial
<220>
   <221> source
   <223> /note="Description of artificial sequence: oligonucleotide"
<400> 61
   acgttggatg tcgaagggac ttattcctcc 30
<210> 62
   <211> 30
   <212> DNA
   <213> Artificial
<220>
   <221> source
   <223> /note="Description of artificial sequence: oligonucleotide"
<400> 62
   acgttggatg cagcagaagg aagacatcag 30
<210> 63
   <211> 30
   <212> DNA
   <213> Artificial
<220>
   <221> source
   <223> /note="Description of artificial sequence: oligonucleotide"
<400> 63.
   acgttggatg agaataaacc tgtgtttctg 30
<210> 64
   <211> 30
   <212> DNA
   <213> Artificial
<220>
   <221> source
   <223> /note="Description of artificial sequence: oligonucleotide"
<400> 64
   acgttggatg aggagcaacc atatttctaa 30
<210> 65
   <211> 30
   <212> DNA
   <213> Artificial
<220>
   <221> source
   <223> /note="Description of artificial sequence: oligonucleotide"
<400> 65
   acgttggatg gggtgaaagt ggcagaacaa 30
<210> 66
   <211> 30
   <212> DNA
   <213> Artificial
<220>
   <221> source
   <223> /note="Description of artificial sequence: oligonucleotide"
<400> 66
   acgttggatg ccgctggcta atgaaaaaac 30
<210> 67
   <211> 30
   <212> DNA
   <213> Artificial
<220>
   <221> source
   <223w /note="Description of artificial sequence: oligonucleotide"
<400> 67
   acgttggatg agacgatgga cccattttac 30
<210> 68
   <211> 30
   <212> DNA
   <213> Artificial
<220>
   <221> source
   <223> /note="Description of artificial sequence: oligonucleotide"
<400> 68
   acgttggatg tcttttccaa gtggtgaacc 30
<210> 69
   <211> 30
   <212> DNA
   <213> Artificial
<220>
   <221> source
   <223> /note="Description of artificial sequence: oligonucleotide"
<400> 69
   acgttggatg aatttttacc actgagcagg 30
<210> 70
   <211> 30
   <212> DNA
   <213> Artificial
<220>
   <221> source
   <223> /note="Description of artificial sequence: oligonucleotide"
<400> 70
   acgttggatg gctttggctt tacggaagag 30
<210> 71
   <211> 30
   <212> DNA
   <213> Artificial
<220>
   <221> source
   <223> /note="Description of artificial sequence: oligonucleotide"
<400> 71
   acgttggatg attctctctt gagtcggtgc 30
<210> 72
   <211> 30
   <212> DNA
   <213> Artificial
<220>
   <221> source
   <223> /note="Description of artificial sequence: oligonucleotide"
<400> 72
   acgttggatg tagaaccttg ccacagagac 30
<210> 73
   <211> 30
   <212> DNA
   <213> Artificial
<220>
   <221> source
   <223> /note="Description of artificial sequence: oligonucleotide"
<400> 73
   acgttggatg tgcaaaaact aacaaaagcc 30
<210> 74
   <211> 30
   <212> DNA
   <213> Artificial
<220>
   <221> source
   <223> /note="Description of artificial sequence: oligonucleotide"
<400> 74
   acgttggatg agggttatca accttgaggc 30
<210> 75
   <211> 17
   <212> DNA
   <213> Artificial
<220>
   <221> source
   <223> /note="Description of artificial sequence: oligonucleotide"
<400> 75
   ccgccctaca ctttcac 17
<210> 76
   <211> 20
   <212> DNA
   <213> Artificial
<220>
   <221> source
   <223> /note="Description of artificial sequence: oligonucleotide"
<400> 76
   atctgtaact tagagccctt 20
<210> 77
   <211> 18
   <212> DNA
   <213> Artificial
<220>
   <221> source
   <223> /note="Description of artificial sequence: oligonucleotide"
<400> 77
   cagttgaaag agcctcac 18
<210> 78
   <211> 17
   <212> DNA
   <213> Artificial
<220>
   <221> source
   <223> /note="Description of artificial sequence: oligonucleotide"
<400> 78
   ccttaggcat aaccacc 17
<210> 79
   <211> 17
   <212> DNA
   <213> Artificial
<220>
   <221> source
   <223> /note="Description of artificial sequence: oligonucleotide"
<400> 79
   ttctcccgag gctccca 17
<210> 80
   <211> 21
   <212> DNA
   <213> Artificial
<220>
   <221> source
   <223> /note="Description of artificial sequence: oligonucleotide"
<400> 80
   ctggacacaa ttccttagtt a 21
<210> 81
   <211> 18
   <212> DNA
   <213> Artificial
<220>
   <221> source
   <223> /note="Description of artificial sequence: oligonucleotide"
<400> 81
   aactgagatc atgccact 18
<210> 82
   <211> 18
   <212> DNA
   <213> Artificial
<220>
   <221> source
   <223> /note="Description of artificial sequence: oligonucleotide"
<400> 82
   ggaaagggct gctcatcc 18
<210> 83
   <211> 21
   <212> DNA
   <213> Artificial
<220>
   <221> source
   <223> /note="Description of Artificial sequence: oligonucleotide"
<400> 83
   tcattaacgc agaaaaacaa a 21
<210> 84
   <211> 18
   <212> DNA
   <213> Artificial -
<220>
   <221> source
   <223> /note="Description of artificial sequence: oligonucleotide"
<400> 84
   gaggacagac aaatgact 18
<210> 85
   <211> 21
   <212> DNA
   <213> Artificial
<220>
   <221> source
   <223> /note="Description of artificial sequence: oligonucleotide"
<400> 85
   cagtaatatg cttgctgtac c 21
<210> 86
   <211> 23
   <212> DNA
   <213> Artificial
<220>
   <221> source
   <223> '/note="Description of artificial sequence: oligonucleotide"
<400> 86
   ttactagtga cattctaaag gag 23
<210> 87
   <211> 21
   <212> DNA
   <213> Artificial
<220>
   <221> source
   <223> /note="Description of artificial sequence: oligonucleotide"
<400> 87
   gatgcaagaa tagcatggat c 21
<210> 88
   <211> 17
   <212> DNA
   <213> Artificial
<220>
   <221> source
   <223> /note="Description of artificial sequence: oligonucleotide"
<400> 88
   gagctcagtt cccaggg 17
<210> 89
   <211> 21
   <212> DNA
   <213> Artificial
<220>
   <221> source
   <223> /note="Description of artificial sequence: oligonucleotide"
<400> 89
   tgtcagttgt ttttccttga a 21
<210> 90
   <211> 22
   <212> DNA
   <213> Artificial
<220>
   <221> source
   <223> /note="Description of artificial sequence: oligonucleotide"
<400> 90
   gaacaagaaa ccagttgtat ca 22
<210> 91
   <211> 19
   <212> DNA
   <213> Artificial
<220>
   <221> source
   <223> /note="Description of artificial sequence: oligonucleotide"
<400> 91
   ctgtaattcc agcactttg 19
<210> 92
   <211> 24
   <212> DNA
   <213> Artificial
<220>
   <221> source
   <223> /note="Description of artificial sequence: oligonucleotide"
<400> 92
   agtctttaag tttaattgca ggtc 24
<210> 93
   <211> 18
   <212> DNA
   <213> Artificial
<220>
   <221> source
   <223> /note="Description of artificial sequence: oligonucleotide"
<400> 93
   attcctctcc tttccagc 18
<210> 94
   <211> 21
   <212> DNA
   <213> Artificial
<220>
   <221> source
   <223> /note="Description of artificial sequence: oligonucleotide"
<400> 94
   aggctcatct gcatctgtta c 21
<210> 95
   <211> 24
   <212> DNA
   <213> Artificial
<220>
   <221> source
   <223> /note="Description of artificial sequence: oligonucleotide"
<400> 95
   tagaatgttc ttacacaaat ctag 24
<210> 96
   <211> 19
   <212> DNA
   <213> Artificial
<220>
   <221> source
   <223> /note="Description of artificial sequence: oligonucleotide"
<400> 96
   gtgtttctgg tttgttacc 19
<210> 97
   <211> 17
   <212> DNA
   <213> Artificial
<220>
   <221> source
   <223> /note="Description of artificial sequence: oligonucleotide"
<400> 97
   tggcagaaca atgcatc 17
<210> 98
   <211> 22
   <212> DNA
   <213> Artificial
<220>
   <221> source
   <223> /note="Description of artificial sequence: oligonucleotide"
<400> 98
   cccattttac agttatgggc tc 22
<210> 99
   <211> 17
   <212> DNA
   <213> Artificial
<220>
   <221> source
   <223> /note="Description of artificial sequence: oligonucleotide"
<400> 99
   ctgagcaggt gaaaaat 17
<210> 100
   <211> 17
   <212> DNA
   <213> Artificial
<220>
   <221> source
   <223> /note="Description of artificial sequence: oligonucleotide"
<400> 100
   ggtgcctcgt gcagagc 17
<210> 101
   <211> 20
   <212> DNA
   <213> Artificial
<220>
   <221> source
   <223> /note="Description of artificial sequence: oligonucleotide"
<400> 101
   tttcatctga taggcccaac 20
<210> 102
   <211> 29
   <212> DNA
   <213> Artificial
<220>
   <221> source
   <223> /note="Description of artificial sequence: oligonucleotide"
<400> 102
   ttgaaaaggg gcatgtgaaa gtgtagggc 29
<210> 103
   <211> 29
   <212> DNA
   <213> Artificial
<220>
   <221> source
   <223> /note="Description of artificial sequence: oligonucleotide"
<400> 103
   ttgaaaaggg gcaggtgaaa gtgtagggc 29
<210> 104
   <211> 25
   <212> DNA
   <213> Artificial
<220>
   <221> source
   <223> /note="Description of artificial sequence: oligonucleotide"
<400> 104
   gggagcctgt aaagggctct aagtt 25
<210> 105
   <211> 25
   <212> DNA
   <213> Artificial
<220>
   <221> source
   <223> /note="Description of artificial sequence: oligonucleotide"
<400> 105
   gggagcctgt gaagggctct aagtt 25
<210> 106
   <211> 22
   <212> DNA
   <213> Artificial
<220>
   <221> source
   <223> /note="Description of artificial sequence: oligonucleotide"
<400> 106
   tccactgaga tgtgaggctc tt 22
<210> 107
   <211> 22
   <212> DNA
   <213> Artificial
<220>
   <221> source
   <223> /note="Description of artificial sequence: oligonucleotide"
<400> 107
   tccactgaga cgtgaggctc tt 22
<210> 108
   <211> 23
   <212> DNA
   <213> Artificial
<220>
   <221> source
   <223> /note="Description of artificial sequence: oligonucleotide"
<400> 108
   agaaactctt aacggtggtt atg 23
<210> 109
   <211> 23
   <212> DNA
   <213> Artificial
<220>
   <221> source
   <223> /note="Description of artificial sequence: oligonucleotide"
<400> 109
   agaaactctt aatggtggtt atg 23
<210> 110
   <211> 23
   <212> DNA
   <213> Artificial
<220>
   <221> source
   <223> /note="Description of artificial sequence: oligonucleotide"
<400> 110
   caccaagatc agtgggagcc tcg 23
<210> 111
   <211> 23
   <212> DNA
   <213> Artificial
<220>
   <221> source
   <223> /note="Description of artificial sequence: oligonucleotide"
<400> 111
   caccaagatc actgggagcc tcg 23
<210> 112
   <211> 25
   <212> DNA
   <213> Artificial
<220>
   <221> source
   <223> /note="Description of artificial sequence: oligonucleotide"
<400> 112
   attccttagt taagtccttg ttctt 25
<210> 113
   <211> 25
   <212> DNA
   <213> Artificial
<220>
   <221> source
   <223> /note="Description of artificial sequence: oligonucleotide"
<400> 113
   attccttagt taggtccttg ttctt 25
<210> 114
   <211> 23
   <212> DNA
   <213> Artificial
<220>
   <221> source
   <223> /note="Description of artificial sequence: oligonucleotide"
<400> 114
   gatcatgcca ctgtactcca gcc 23
<210> 115
   <211> 23
   <212> DNA
   <213> Artificial
<220>
   <221> source
   <223> /note="Description of artificial sequence: oligonucleotide"
<400> 115
   gatcatgcca ctatactcca gcc 23
<210> 116
   <211> 22
   <212> DNA
   <213> Artificial
<220>
   <221> source
   <223> /note="Description of artificial sequence: oligonucleotide"
<400> 116
   acctggatgt cggatgagca gc 22
<210> 117
   <211> 22
   <212> DNA
   <213> Artificial
<220>
   <221> source
   <223> /note="Description of artificial sequence: oligonucleotide"
<400> 117
   acctggatgt gggatgagca gc 22
<210> 118
   <211> 23
   <212> DNA
   <213> Artificial
<220>
   <221> source
   <223> /note="Description of artificial sequence: oligonucleotide"
<400> 118
   agaaaaacaa agttgatcaa aat 23
<210> 119
   <211> 23
   <212> DNA
   <213> Artificial
<220>
   <221> source
   <223> /note="Description of artificial sequence: oligonucleotide"
<400> 119
   agaaaaacaa aattgatcaa aat 23
<210> 120
   <211> 23
   <212> DNA
   <213> Artificial
<220>
   <221> source
   <223> /note="Description of artificial sequence: oligonucleotide"
<400> 120
   ggttctgtct cagtcatttg tct 23
<210> 121
   <211> 23
   <212> DNA
   <213> Artificial
<220>
   <221> source
   <223> /note="Description of artificial sequence: oligonucleotide"
<400> 121
   ggttctgtct tagtcatttg tct 23
<210> 122
   <211> 19
   <212> DNA
   <213> Artificial
<220>
   <221> source
   <223> /note="Description of artificial sequence: oligonucleotide"
<400> 122
   tcttcatgcc gggtacagc 19
<210> 123
   <211> 19
   <212> DNA
   <213> Artificial
<220>
   <221> source
   <223> /note="Description of artificial sequence: oligonucleotide"
<400> 123
   tcttcatgcc aggtacagc 19
<210> 124
   <211> 21
   <212> DNA
   <213> Artificial
<220>
   <221> source
   <223> /note="Description of artificial sequence: oligonucleotide"
<400> 124
   cagggtcttc tgctccttta g 21
<210> 125
   <211> 21
   <212> DNA
   <213> Artificial
<220>
<221> source
   <223> /note="Description of artificial sequence: oligonucleotide"
<400> 125
   cagggtcttc tcctccttta g 21
<210> 126
   <211> 24
   <212> DNA
   <213> Artificial
<220>
   <221> source
   <223> /note="Description of artificial sequence: oligonucleotide"
<400> 126
   tagcatggat cagttcatca acta 24
<210> 127
   <211> 24
   <212> DNA
   <213> Artificial
<220>
   <221> source
   <223> /note="Description of artificial sequence: oligonucleotide"
<400> 127
   tagcatggat ctgttcatca acta 24
<210> 128
   <211> 25
   <212> DNA
   <213> Artificial
<220>
   <221> source
   <223> /note="Description of artificial sequence: oligonucleotide"
<400> 128
   gaatcttgaa tgtccctggg aactg 25
<210> 129
   <211> 25
   <212> DNA
   <213> Artificial
<220>
   <221> source
   <223> /note="Description of artificial sequence: oligonucleotide"
<400> 129
   gaatcttgaa tgcccctggg aactg 25
<210> 130
   <211> 24
   <212> DNA
   <213> Artificial
<220>
   <221> source
   <223> /note="Description of artificial sequence: oligonucleotide"
<400> 130
   tttttccttg aagtgacaga ctta 24
<210> 131
   <211> 24
   <212> DNA
   <213> Artificial
<220>
   <221> source
   <223> /note="Description of artificial sequence: oligonucleotide"
<400> 131
   tttttccttg aaatgacaga ctta 24
<210> 132
   <211> 21
   <212> DNA
   <213> Artificial
<220>
   <221> source
   <223> /note="Description of artificial sequence: oligonucleotide"
<400> 132
   gttgtatcat tcagtatata t 21
<210> 133
   <211> 21
   <212> DNA
   <213> Artificial
<220>
   <221> source
   <223> /note="Description of artificial sequence: oligonucleotide"
<400> 133
   gttgtatcac tcagtatata t 21
<210> 134
   <211> 23
   <212> DNA
   <213> Artificial
<220>
   <221> source
   <223> /note="Description of artificial sequence: oligonucleotide"
<400> 134
   tccagcactt tgggaggcca agg 23
<210> 135
   <211> 23
   <212> DNA
   <213> Artificial
<220>
   <221> source
   <223> /note="Description of artificial sequence: oligonucleotide"
<400> 135
   tccagcactt tgcgaggcca agg 23
<210> 136
   <211> 22
   <212> DNA
   <213> Artificial
<220>
   <221> source
   <223> /note="Description of artificial sequence: oligonucleotide"
<400> 136
   aatgtagcca cgacctgcaa tt 22
<210> 137
   <211> 22
   <212> DNA
   <213> Artificial
<220>
   <221> source
   <223> /note="Description of artificial sequence: oligonucleotide"
<400> 137
   aatgtagcca agacctgcaa tt 22
<210> 138
   <211> 22
   <212> DNA
   <213> Artificial
<220>
   <221> source
   <223> /note="Description of artificial sequence: oligonucleotide"
<400> 138
   ctcctttcca gcgctattat tg 22
<210> 139
   <211> 22
   <212> DNA
   <213> Artificial
<220>
   <221> source
   <223> /note="Description of artificial sequence: oligonucleotide"
<400> 139
   ctcctttcca gcactattat tg 22
<210> 140
   <211> 22
   <212> DNA
   <213> Artificial
<220>
   <221> source
   <223> /note="Description of artificial sequence: oligonucleotide"
<400> 140
   gccctgattg cggtaacaga tg 22
<210> 141
   <211> 22
   <212> DNA
   <213> Artificial
<220>
   <221> source
   <223> /note="Description of artificial sequence: oligonucleotide"
<400> 141
   gccctgattg cagtaacaga tg 22
<210> 142
   <211> 23
   <212> DNA
   <213> Artificial
<220>
   <221> source
   <223> /note="Description of artificial sequence: oligonucleotide"
<400> 142
   cacaaatcta ggtggaacag cct 23
<210> 143
   <211> 23
   <212> DNA
   <213> Artificial
<220>
   <221> source
   <223> /note="Description of artificial sequence: oligonucleotide"
<400> 143
   cacaaatcta gatggaacag cct 23
<210> 144
   <211> 24
   <212> DNA
   <213> Artificial
<220>
   <221> source
   <223> /note="Description of artificial sequence: oligonucleotide"
<400> 144
   gacagtttag tgggtaacaa acca 24
<210> 145
   <211> 24
   <212> DNA
   <213> Artificial
<220>
   <221> source
   <223> /note="Description of artificial sequence: oligonucleotide"
<400> 145
   gacagtttag taggtaacaa acca 24
<210> 146
   <211> 23
   <212> DNA
   <213> Artificial
<220>
   <221> source
   <223> /note="Description of artificial sequence: oligonucleotide"
<400> 146
   tttttagcaa cgatgcattg ttc 23
<210> 147
   <211> 23
   <212> DNA
   <213> Artificial
<220>
   <221> source
   <223> /note="Description of artificial sequence: oligonucleotide"
<400> 147
   tttttagcaa tgatgcattg ttc 23
<210> 148
   <211> 19
   <212> DNA
   <213> Artificial
<220>
   <221> source
   <223> /note="Description of artificial sequence: oligonucleotide"
<400> 148
   tatgggctct caaaattca 19
<210> 149
   <211> 19
   <212> DNA
   <213> Artificial
<220>
   <221> source
   <223> /note="Description of artificial sequence: oligonucleotide"
<400> 149
   tatgggctcc caaaattca 19
<210> 150
   <211> 23
   <212> DNA
   <213> Artificial
<220>
   <221> source
   <223> /note="Description of artificial sequence: oligonucleotide"
<400> 150
   atacttattt gaatttttca cct 23
<210> 151
   <211> 23
   <212> DNA
   <213> Artificial
<220>
   <221> source
   <223> /note="Description of artificial sequence: oligonucleotide"
<400> 151
   atacttattt ggatttttca cct 23
<210> 152
   <211> 25
   <212> DNA
   <213> Artificial
<220>
   <221> source
   <223> /note="Description of artificial sequence: oligonucleotide"
<400> 152
   ctcgtgcaga gcccctcctc tgctg 25
<210> 153
   <211> 25
   <212> DNA
   <213> Artificial
<220>
   <221> source
   <223> /note="Description of artificial sequence: oligonucleotide"
<400> 153
   ctcgtgcaga gctcctcctc tgctg 25
<210> 154
   <211> 22
   <212> DNA
   <213> Artificial
<220>
   <221> source
   <223> /note="Description of artificial sequence: oligonucleotide"
<400> 154
   aggcccaaca gtataccttt gc 22
<210> 155
   <211> 22
   <212> DNA
   <213> Artificial
<220>
   <221> source
   <223> /note="Description of artificial sequence: oligonucleotide"
<400> 155
   aggcccaacg gtataccttt gc 22
   2

## Description

The present invention relates to a method of classifying an individual comprising analyzing the nucleic acid of a sample taken from said patient for nucleotide polymorphisms in a haplotype block comprising the gene encoding FKBP51 and/or determining the expression level of FKBP51 in said sample. In a preferred embodiment, the invention provides a method of predicting the response to antidepressant therapy. Furthermore, a method of developing an anti-depressant drug, and pharmaceutical compositions are provided.

In this specification, a number of documents is cited.

National surveys in Germany and in the United States agree that depression and other affective disorders (e.g. anxiety disorders and bipolar disorder) are among the most common medical conditions worldwide with lifetime prevalence for major depression reaching up to 14%. Pharmacotherapy is an effective treatment of depression and since the serendipitous discovery of the first antidepressant drug imipramine, a number of antidepressant drugs are now available. So far, all commercially available antidepressant drugs share the same pharmacological principle of enhancing monoaminergic neurotransmission, even though this may be achieved through a variety of mechanisms.' Despite intensive efforts in the development of antidepressant drugs, major breakthroughs have only been achieved on the side effect profile of these drugs. Even though antidepressants are the most effective treatment for depressive disorders, there is still substantial need for improvement. Adequate therapy response to a single antidepressant is only observed in 40-60% of patients, even when given in sufficiently high dose for enough time. There is also a substantial lag between the onset of treatment and clinical improvement that can last up to several weeks, even if "therapeutical" plasma' concentrations can now be reached in a shorter amount of time. Furthermore, there are a percentage of patients that develop therapy-resistant depression, unresponsive to multiple treatment trials.

Although antidepressant drugs elicit quite divergent immediate effects, it is hypothesized that they all target a common final signaling pathway. The hypothalamic-pituitary-adrenal (HPA) system is considered to play a central pathophysiological role in this disease, because this system is not only consistently dysregulated in patients suffering from major depression, but normalization of this dyregulation is a prerequisite for successful treatment¹. Currently used antidepressant drugs exert instant actions at various elements involved in monoaminergic neurotransmission². These trigger a cascade of events that ultimately lead to the resolution of the clinical symptoms of depression. A host of data implicates central and peripheral disturbances of stress hormone regulation in the pathogenesis of depression and normalization of these defects as a necessary predecessor of clinical response to medication^{3,4}. Central mechanisms for HPA-axis hyperactivity are an increased neurotransmission of the hypothalamic peptides corticotropin releasing hormone (CRH) and arginine vasopressin (AVP) that stimulate adrenocorticotropic hormone (ACTH) and cortisol release and an impaired negative feedback of this system due to glucocorticoid (GC) receptor (GR) insensitivity^{5,6,7,8} Preclinical and clinical studies suggest that one mechanism of action of antidepressant drugs may be to restore negative HPA-axis feedback via the GR leading to a downregulation of the overexpressed peptides CRH and AVP^{9,10,11,12,13,14}. For proper functioning the GR, a ligand-activated transcription factor, depends on a large molecular complex that is necessary for proper receptor activation and transcriptional regulation of GR target genes^{15,16}.

A series of *in vivo* and *in vitro* studies have implicated FK506-binding protein 51 (FKBP51) as one among a host of physiologically relevant regulators of GR sensitivity. FKBP51 is part of the mature GR heterocomplex¹⁹. Upon hormone binding, FKBP51 is replaced by FKBP52, which then recruits dynein into the complex, allowing its nuclear translocation and transcriptional activity²⁰. A naturally occurring overexpression of FKBP51 appears to be the common cause of GR insensitivity in New world monkeys^{21.22,23,26}. Moreover, overexpression of human FKBP51²¹ *in vitro* clearly reduces hormone binding affinity and nuclear translocation of GR. Glucocorticoids induce FKBP51 mRNA expression via an ultra-short negative feedback loop for GR activity^{2a}.

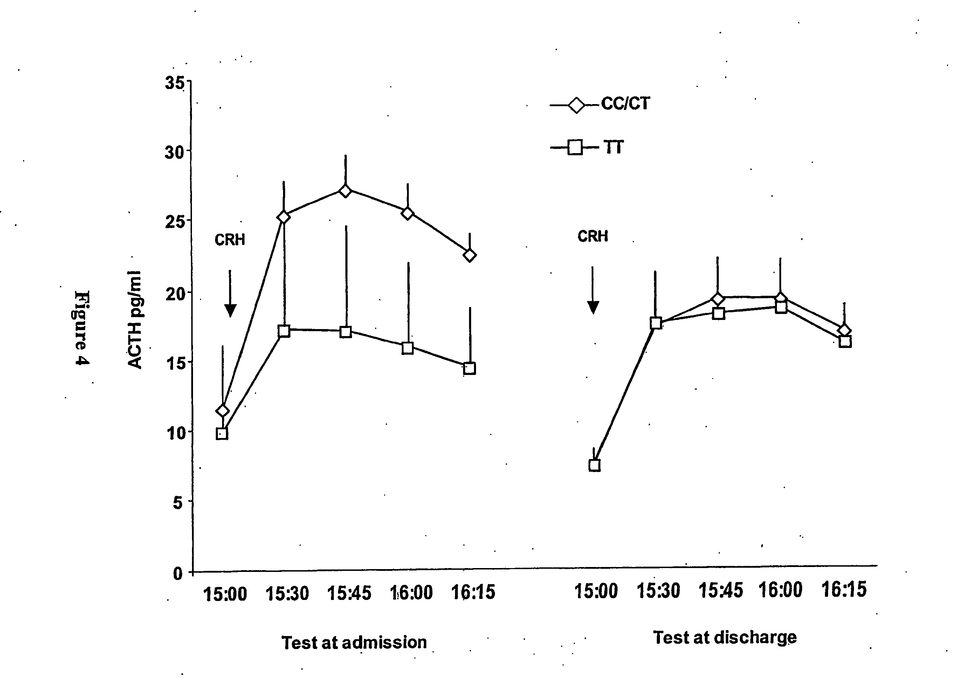

WO04/039837 relates to polynucleotides and polypeptides which can be used in the diagnosis and therapy of depression. Figure 4 of the application shows that FKPB51 (named in the application as FKPB5, FK506 binding protein 5, SEQ ID NO 32 or SEQ ID NO 33) is upregulated in transgenic animals overexpressing the corticotropin releasing hormone (CRH; see pages 4, 46-47). Furthermore, claims 1-25, 27, 29-31 relates to methods to diagnose a CRH induced gene expression profile by determining the expression or protein levels of FKBP51. WO2004/039837 does not however disclose any polymorphisms in FKBP51, nor does it make any association between polymorphisms in FKBP51 and improved response to antidepressants.

WO03/082210 relates to gene targets, polymorphisms, variants and methods for the diagnosis and treatment of schizophrenia. The document identifies four polymorphisms in FKBP51 (see pages 104 to 105 and Table 2) that are associated with schizophrenia. Two of these polymorphisms are known in the art (rs3777747 and rs992105). The application fails to provide, however, any implications for depression, let alone an association between polymorphisms in the FKBP51 locus and improved response to antidepressants.

Yu et al. (Molecular Psychiatry (2002) 7, 1115-1119) describes correlations between a polymorphism of the serotonin transporter promoter and the anti-depressant response in major depressive disorders.

It is known that the response to antidepressant therapy varies significantly from patient to patient. So far, these variations can only be determined and analysed in a retrospective manner. However, it would be highly desirable to classify individuals and to predict their response to therapy in order to tailor treatment to the individual patient's specific needs. The technical problem underlying the present invention therefore was to provide methods of classifying individuals.

The solution to said technical problem is achieved by providing the embodiments characterized in the claims.

Accordingly, the present invention relates to a method of classifying an individual comprising analyzing the nucleic acid of a sample taken from said individual for nucleotide polymorphisms in the gene encoding FKBP51 or in a haplotype block comprising the gene encoding FKBP51 and optionally determining the expression level of FKBP51 in said sample, wherein said individual is a patient suffering from depression and said classifying (a) consists of or comprises predicting the response to therapy, wherein the haplotype block comprises at least one SNP selected from a first group consisting of rs2766534, rs4711429, rs4713921, rs9462104, rs9394312, rs4713916, rs943297, rs9380528, rs9380526, rs10947563, rs9380525, rs6912833, rs2143404, rs1360780, rs1591365, rs7748266, rs9470069, rs6926133, rs3777747, rs4713899, rs2395634, rs7753746, rs3800373, rs10807151, rs3800374, rs9348978, rs11751447 and rs2395631, wherein the respective alleles of each SNP are recognized by the respective sequences provided in Table 4; and/or wherein the polymorphism is at least one SNP selected from said first group; or (b) consists of or comprises predicting the predisposition for an elevated number of episodes of depression, wherein the haplotype block comprises at least one SNP selected from a second group consisting of rs4713916, rs1360780, and rs3800373, wherein the respective alleles of each SNP are recognized by the respective sequences provided in Table 4; and/or wherein the polymorphism is at least one SNP selected from said second group. In other words, said analyzing is performed in vitro. Described herein is also that said analyzing is performed ex vivo or in vivo.

The term "classifying" refers to the assignment of individuals to two or more groups or classes. In other words, individuals, previously unclassified, get labelled by their respective class. The assigned class label may refer to parameters used for classification, e.g. polymorphisms and/or expression levels, or may refer to parameters not used for classification because their values are not known beforehand, e.g. fast or slow response to therapy. In the first case, class discovery methods, e.g. clustering may be applied, whereas in the second case predictive classification methods are used. Classification may be done manually by a trained person or by a computer program provided with the values of the parameters used for class distinction. Patients have to give informed consent. Data have to be handled and kept secret in accordance with national laws.

The term "nucleotide polymorphism" refers to the occurrence of one or more different nucleotides or bases at a given location on a chromosome. Usually, polymorphisms are distinguished from mutations based on their prevalence. Sometimes a threshold of 1% prevalence in a population of individuals is considered for separating polymorphisms (more frequent) from mutations (less frequent). A single nucleotide polymorphism (SNP) is a polymorphism of a single nucleotide or base. The SNP database maintained at NCBI (http://www.ncbi.nlm.nih.gov/SNP/) divides SNPs into SNPs in the proximity of a known locus and such that are 5' further away than 2 kb from the most 5' feature of a gene and 3' further away than 500 bases from the most 3' feature of a gene. SNPs in the proximity of a known locus are further divided into SNPs occurring at an mRNA location and such that do not. SNPs occurring at an mRNA location comprise coding and non-coding SNPs.

In view of the evidence relating to nucleotide polymorphisms in a haplotype block comprising the gene encoding FKBP51 laid down in the present application, the inventors for the first time envisaged classifying individuals based on said polymorphisms.

Nucleotide polymorphisms may be associated or linked to a particular phenotype. Genetic screening exploits this association.

Genetic screening (also called genotyping or molecular screening), can be broadly defined as testing to determine if an individual has mutations, alleles or polymorphisms that either cause a specific phenotype or are "linked" to the mutation causing the phenotype. Linkage refers to the phenomenon that the DNA sequences which are close together in the genome have a tendency to be inherited together. Two or more sequences may be linked because of some selective advantage of co-inheritance. More typically, however, two or more polymorphic sequences are co-inherited because of the relative infrequency with which meiotic recombination events occur within the region between the two polymorphisms. The co-inherited polymorphic alleles are said to be in linkage disequilibrium (LD) with one another because, in a given population, they tend to either both occur together or else not occur at all in any particular member of the population. Indeed, where multiple polymorphisms in a given chromosomal region are found to be in linkage disequilibrium with one another, they define a quasi-stable genetic "haplotype", "haplotype block" or "LD block".

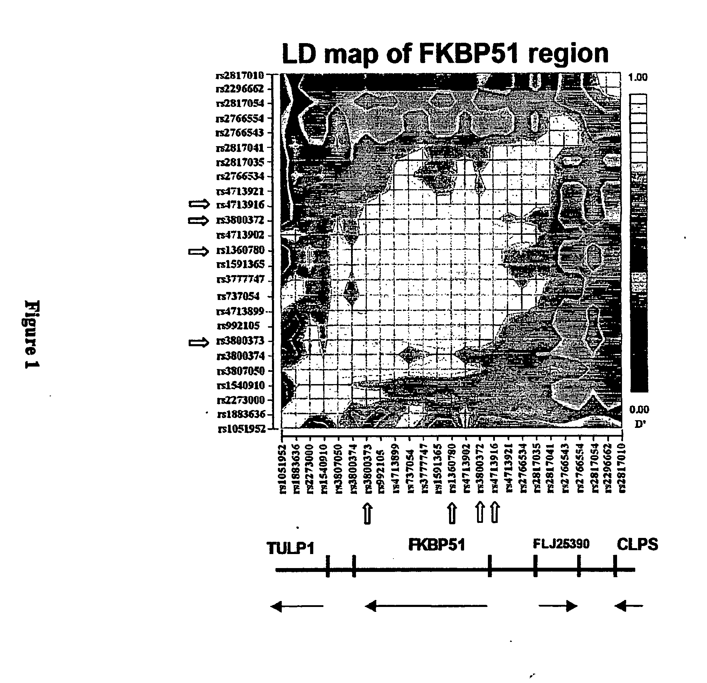

Location and size of haplotype blocks can be determined using computer programs, e.g. the program HaploBlockFinder²⁵. Figure 1 shows a linkage disequilibrium map of the FKBP51 region. A haplotype block according to the invention extends, for example, from the polymorphism rs3800374 to rs4713921.

Furthermore, where a phenotype-causing polymorphism is found within a gene or haplotype or in linkage with a haplotype, one or more polymorphic alleles of the haplotype can be used as a diagnostic or prognostic indicator of the likelihood of developing a specific phenotype. Identification of a haplotype which spans or is linked to a phenotype-causing polymorphism, serves as a predictive measure of an individual's likelihood of exhibiting that phenotype-causing polymorphism. Importantly, such prognostic or diagnostic procedures can be utilized without necessitating the identification and isolation of the actual phenotype-causing molecule. This is significant because the precise determination of the molecular basis of the establishment of a specific phenotype can be difficult and laborious, especially in the case of multifactorial phenotype.

Surprisingly, it has been found in healthy individuals that nucleotide polymorphisms in a haplotype block comprising the gene encoding FKBP51 correlate with the expression level of FKBP51. Therefore, according to the invention, the expression level of FKBP51 may be determined in addition to said nucleotide polymorphisms for the purpose of classifying individuals.

As stated above, there is an unmet need for methods of classifying patients suffering from depression with regard to their response to therapy. Unexpectedly, nucleotide polymorphisms in a haplotype block comprising the gene encoding FKBP51 were found to correlate significantly with the response to antidepressant therapy.

Therefore, in accordance with part (a) of the main embodiment, said classifying consists of or comprises predicting the response to therapy of an individual, wherein said individual is a patient suffering from depression. The availability of information as to the expected response to therapy is of great advantage for the patient, as a rational choice of drug and/or a tailored therapy regimen minimize adverse side effects and ensure the fastest decline of the symptoms of the disease.

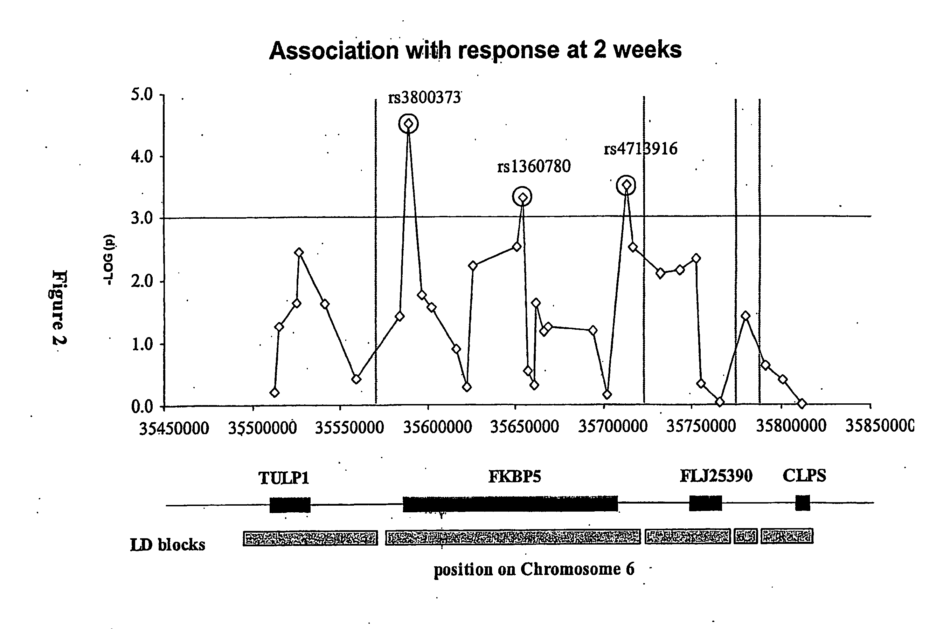

Figure 2 shows a plot of the significance of the association with response to antidepressant therapy of SNPs in or in the proximity of the FKBP51 locus investigated by the inventors.

"Therapy" refers in accordance with the present invention to treatment with antidepressant drugs. Most antidepressant drugs target the transport of the neurotransmitters serotonin and/or norepinephrine, or the activity of the enzyme monoamine oxidase. Common antidepressant drugs include: Selective serotonin-reuptake inhibitors (e.g., fluoxetine, paroxetine, sertraline, fluvoxamine), tricyclic antidepressants (e.g., amitriptyline, imipramine, desipramine, nortriptyline), monoamine oxidase inhibitors (e.g., phenelzine, isocarboxazid, tranylcypromine), and designer antidepressants such as mirtazapine, reboxetine, nefazodone. Benzodiazepines, or lithium carbonate may also be administered.

The inventors surprisingly found that elevated levels of FKBP51 correlate significantly with a faster response to therapy, i.e., the decline of symptoms of depression as determined by the Hamilton Depression Rating Scale (HAM-D) sets in earlier than in patients with lower levels of FKBP51. This observation is indeed unexpected, considering that transcriptional activity of GR heterocomplex requires that FKBP51 dissociates from the heterocomplex. The skilled person would expect that elevated levels of FKBP51 lead to an accumulation of the GR heterocomplex in the cytoplasm, thereby preventing GR from activating its target genes, the latter being considered essential for effective antidepressant therapy.

The authors of reference 24 (Vermeer et al., 2003) report a glucocorticoid-induced increased in lymphocytic FKBP51 mRNA expression and propose induction of FKBP51 mRNA as a marker for the assessment of individual GC sensitivity, for the in vitro measurement of GC potency, and the in vivo determination of GC bioavailability. While Vermeer et al. describe an induced increase of FKBP51 expression, the present invention relates to elevated levels of FKBP51 expression associated with nucleotide polymorphisms. Vermeer et al. do not refer to any polymorphisms in the FKBP51 locus. Furthermore, Vermeer et al. do not refer to any implications of their observations for the field of depression therapy.

Also reference 1 (Holsboer, 2000) discusses the implications of an altered FKBP51 level for corticosteroid signaling. According to Holsboer, ligand-bound GR associates with other proteins comprising heat shock proteins and immunophilins, specifically FKBP51 or FKBP52. An elevated FKBP51 level is discussed in relation to excessively high cortisol levels in squirrel monkeys²⁶, whereby it is speculated that the elevated FKBP51 level serves for compensating the notoriously high cortisol levels in squirrel monkeys.

Holsboer furthermore discusses possible causes of inherited glucocorticoid resistance, viz, a polymorphism in the GR gene or other alterations in genes, whose products are involved in glucocorticoid signaling. However, Holsboer does not designate any specific protein involved in glucocorticoid signaling.

The application of genotyping in the field of pharmacology gave rise to the fields of pharmacogenetics and, more recently, pharmacogenomics, the latter being used to denote studies correlating drug response with the analysis of multiple genes, e.g. their expression and/or polymorphisms or mutations in said genes. It is envisaged to stratify the population such that groups of individuals are obtained which receive tailored treatments. In order to get approval for tailored drugs and/or treatment regimens, the stratification of the population has to be extended to clinical trials.

Therefore, in a preferred embodiment, classifying according to the present invention comprises selecting an individual for a clinical trial. Individuals selected for clinical trials in accordance with the present invention may comprise healthy individuals and/or patients suffering from depression.

There are several published patent applications in the field of pharmacogenomics, that describe the use of gene sequence variations including SNPs (WO 00/50639, WO 99/64626) or expression profiling on a large scale for evaluating antidepressant and other drug response in patients. They give detailed technical descriptions on how to use SNPs to assess drug response. However, these documents neither anticipate nor suggest to investigate polymorphisms in the FKBP51 locus or FKBP51 expression levels.

It has been found by the inventors that nucleotide polymorphisms in a haplotype block comprising the gene encoding FKBP51 correlate with the number of a patient's previous episodes of depression. Therefore, said polymorphisms and/or the expression level of FKBP51 can be used as (a) prognostic marker/s for an elevated number of episodes of depression.

Accordingly, in accordance with part (b) of the main embodiment, classifying according to the present invention consists of or comprises predicting the predisposition of an individual for an elevated number of episodes of depression, wherein said individual is a patient suffering from depression.

In a referred embodiment, the nucleic acid to be analyzed is genomic DNA (gDNA).

According to the invention, the haplotype block comprises at least one SNP selected from the group consisting of rs2766534, rs4711429, rs4713921, rs9462104, rs9394312, rs4713916, rs943297, rs9380528, rs9380526, rs10947563, rs9380525, rs6912833, rs2143404, rs1360780, rs1591365, rs7748266, rs9470069, rs6926133, rs3777747, rs4713899, rs2395634, rs7753746, rs3800373, rs10807151, rs3800374, rs9348978, rs11751447 and, rs2395631. According to this embodiment, the identification and analysis of any nucleotide polymorphism in said haplotype block is envisaged.

The identifiers recited above permit retrieval of sequence and other information for the respective SNP in the SNP database maintained at the NCBI (http://www.ncbi.nlm.nih.gov/SNP/). Obviously, this does not apply for the novel SNPs according to the invention, i.e. FKBP5UT5A and FKBP5UT5C.

In a yet more preferred embodiment, the polymorphism is a SNP in a non-coding region of said gene or haplotype block.

According to the invention, the polymorphism is at least one SNP selected from the group consisting of rs2766534, rs4711429, rs4713921, rs9462104, rs9394312, rs4713916, rs943297, rs9380528, rs9380526, rs10947563, rs9380525, rs6912833, rs2143404, rs1360780, rs1591365, rs7748266, rs9470069, rs6926133, rs3777747, rs4713899, rs2395634, rs7753746, rs3800373, rs10807151, rs3800374, rs9348978, rs11751447 and, rs2395631.

These 28 SNPs are most significantly associated with improved response to antidepressant treatment. The following table summarises the position of these SNPs on chromosome 6, the region of the FKBP51 locus containing the SNP, nucleotide changes for each genotype, heterozygote frequency and the p-value for an association with fast response to an antidepressant drug.

**Table 1**

| SNP | Position On Chr 6 | type | Nucleotide change | Heterozygote frequency | p-value |
|---|---|---|---|---|---|
| rs2766534 | 35732569 | Promoter | G/T | 33,2% | 0,0057 |
| rs4711429 | 35730403 | Promoter | G/A | 38,3% | 0,0076 |
| rs4713921 | 35728632 | Promoter | C/T | 39,1% | 0,0036 |
| rs9462104 | 35721700 | Promoter | T/C | 38,5% | 0,0043 |
| rs9394312 | 35719185 | Promoter | C/G | 49% | 0,0016 |
| rs4713916 | 35716838 | Promoter | G/A | 36.6% | 5.5 x 10⁻⁵ |
| rs943297 | 35714715 | Promoter | G/A | 38.8% | 3.1 x 10⁻⁴ |
| rs9380528 | 35711107 | Promoter | A/G | 49% | 0,0034 |
| FKBP5UT5A | 35706425 | Promoter | G/C | 0.3% | * |
| FKBP5UT5C | 35705284 | Promoter | A/G | 1.6% | * |
| rs9380526 | 35705182 | Promoter | T/C | 41.4% | 4.8 x 10⁻⁴ |
| rs10947563 | 35700292 | Intron 1 | A/G | 35.8% | 0,0013 |
| rs9380525 | 35679893 | Intron 1 | C/G | 42.1% | 2.7 x 10⁻⁴ |
| rs6912833 | 35664440 | Intron 1 | T/A | 44.8% | 3.5 x 10⁻⁴ |
| rs2143404 | 35657536 | Intron 2 | C/T | 24.8% | 0,0460 |
| rs1360780 | 35654426 | Intron 2 | C/T | 40.1% | 1.2 x 10⁻⁴ |
| rs1591365 | 35650962 | Intron 3 | A/G | 38.6% | 6.2 x 10⁻⁴ |
| rs7748266 | 35639599 | Intron 3 | C/T | 26,4% | 0,0348 |
| rs9470069 | 35629516 | Intron 5 | C/G | 18,4% | 0,0450 |
| rs6926133 | 35626230 | Intron 5 | A/C | 27,9% | 0,0135 |
| rs3777747 | 35625857 | Intron 5 | A/G | 49,6% | 0,0061 |
| rs4713899 | 35616136 | Intron 5 | A/G | 25,4% | 0,0244 |
| rs2395634 | 35614615 | Intron 5 | A/G | 39,9% | 0,0036 |
| rs7753746 | 35612277 | Intron 5 | A/G | 26,1% | 0,0328 |
| rs3800373 | 35589331 | 3'UTR | A/C | 33.0% | 2.8 x 10⁻⁵ |
| rs10807151 | 35587723 | 3' of FKBP51 | T/C | 25,9% | 0,0125 |
| rs3800374 | 35584261 | 3' of FKBP51 | C/T | 26,7% | 0,0354 |
| rs9348978 | 35564135 | 3' of FKBP51 | G/A | 48.2% | 5.8 x 10⁻⁴ |
| rs11751447 | 35556977 | 3' of FKBP51 | T/C | 48% | 0,0179 |
| rs2395631 | 35554170 | 3' of FKBP51 | G/A | 47,2% | 0,0114 |

| | | | | | |
|---|---|---|---|---|---|
| * Patient sample too small to estimate p-value | | | | | |

Following the identification of individuals homozygous for the TT allele of rs2766534, or the AA allele of rs4711429, or the TT allele of rs4713921, or the CC allele of rs9462104, or the GG allele of rs9394312, or the AA allele of rs4713916, or the AA allele of rs943297, or the GG allele of rs9380528, or the CC allele of rs9380526, or the GG allele of rs10947563, or the GG allele of rs9380525, or the AA allele of rs6912833, or the TT allele of rs2143404, or the TT allele of rs1360780, or the GG allele of rs1591365, or the TT allele of rs7748266, or the GG allele of rs9470069, or the CC allele of rs6926133, or the GG allele of rs3777747, or the GG allele of rs4713899, or the GG allele of rs2395634, or the GG allele of rs7753746, or the CC allele of rs3800373, or the CC allele of rs10807151, or the TT allele of rs3800374, or the AA allele of rs9348978, or the CC allele of rs11751447, or the AA allele of rs2395631, and optionally an increase in the expression or protein levels of FKBP51, a normal prudent physician would recommend prescription or administration of an antidepressant drug. Administration and dosage of antidepressant drugs can vary between patients and are well known in the medical art, see, for example Benkert and Hippius, "compendium der Psychiatrischen Pharmakotherapie", Springer Verlag Publishing, 2000; Albers, "Handbook of Psychiatric Drugs: 2001-2002 Edition", Current Clinical Strategies Publishing, 2000. Preferred examples include between 5 mg and 80 mg per day, preferably 20 mg, fluoxetine; between 5 mg and 50 mg per day, preferably 20 mg, paroxetine; between 5 mg and 200 mg per day, preferably 50 mg, sertraline; between 5 mg and 300 mg per day, preferably 100 mg, fluvoxamine; between 5 mg and 100 mg per day, preferably 30 mg, mirtazapine; between 4 mg and 50 mg, preferably 8 mg, reboxetine; between 5 mg and 600 mg per day, preferably 200 mg, nefazodone; between 450 mg and 1800 mg per day, preferably 900 mg, lithium carbonate.

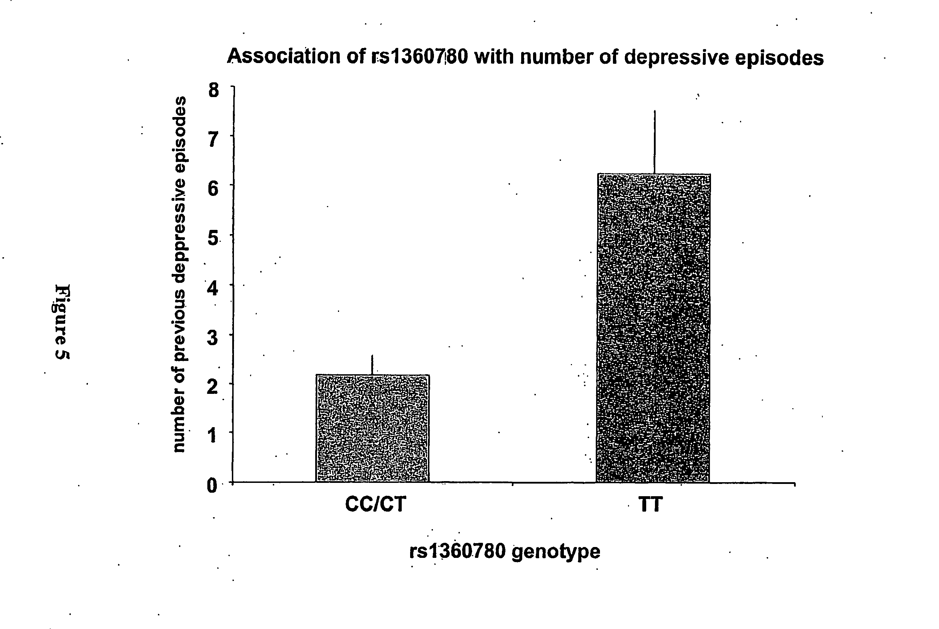

Furthermore, the SNPs of said second group correlate with the number of previous episodes of depression. This is exemplified by the data shown in Figures 5 (rs1360780), 6 (rs4713916) and 7 (rs3800373).

Several methods of analyzing a nucleic acid for nucleotide polymorphisms are known in the art. Accordingly, in a preferred embodiment, analyzing the nucleic acid by the method of the invention comprises (a) a primer extension assay; (b) a differential hybridization assay; and/or (c) an assay which detects allele-specific enzyme cleavage.

The underlying principles and the use of said assays has been described in an article of Asil Memisoglu (www.thebiotechclub.org/industry/emerging/pharmacogenomics.php). Examples for said assays are known by a person skilled in the art.

In a more preferred embodiment, the method according to the invention comprises, prior to analyzing, amplification of at least a portion of said gene or haplotype block. Preferably, said amplification is effected by or said amplification is the polymerase chain reaction (PCR). The principles of and procedures to perform PCR are known in the art.

In a more preferred embodiment, said amplification reaction uses primers which hybridize specifically with a portion of said gene or haplotype block. Means of ensuring specificity of hybridization according to the present invention are known in the art and include stringent hybridization conditions. The term "stringent hybridization conditions", as used in the description of the present invention, is well known to the skilled artisan. Appropriate stringent hybridization conditions for each sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1 x SSC at about 65°. Other stringent hybridization conditions are for example 0.2 x SSC (0.03 M NaCl, 0.003M Natriumcitrat, pH 7) at 65°C.

In a preferred embodiment, the primers to be used for said amplification reaction have the sequence as set forth in SEQ ID NOs: 21 and 22; 23 and 24; 25 and 26; 27 and 28; 29 and 30; 1 and 2; ; 31 and 32; 33 and 34; 39 and 40; 41 and 42; 43 and 44; 45 and 46; 47 and 48; 5 and 6; 49 and 50; 51 and 52; 53 and 54; 55 and 56; 57 and 58; 59 and 60; 61 and 62; 63 and 64; 7 and 8; 65 and 66; 67 and 68; 69 and 70; 71 and 72; or 73 and 74 and disclosed in the following table.

**Table 2**

| SNP-ID | sense | antisense |
|---|---|---|
| rs2766534 | ACGTTGGATGTCGAAGGGACTTATT CCTCC (SEQ ID NO: 21) | ACGTTGGATGCAGCAGAAGGAAGAC ATCAG (SEQ ID NO: 22) |
| rs4711429 | ACGTTGGATGACACACGGCTCATCT GTAAC (SEQ ID NO: 23) | ACGTTGGATGATGCCAGGCATTTGG GTTTC (SEQ ID NO: 24) |
| rs4713921 | ACGTTGGATGAAGCCCTGTGGTTTT ATGCC (SEQ ID NO: 25) | ACGTTGGATGTGGAACAATTCTGTC CCCAG (SEQ ID NO: 26) |
| rs9462104 | ACGTTGGATGTTTCCTCCCTAGATTC CCAG (SEQ ID NO: 27) | ACGTTGGATGGTCCCAGTGGAACTT TATGG (SEQ ID NO: 28) |
| rs9394312 | ACGTTGGATGACAAGCTATCGGGCT TTCTC (SEQ ID NO: 29) | ACGTTGGATGAGACAATCTCCCAGT TGCAG (SEQ ID NO: 30) |
| rs4713916 | ACGTTGGATGTATCTGGCAACCCTA ACCTC (SEQ ID NO: 1) | ACGTTGGATGCCTAACGAGATAGTG AGGAG (SEQ ID NO: 2) |
| rs943297 | ACGTTGGATGTCTGAAATCCAGCTG GACAC (SEQ ID NO: 31) | ACGTTGGATGAGTGAGACTCTGTCC AAAAG (SEQ ID NO: 32) |
| rs9380528 | ACGTTGGATGTTGAGACCAGATGTT GGAGG (SEQ ID NO: 33) | ACGTTGGATGGACAGAGTCTGGCTC TGTTG (SEQ ID NO: 34) |
| FKBP5UT5A | ACGTTGGATGTACAGACCCTGCAAA GAACC (SEQ ID NO: 35) | ACGTTGGATGTGCGCTATGGCTGCA GATAC (SEQ ID NO: 36) |
| FKBP5UT5C | ACGTTGGATGTACAGACCCTGCAAA GAACC (SEQ ID NO: 37) | ACGTTGGATGTGCGCTATGGCTGCA GATAC (SEQ ID NO: 38) |
| rs9380526 | ACGTTGGATGAAACAGGGAAGCTTC TAGGC (SEQ ID NO: 39) | ACGTTGGATGAAGCCAAGACTGAAA CCGCC (SEQ ID NO: 40) |
| rs10947563 | ACGTTGGATGTATTACAGGCGTGAG CCATC (SEQ ID NO: 41) | ACGTTGGATGATGGTCTTTAGAGGA ATTCT (SEQ ID NO: 42) |
| rs9380525 | ACGTTGGATGGACAGAGTGAGACTC CTCTA (SEQ ID NO: 43) | ACGTTGGATGTTCTCAAACCAGCAC TCAGG (SEQ ID NO: 44) |
| rs6912833 | ACGTTGGATGGGAAAAGAATCAAG GGAAG (SEQ ID NO: 45) | ACGTTGGATGCTGGAACTAAGTGAT CTGTG (SEQ ID NO: 46) |
| rs2143404 | ACGTTGGATGGGTTAGGTAGAGCTC AGTTC (SEQ ID NO: 47) | ACGTTGGATGGTAGAGAACCTGGTA AGAAG (SEQ ID NO: 48) |
| rs1360780 | ACGTTGGATGAAGAGATCCAGGCAC AGAAG (SEQ ID NO: 5) | ACGTTGGATGTGCCAGCAGTAGCAA GTAAG (SEQ ID NO: 6) |
| rs1591365 | ACGTTGGATGGTGGCAAATAGGAGT TCTCC (SEQ ID NO: 49) | ACGTTGGATGTTGGCAGGTGTTTTT CTGAG (SEQ ID NO: 50) |
| rs7748266 | ACGTTGGATGAGACCACACCACAGA ACAAG (SEQ ID NO: 51) | ACGTTGGATGTTGCTTAACCCTCTC CAGAC (SEQ ID NO: 52) |
| rs9470069 | ACGTTGGATGATATACATACAGGCT GGCCG (SEQ ID NO: 53) | ACGTTGGATGACTCCTGACCTCGTG ATCTG (SEQ ID NO: 54) |
| rs6926133 | ACGTTGGATGGATGACTGATACTTC AGTCT (SEQ ID NO: 55) | ACGTTGGATGGAAAGCAAAGCTGAA AAGTAG (SEQ ID NO: 56) |
| ras3777747 | ACGTTGGATGCCACTCTTACATTCCT CTCC (SEQ ID NO: 57) | ACGTTGGATGTCCCTCTCCAAATCT CACTG (SEQ ID NO: 58) |
| rs4713899 | ACGTTGGATGAGTGGAGCTATAGGA GCTAG (SEQ ID NO: 59) | ACGTTGGATGACGGAAAGACTGCTG ATTGC (SEQ ID NO: 60) |
| rs2395634 | ACGTTGGATGTCGAAGGGACTTATT CCTCC (SEQ ID NO: 61) | ACGTTGGATGCAGCAGAAGGAAGAC ATCAG (SEQ ID NO: 62) |
| rs7753746 | ACGTTGGATGAGAATAAACCTGTGT TTCTG (SEQ ID NO: 63) | ACGTTGGATGAGGAGCAACCATATT TCTAA (SEQ ID NO: 64) |
| rs3800373 | ACGTTGGATGAAACCCCTAGTGTAG AAGAG (SEQ ID NO: 7) | ACGTTGGATGTTTACACTCCTCTATC ATGC (SEQ ID NO: 8) |
| rs10807151 | ACGTTGGATGGGGTGAAAGTGGCA GAACAA (SEQ ID NO: 65) | ACGTTGGATGCCGCTGGCTAATGAA AAAAC (SEQ ID NO:.66) |
| rs3800374 | ACGTTGGATGAGACGATGGACCCAT TTTAC (SEQ ID NO: 67) | ACGTTGGATGTCTTTTCCAAGTGGT GAACC '(SEQ ID NO: 68 |
| rs9348978 | ACGTTGGATGAATTTTTACCACTGAG CAGG (SEQ ID NO: 69) | ACGTTGGATGGCTTTGGCTTTACGG AAGAG (SEQ ID NO: 70) |
| rs11751447 | ACGTTGGATGATTCTCTCTTGAGTC GGTGC (SEQ ID NO: 71) | ACGTTGGATGTAGAACCTTGCCACA GAGAC (SEQ ID NO: 72) |
| rs2395631 | ACGTTGGATGTGCAAAAACTAACAA AAGCC (SEQ ID NO: 73) | ACGTTGGATGAGGGTTATCAACCTT GAGGC (SEQ ID NO: 74) |

In the primer extension assay according to the invention the target sequence annealed to a primer complementary to the region adjacent to the SNP site. Dideoxyribonucleotides (ddNTPS) and DNA polymerase are added to the mixture and the primer is extended by a single nucleotide. The single nucleotide added is dependent on the allele of the amplified DNA. Primer extension biochemistry can be coupled with a variety of detection schemes, comprising fluorescence, fluorescence polarization (FP), luminescence and mass spectrometry (MS).

Accordingly, in a preferred embodiment of the method of the invention, the primer extension assay uses a primer which hybridizes specifically with a portion of said gene or haplotype block which is adjacent to a polymorphism. The following table disposes the primers used for genotyping using single-base extension assays.

**Table 3**

| SNP-ID | Extension primer | |
|---|---|---|
| rs2766534 | CCGCCCTACACTTTCAC | (SEQ ID NO : 75) |
| rs4711429 | ATCTGTAACTTAGAGCCCTT | (SEQ ID NO : 76) |
| rs4713921 | CAGTTGAAAGAGCCTCAC | (SEQ ID NO: 77) |
| rs9462104 | CCTTAGGCATAACCACC | (SEQ ID NO: 78) |
| rs9394312 | TTCTCCCGAGGCTCCCA | (SEQ ID NO : 79) |
| rs4713916 | GACTCCTACATTTTCCTCT | (SEQ ID NO : 9) |
| rs943297 | CTGGACACAATTCCTTAGTTA | (SEQ ID NO : 80) |
| rs9380528 | AACTGAGATCATGCCACT | (SEQ ID NO: 81) |
| FKBP5UT5A | GGAAAGGGCTGCTCATCC | (SEQ ID NO : 82) |
| FKBP5UT5C | TCATTAACGCAGAAAAACAAA | (SEQ ID NO : 83) |
| rs9380526 | GAGGACAGACAAATGACT | (SEQ ID NO : 84) |
| rs10947563 | CAGTAATATGCTTGCTGTACC | (SEQ ID NO : 85) |
| rs9380525 | TTACTAGTGACATTCTAAAGGAG | (SEQ ID NO: 86) |
| rs6912833 | GATGCAAGAATAGCATGGATC | (SEQ ID NO : 87) |
| rs2143404 | GAGCTCAGTTCCCAGGG | (SEQ ID NO: 88) |
| rs1360780 | GGCTTTCACATAAGCAAAGTTA | (SEQ ID NO : 11) |
| rs1591365 | TGTCAGTTGTTTTTCCTTGAA | (SEQ ID NO : 89) |
| rs7748266 | GAACAAGAAACCAGTTGTATCA | (SEQ ID NO : 90) |
| rs9470069 | CTGTAATTCCAGCACTTTG | (SEQ ID NO: 91) |
| rs6926133 | AGTCTTTAAGTTTAATTGCAGGTC | (SEQ ID NO: 92) |
| rs3777747 | ATTCCTCTCCTTTCCAGC | (SEQ ID NO : 93) |
| ras4713899 | AGGCTCATCTGCATCTGTTAC | (SEQ ID NO: 94) |
| rs2395634 | TAGAATGTTCTTACACAAATCTAG | (SEQ ID NO : 95) |
| rs7753746 | GTGTTTCTGGTTTGTTACC | (SEQ ID NO : 96) |
| rs3800373 | AAGAGCAACTATTTATTTGTCAAC | (SEQ ID NO: 12) |
| rs10807151 | TGGCAGAACAATGCATC | (SEQ ID NO : 97) |
| rs3800374 | CCCATTTTACAGTTATGGGCTC | (SEQ ID NO : 98) |
| rs9348978 | CTGAGCAGGTGAAAAAT | (SEQ ID NO : 99) |
| rs11751447 | GGTGCCTCGTGCAGAGC | (SEQ ID NO: 100) |
| rs2395631 | TTTCATCTGATAGGCCCAAC | (SEQ ID NO: 101) |

Therefore, in a preferred embodiment of the method of the invention, the primer to be used for said primer extension assay has the sequence as set forth in SEQ ID NO: 75, 76, 77, 78, 79, 9, 80, 81, 84, 85, 86, 87, 88, 11, 89, 90, 91, 92, 93, 94, 95, 96, 12, 97, 98, 99, 100, or 101.

Further methods of analyzing a nucleic acid for the occurrence of polymorphisms are differential hybridization and allele-specific enzyme cleavage. The latter is also referred to as Restriction Fragment Length Polymorphism (RFLP).

Restriction Fragment Length Polymorphism (RFLP) is a technique in which species, strains, or, as envisaged by the inventors, polymorphic alleles may be differentiated by analysis of patterns derived from cleavage of their DNA. If two organisms differ in the distance between sites of cleavage of a particular restriction endonuclease, the length of the fragments produced will differ when the DNA is digested with a restriction enzyme.

In a preferred embodiment, the differential hybridization assay according to the invention or said assay detecting allele-specific enzyme cleavage according to the invention uses probes which hybridize specifically with a portion of said gene or haplotype block which comprises a polymorphism. The detection of allele-specific enzyme cleavage comprises (a) hybridizing said probes, preferably under stringent conditions, to nucleic acid molecules comprised in a sample taken from an individual; (b) digesting the product of said hybridization with a restriction endonuclease; and (c) analyzing the product of said digestion.

In a more preferred embodiment, said probes have the sequence as set forth in SEQ ID NO: 102 and 103; 104 and 105; 106 and 107; 108 and 109; 110 and 111; 13 and 14; 112 and 113; 114 and 115; 120 and 121; 122 and 123; 124 and 125; 126 and 127; 128 and 129; 17 and 18; 130 and 131; 132 and 133; 134 and 135; 136 and 137; 138 and 139; 140 and 141; 142 and 143; 144 and 145; 19 and 20; 146 and 147; 148 and 149; 150 and 151; 152 and 153; or 154 and 155. The sequences are shown in the table below.

**Table 4**

| SNP | **Column A** Probe for allele associated with improved response when present in a homozygous state | **Column B** Probe for allele associated with inferior response when present in a patient |
|---|---|---|
| rs2766534 | TTGAAAAGGGGCATGTGAAAGTGTAGGGC (SEQ ID NO: 102) | TTGAAAAGGGGCAGGTGAAAGTGTAGGGC (SEQ ID NO: 103) |
| rs4711429 | GGGAGCCTGTAAAGGGCTCTAAGTT (SEQ ID NO: 104) | GGGAGCCTGTGAAGGGCTCTAAGTT (SEQ ID NO: 105) |
| rs4713921 | TCCACTGAGATGTGAGGCTCTT (SEQ ID NO: 106) | TCCACTGAGACGTGAGGCTCTT (SEQ ID NO: 107) |
| rs9462104 | AGAAACTCTTAACGGTGGTTATG (SEQ ID NO: 108) | AGAAACTCTTAATGGTGGTTATG (SEQ ID NO: 109) |
| ras9394312 | CACCAAGATCAGTGGGAGCCTCG (SEQ ID NO: 110) | CACCAAGATCACTGGGAGCCTCG (SEQ ID NO: 111) |
| rs4713916 | ATTTTCCTCTATCTTGGTCCA (SEQ ID NO: 13) | ATTTTCCTCTGTCTTGGTCCA (SEQ ID NO: 14) |
| rs943297 | ATTCCTTAGTTAAGTCCTTGTTCTT (SEQ ID NO: 112) | ATTCCTTAGTTAGGTCCTTGTTCTT (SEQ ID NO: 113) |
| rs9380528 | GATCATGCCACTGTACTCCAGCC (SEQ ID NO: 114) | GATCATGCCACTATACTCCAGCC (SEQ ID NO: 115) |
| FKBP5UT5A | ACCTGGATGTCGGATGAGCAGC (SEQ ID NO: 116) | ACCTGGATGTGGGATGAGCAGC (SEQ ID NO: 117) |
| FKBP5UT5C | AGAAAAACAAAGTTGATCAAAAT (SEQ ID NO: 118) | AGAAAAACAAAATTGATCAAAAT (SEQ ID NO: 119) |
| rs9380526 | GGTTCTGTCTCAGTCATTTGTCT (SEQ ID NO: 120) | GGTTCTGTCTTAGTCATTTGTCT (SEQ ID NO: 121) |
| rs10947563 | TCTTCATGCCGGGTACAGC (SEQ ID NO: 122) | TCTTCATGCCAGGTACAGC (SEQ ID NO: 123) |
| rs9380525 | CAGGGTCTTCTGCTCCTTTAG (SEQ ID NO: 124) | CAGGGTCTTCTCCTCCTTTAG (SEQ ID NO: 125) |
| rs6912833 | TAGCATGGATCAGTTCATCAACTA (SEQ ID NO: 126) | TAGCATGGATCTGTTCATCAACTA (SEQ ID NO: 127) |
| rs2143404 | GAATCTTGAATGTCCCTGGGAACTG (SEQ ID NO: 128) | GAATCTTGAATGCCCCTGGGAACTG (SEQ ID NO: 129) |
| rs1360780 | AGCAAAGTTATACAAAACAAA (SEQ ID NO: 17) | AGCAAAGTTACACAAAACAAA (SEQ ID NO: 18) |
| rs1591365 | TTTTTCCTTGAAGTGACAGACTTA (SEQ ID NO: 130) | TTTTTCCTTGAAATGACAGACTTA (SEQ ID NO: 131) |
| rs7748266 | GTTGTATCATTCAGTATATAT (SEQ ID NO: 132) | GTTGTATCACTCAGTATATAT (SEQ ID NO: 133) |
| rs9470069 | TCCAGCACTTTGGGAGGCCAAGG (SEQ ID NO: 134) | TCCAGCACTTTGCGAGGCCAAGG (SEQ ID NO: 135) |
| rs6926133 | AATGTAGCCACGACCTGCAATT (SEQ ID NO: 136) | AATGTAGCCAAGACCTGCAATT (SEQ ID NO: 137) |
| rs3777747 | CTCCTTTCCAGCGCTATTATTG (SEQ ID NO: 138) | CTCCTTTCCAGCACTATTATTG (SEQ ID NO: 139) |
| rs4713899 | GCCCTGATTGCGGTAACAGATG (SEQ ID NO: 140) | GCCCTGATTGCAGTAACAGATG (SEQ ID NO: 141) |
| rs2395634 | CACAAATCTAGGTGGAACAGCCT (SEQ ID NO: 142) | CACAAATCTAGATGGAACAGCCT (SEQ ID NO: 143) |
| rs7753746 | GACAGTTTAGTGGGTAACAAACCA (SEQ ID NO: 144) | GACAGTTTAGTAGGTAACAAACCA (SEQ ID NO: 145) |
| rs3800373 | ATTTGTCAACCCTACAGATTT (SEQ ID NO: 19) | ATTTGTCAACACTACAGATTT (SEQ ID NO: 20) |
| rs10807151 | TTTTTAGCAACGATGCATTGTTC (SEQ ID NO: 146) | TTTTTAGCAATGATGCATTGTTC (SEQ ID NO: 147) |
| rs3800374 | TATGGGCTCTCAAAATTCA (SEQ ID NO: 148) | TATGGGCTCCCAAAATTCA (SEQ ID NO: 149) |
| rs9348978 | ATACTTATTTGAATTTTTCACCT (SEQ ID NO: 150) | ATACTTATTTGGATTTTTCACCT (SEQ ID NO: 151) |
| rs11751447 | CTCGTGCAGAGCCCCTCCTCTGCTG (SEQ ID NO: 152) | CTCGTGCAGAGCTCCTCCTCTGCTG (SEQ ID NO: 153) |
| rs2395631 | AGGCCCAACAGTATACCTTTGC (SEQ ID NO: 154) | AGGCCCAACGGTATACCTTTGC (SEQ ID NO: 155) |

Each of these probes recognizes one of the two alleles at the respective SNPs only. For example, in a hybridization experiment for the rs4713916 SNP, a hybridization signal with the probe in column A and no signal with the probe in column B would be indicative of a person with improved response to therapy, whilst a signal with both probes and with only the probe from column B would be indicative of inferior response. The same also applies to the other SNPs (rs2766534, rs4711429, rs4713921, rs9462104, rs9394312, rs943297, rs9380528, ras9380526, rs10947563, rs9380525, rs6912833, rs2143404, rs1360780, rs1591365, rs7748266, rs9470069, rs6926133, rs3777747, rs4713899, rs2395634, rs7753746, rs3800373, rs10807151, rs3800374, rs9348978, rs11751447 and, rs2395631).

In a more preferred embodiment, the probes used for the differential hybridization assay are immobilized on a supporting material. Probe sets, immobilized on a filter or solid support and arranged in arrayed form, are generally referred to as microarrays or DNA chips.

As described above, SNPs which correlate with response to therapy in patients suffering from depression, correlate also with FKBP51 expression levels. The latter correlation has been observed in healthy individuals. Therefore, the administration of antidepressant drugs does not account for the observed differences in FKBP51 expression level. Without being bound by a specific theory, it is assumed that SNPs in the FKBP51 locus are responsible for different FKBP51 expression levels. Therefore, the use of FKBP51 expression levels for classification and prognostic methods according to the invention is envisaged.

In a preferred embodiment, the FKBP51 expression level to be determined is the mRNA expression level. Methods for the determination of mRNA expression levels are known in the art and comprise Real Time RT-PCR, Northern blotting and hybridization on microarrays or DNA chips equipped with one or more probes or probe sets specific for FKBP51 transcripts.

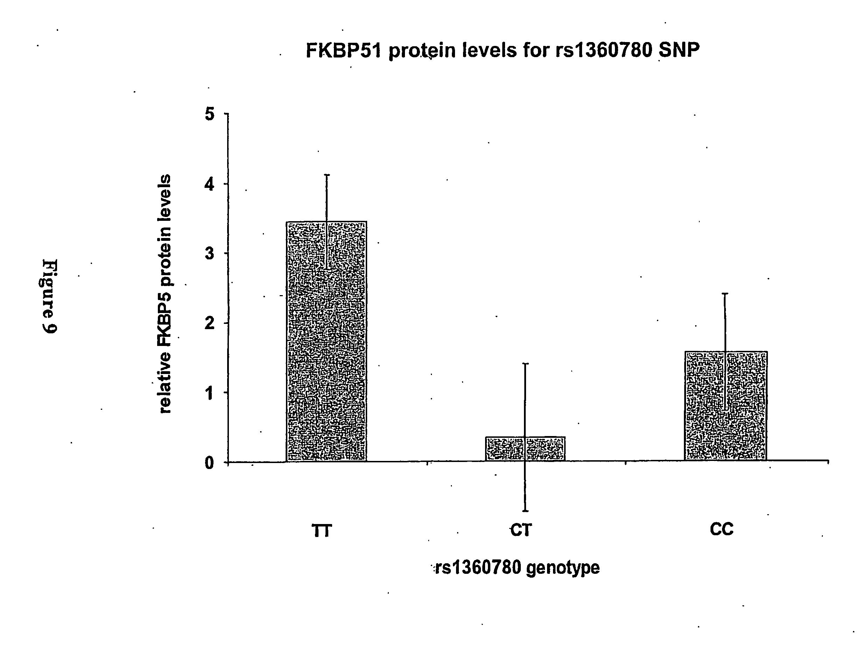

In a further preferred embodiment, the expression level to be determined is the protein expression level. The skilled person is aware of methods for the quantitation of proteins. Amounts of purified protein in solution can be determined by physical methods, e.g. photometry. Methods of quantifying a particular protein in a mixture rely on specific binding, e.g of antibodies. Specific detection and quantitation methods exploiting the specificity of antibodies comprise immunohistochemistry (in situ) and surface plasmon resonance. Western blotting combines separation of a mixture of proteins by electrophoresis and specific detection with antibodies. Example 1, sub-heading "Quantification of FKBP51 protein levels in lymphocytes", provides an example of how protein expression levels can be determined according to the invention. Example 4 and Figure 9 present and discuss results obtained by Western blotting and their implications for the methods according to the invention.

Described herein is also that the method of selecting an individual for a clinical trial further comprises the steps: (a) identifying a compound modulating the activity of FKBP51; and optionally (b) optimizing the pharmacological properties of the compound identified in (a); and (c) performing said clinical trials; wherein said clinical trials are clinical trials of the compound identified in (a) or optimized in (b). In this embodiment, the method of the invention may be used for the development of an antidepressant drug.

In principle, and regarding step (a), to change the activity of FKBP51 either protein level or protein function could be altered. To change protein level either promoter activity or protein stability could be altered.

The compound modulating the activity of FKBP51 may be an activator of FKBP51.

The compound modulating the activity of FKBP51 may increase the expression of FKBP51. The screen described below exemplifies the method for the identification of such compounds.

### Screen for compounds that alter the promoter activity of FKBP51

The screen may employ any of the assays described below.

Reporter gene assays employing transient transfection into various cells from yeast to mammals is a standard procedure. For FKBP51, the regulatory regions, i.e. gene promoter and enhancer have to be cloned upstream of the structural part of a gene encoding for a reporter (cf. e.g. Mühlhardt, Der Experimentator: Molekularbiologie, Gustav Fischer Verlag 1999), for example, β-galactosidase, firefly luciferase, renilla luciferase, a fluorescing protein (e.g. GFP, EGFP, EYFP etc.), human growth hormone, CAT (chloramphenicolacetyltransferase), TAT (tyrosylaminotransferase), alkaline phosphatase including SEAP, and peroxidase. Suitable eukaryotic cell lines include immortalized tumour cell lines, listed for example in the catalogues of ATCC and ETCC, in particular the cell lines HeLa, HEK, L929, NIH3T3, COS1, COS7, HepG2, H4-II-E-C3, Saos, K562, SK-N-MC, HT22, CV-1 preferably SK-N-MC or HEK cells. As an alternative to transient transfection assays, an in vitro transcription/translation system may be adapted, or stably transfected cell lines may be used accordingly. A number of methods are established to generate cell lines stably transfected with a reporter gene plasmid.

All assays have to be performed in the presence or absence of test compounds that comprise small molecules, peptides, aptamers and antibodies or fragments or derivatives thereof. An increase in expression of FKBP51 in the presence of a test compound identifies the compound as a hit.

This screen can be extended to modulators of the expression of FKBP52 by replacing the FKBP51 nucleic acid or amino acid sequence with the FKBP52 nucleic acid or amino acid sequence.

The compound modulating the activity of FKBP51 may enhance function and/or stability of FKBP51. The screen described below exemplifies the method for the identification of such compounds.

### Screen for compounds that bind to FKBP51 and change its function or stability

This screen is designed to first identify compounds that bind to FKBP51 and subsequently determine FKBP51-binding compounds that in addition have functional effects.

For the purpose of identifying FKBP51-binding compounds, for example a phage display library may be used. While using a phage display library allows to screen peptide compounds, the Biacore 3000 system is suitable for detection of any compound that binds to FKBP51 larger than 180 dalton. The Biacore system requires purified, human FKBP51, which can be from any source, preferably expressed in a bacterial expression system, preferably as a His-tagged protein. Purification of His-tagged protein is an established, standardized procedure and can be performed according to the manufacturers recommendation, e.g. Qiagen or Invitrogen. Any expression vector for recombinant proteins in bacteria can be used, preferably one that provides inducible expression, preferably the vector series pProExHTa (Invitrogen).

Any technique that attaches the purified FKBP51 protein to the Biacore sensor chip surface may be used. In particular, the use of NTA sensor chips is suitable for the His-tagged version of the recombinant FKBP51. Automatic screening of a large number of compounds can be performed according to established protocols by the Biacore manufacturer. Compounds that bind to FKBP51 will then be subjected to subsequent analyses to identify those with an effect on protein stability of FKBP51, on hormone binding of GR or on GR-dependent transcription.

For protein stability, mammalian cell lines like HeLa, HEK, L929, NIH3T3, COS1, COS7, HepG2, H4-II-E-C3, Saos, K562, SK-N-MC, HT22, CV-1 preferably SK-N-MC or HEK cells, are treated for different time intervals from 0h to 48h, eventually up to several weeks with the compound and levels FKBP51 are assessed by standard western blotting techniques on an Imaging System, preferably an imaging station like Kodak 440CF.

To determine hormone binding affinity, a number of different protocols are available. Binding affinity can be measured in tissues, in cell extracts, or in a partially purified extract of the glucocorticoid receptor, which needs to be supplemented with recombinant FKBP51. A recombinant, partially purified human glucocorticoid receptor expressed in the Baculovirus system is available from Panvera.

Assays for determining glucocorticoid receptor-dependent gene transcription are well known in the art, see for example Herr et al., Mol.Pharmacol. 57 (2000), 732-737; Hollenberg et al., Cancer Res. 49 (1989), 2292s-2294s; Kullmann et al., J.Biol.Chem. 273 (1998), 14620-14625; Rupprecht et al., Eur. J. Pharmacol. 247 (1993), 145-154. These assays may be performed using one of the transient transfection methods or in stably transfected cell lines.

This screen can be extended to FKBP52 binders and modulators by replacing the FKBP51 nucleic acid or amino acid sequence with the FKBP52 nucleic acid or amino acid sequence.

Methods for the optimization of the pharmacological properties of compounds identified in screens, generally referred to as lead compounds, are known in the art and comprise a method of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiozolidines or combinations thereof; said method optionally further comprising the steps of the above described methods.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Clinical trials according to the invention can for example be performed as described in the following protocol.

### Protocols for clinical trials

Response to treatment by an antidepressant, time until response (i.e. latency) to an antidepressant, or patient outcome to an antidepressant treatment protocol can be predicted by determining in patients the presence of at least one of the FKBP51 SNPs disclosed in Table 1. Accordingly, an individual homozygous for the TT allele of rs2766534, or the AA allele of rs4711429, or the TT allele of rs4713921, or the CC allele of rs9462104, or the GG allele of rs9394312, or the AA allele of rs4713916, or the AA allele of rs943297, or the GG allele of rs9380528, or the CC allele of rs9380526, or the GG allele of rs10947563, or the GG allele of rs9380525, or the AA allele of rs6912833, or the TT alllele of rs2143404, or the TT allele of rs1360780, or the GG allele of rs1591365, or the TT allele of rs7748266, or the GG allele of rs9470069, or the CC allele of rs6926133, or the GG allele of rs3777747, or the GG allele of rs4713899 or the GG allele of rs2395634, or the GG allele of rs7753746, or the CC allele of rs3800373, or the CC allele of rs10807151, or the TT allele of rs3800374, or the AA allele of rs9348978, or the CC allele of rs11751447, or the AA alleles of rs2395631, and optionally with elevated FKBP51 expression or protein levels would have an improved, or quicker response to an antidepressant drug. Patients should have at least one of these geneotypes or alleles, but may have two, three, or any combination or number of all 28 SNPs.

Individuals with either a GG or TG allele of rs2766534, a GG or AG allele of rs4711429, a CC or TC allele of rs4713921, a TT or CT allele of rs9462104, a CC or GC allele of rs9394312, or the a GG or AG allele of rs4713916, a GG or AG allele of rs943297, a AA or GA allele of rs9380528, a TT or CT allele of rs9380526, a AA or GA allele of rs10947563, a CC or GC allele of rs9380525, a TT or AT allele of rs6912833, a CC or TC alllele of rs2143404, a CC or CT allele of rs1360780, a AA or GA allele of rs1591365, a CC or TC allele of rs7748266, a CC or GC allele of rs9470069, a AA or CA allele of rs6926133, a AA or GA allele of rs3777747, a AA or GA allele of rs4713899, a AA or GA allele of rs2395634, a AA or GA allele of rs7753746, a AA or AC allele of rs3800373, aTT or CT allele of rs10807151, a CC or CT allele of rs3800374, a GG or AG allele of rs9348978, a TT or CT allele of rs11751447, a GG or AG allele of rs2395631, and optionally low FKBP51 expression or low FKBP51 protein level would have an inferior, or slower response to an antidepressant drug. The various alleles are summarised in the following table.

**Table 5**

| SNP | Column A Allele associated with improved response | Column B Alleles associated with inferior response |
|---|---|---|
| rs2766534 | TT | GG or TG |
| rs4711429 | AA | GG or AG |
| rs4713921 | TT | CC or TC |
| rs9462104 | CC | TT or CT |
| rs9394312 | GG | CC or GC |
| rs4713916 | AA | GG or AG |
| rs943297 | AA | GG or AG |
| rs9380528 | GG | AA or GA |
| FKBP5UT5A | CC or CG or GG | -- |
| FKBP5UT5C | GG or GA or AA | -- |
| rs9380526 | CC | TT or CT |
| rs10947563 | GG | AA or GA |
| rs9380525 | GG | CC or GC |
| rs6912833 | AA | TT or AT |
| rs2143404 | TT | CC or TC |
| rs1360780 | TT | CC or CT |
| rs1591365 | GG | AA or GA |
| rs7748266 | TT | CC or TC |
| rs9470069 | GG | CC or GC |
| rs6926133 | CC | AA or CA |
| rs3777747 | GG | AA or GA |
| rs4713899 | GG | AA or GA |
| rs2395634 | GG | AA or GA |
| rs7753746 | GG | AA or GA |
| rs3800373 | CC | AA or AC |
| rs10807151 | CC | TT or CT |
| rs3800374 | TT | CC or TC |
| rs9348978 | AA | GG or AG |
| rs11751447 | CC | TT or CT |
| rs2395631 | AA | GG or AG |

In a clinical trial for a new antidepressant drugs, patients would be grouped or selected according to the presence of one or more of the alleles found in column A. In addition, patients could be selected according to elevated FKBP51 levels. Such patients would then be enrolled into a clinical trial for testing the efficacy of a new antidepressant drug where the endpoint would be improvement in the Hamilton Depression Rating score, dexamethasone suppression test, or combined dexamethasone - corticotropin releasing hormone test. Results obtained with this group of patients could be compared to a second group of patients which possess one or more of the alleles found in column B or a group of patients with low FKBP51 expression.

Described herein is also a method of classifying patients suffering from depression which further comprises the step of treating said patient with an antidepressant.

Furthermore described is also a nucleic acid molecule comprising the sequence of SEQ ID NO: 116 or/and 118. This embodiment relates to sequences comprising the SNP(s) designated FKBP5UT5A or/and FKBP5UT5C and disclosed herein for the first time. All sequences comprising the FKBP51 locus or parts thereof and optionally adjacent regions are encompassed, provided they comprise the SNP(s) defined by SEQ ID NO: 116 or/and 118. The remainder of the comprised sequence from the FKBP51 locus and optionally adjacent regions may be wildtype sequence throughout, or may exhibit one or any combination of more than one of the polymorphisms disclosed herein and/or described in the art. It is understood that the wording "comprising the SNP(s) defined by SEQ ID NO: 116 or/and 118" as used herein refers to sequences comprising the polymorphic nucleotide site(s) in SEQ ID NO: 116 or/and 118. Such sequences may comprise SEQ ID NO: 116 or/and 118 in their entirety, but may also comprise only part thereof, with the proviso that the polymorphic site is present. Accordingly, also sequences beginning or ending with the polymorphic site in SEQ ID NO: 116 or 118 are included.

In view of the favorable response to therapy associated with elevated levels of FKBP51, described is also a pharmaceutical composition comprising (a) a nucleic acid encoding a polypeptide with the sequence of SwissProt accession number Q13451; (b) a nucleic acid hybridizing to the complementary strand of the nucleic acid of (a) and encoding a polypeptide having the biological activity of the polypeptide with the sequence of SwissProt accession number Q13451; and/or (c) a polypeptide encoded by the nucleic acid of (a) or (b).

Said nucleic acids are provided in a formulation and to be administered via a route which ensures their expression.

said hybridizing may occur under stringent conditions.

SwissProt (http://us.expasy.org/) is a manually curated database of annotated protein sequences. The recited identifier (Q13451) refers to the SwissProt entry for human FK506-binding protein 51 (FKBP51), sometimes also referred to as FKBP5, FKBP54 or AIG6. FKBP51 acts as a peptidyl-prolyl cis-trans isomerase (rotamase).

Described is also the use of (a) a nucleic acid encoding a polypeptide with the sequence of SwissProt accession number Q13451; (b) a nucleic acid hybridizing to the complementary strand of the nucleic acid of (a) and encoding a polypeptide having the biological activity of the polypeptide with the sequence of SwissProt accession number Q13451; (c) a polypeptide encoded by the nucleic acid of (a) or (b); (d) an activator of the expression of the polypeptide with the sequence of SwissProt accession number Q13451; and/or (e) an activator of the polypeptide with the sequence of SwissProt accession number Q13451 for the manufacture of a pharmaceutical composition for the treatment of depression. Said activator of expression can be obtained by the above described screen for compounds that alter the promoter activity of FKBP51. Said activator of the polypeptide can be identified by the above described screen for compounds that bind FKBP51 and change its function or stability.

Also described herein is a method of treating depression comprising the administration of (a) a nucleic acid encoding a polypeptide with the sequence of SwissProt accession number Q13451; (b) a nucleic acid hybridizing to the complementary strand of the nucleic acid of (a) and encoding a polypeptide having the biological activity of the polypeptide with the sequence of SwissProt accession number Q13451; (c) a polypeptide encoded by the nucleic acid of (a) or (b); (d) an activator of the expression of the polypeptide with the sequence of SwissProt accession number Q13451; and/or (e) an activator of the polypeptide with the sequence of SwissProt accession number Q13451 to a patient suffering from depression.

Recent studies have shown that FKBP51 and FKBP52 are physiologically relevant regulators of glucocorticoid receptor (GR) sensitivity. In the absence of steroids, FKBP51 interacts with the GR complex to retain it in the cytoplasm where it is inactive. Upon steroid binding, FKBP51 is replaced by FKBP52 which results in a recruitment of dynein into the GR complex, allowing nuclear translocation of the complex and transcriptional activity of the glucocorticoid receptor (Davies et al., J. Biol. Chem. 227 (2002), 4597-4600).

Thus, inhibitors of FKBP52 may have antidepressant effects by allowing FKBP51 to remain in contact with the GR complex and keeping the glucocorticoid receptor in an cytoplasmic inactive form despite steroid binding. The term "inhibitor of FKBP52" refers to substances inhibiting the biochemical or catalytic activity and/or the biological activity of FKBP52. The latter class of substances comprises compounds interfering with the interaction between FKBP52 and its interaction partners. Said interaction partners comprise components of the GR complex, including heat shock proteins such as Hsp90.

There are several examples of known or recognised FKBP52 inhibitors described in the art. Furthermore, inhibitors of immunophilins (or pepidyl-prolyl cistransisomerases or rotamases) in general or of any specific immunophilin other than FKBP52 is a potential inhibitor of FKBP52. Whether such substance does inhibit FKBP52 can be assayed with the screens or assays described above.

A screening method to identify inhibitors of FKBP52 binding to partner proteins such as Hsp90 can be performed using bioluminescence resonance energy transfer (BRET). Briefly, the FKBP52 protein is fused to Renilla luciferase, whereas the Hsp90 protein is fused to a fluorescent protein, for example the yellow fluorescent protein (YFP). The emission of the luciferase signal is detected following addition of its substrate coelenterazine. When FKBP52 binds to Hsp90, a resonance energy transfer occurs between the luciferase and the YFP resulting in the detection of both the luciferase and the YFP signals. An inhibitor compound preventing binding of FKBP52 to Hsp90 would result in the emission of the luciferase signal only. The use of BRET for drug screening is well known in the art, see for example Xu, Y. et al. (1999) A bioluminescence resonance energy transfer (BRET) system: application to interacting circadian clock proteins. Proc. Natl. Acad. Sci. U. S. A. 96: 151-156; Boute et al. (2002) The use of resonance energy transfer in high-throughput screening: BRET versus FRET. Trends Pharmacol. Sci. 23:351-354.

Identification of inhibitors of FKBP52 protein binding can also be done using the CheckMate^{™} Mammalian Two-Hybrid System from Promega or fluorescence resonance energy transfer (FRET). Such methods are well known in the art, see for example Serebriiskii et al. (2002) Detection of peptides, proteins, and drugs that selectively interact with protein targets. Genome Res. 12:1785-1791; Bergendahl et al. (2003) Luminescence resonance energy transfer-based high-throughput screening assay for inhibitors of essential protein-protein interactions in bacterial RNA polymerase. Appl. Environ. Microbiol. 69:1492-1498.

International Patent Applications WO 98/13343, WO 98/13355, WO 98/20891, WO 98/20892 and WO 98/20893 describe various pyrrolidine, piperidine and homopiperidine derivatives having an acyl, amide, oxalyl, or similar linking group, at the 1-position of the heterocycle.

US 5,721,256 describes various pyrrolidine, piperidine and homopiperidine derivatives having an SO₂ linking group at the 1-position of the heterocycle.

US 6,166,011 and US 6,509,464 disclose compounds that inhibit FKBP12 and FKBP52 without inhibiting the protein phosphatase calcineurin and therefore lack any significant immunosuppressive activity.

WO 99/21552 discloses an FKBP52 antibody and geldanamycin derivatives that can disrupt and inhibit the association between FKBP52 and Hsp90.

Furthermore described is a pharmaceutical composition containing a recognised inhibitor of FKBP52.

Also described is the use of (a) geldanamycin or a geldanamycin derivative that can disrupt and inhibit the association between FKBP52 and Hsp90; and/or (b) an antibody or an aptamer specifically recognizing FKBP52 or a fragment or epitope thereof for the manufacture of a pharmaceutical composition for the treatment of depression. Suitable geldanamycin derivatives or analogs can be identified by the screens described above.

Said inhibitor may be an antibody or an aptamer specifically recognizing FKBP52 or a fragment or epitope thereof. Said antibody may be a monoclonal or a polyclonal antibody.

Said antibody, which is monoclonal antibody, polyclonal antibody, single chain antibody, or fragment thereof that specifically binds FKBP52 also includes a bispecific antibody, synthetic antibody, antibody fragment, such as Fab, a F(ab₂)', Fv or scFv fragments etc., or a chemically modified derivative of any of these (all comprised by the term "antibody"). Monoclonal antibodies can be prepared, for example, by the techniques as originally described in Köhler and Milstein, Nature 256 (1975), 495, and Galfré, Meth. Enzymol. 73 (1981), 3, which comprise the fusion of mouse myeloma cells to spleen cells derived from immunized mammals with modifications developed by the art. Furthermore, antibodies or fragments thereof to the FKBP52 can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of FKBP52 (Schier, Human Antibodies Hybridomas 7 (1996), 97-105; Malmborg, J. Immunol. Methods 183 (1995), 7-13). The production of chimeric antibodies is described, for example, in WO89/09622. A further source of antibodies to be utilized in accordance with the present invention are so-called xenogenic antibodies. The general principle for the production of xenogenic antibodies such as human antibodies in mice is described in, e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook (1989), loc. cit..

The term "monoclonal" or "polyclonal antibody" (see Harlow and Lane, (1988), loc. cit.) also relates to derivatives of said antibodies which retain or essentially retain their binding specificity. Whereas particularly preferred embodiments of said derivatives are specified further herein below, other preferred derivatives of such antibodies are chimeric antibodies comprising, for example, a mouse or rat variable region and a human constant region.

The term "scFv fragment" (single-chain Fv fragment) is well understood in the art and preferred due to its small size and the possibility to recombinantly produce such fragments.

The term "specifically binds" in connection with the antibody used in accordance with the present invention means that the antibody etc. does not or essentially does not cross-react with (poly)peptides of similar structures. Cross-reactivity of a panel of antibodies etc. under investigation may be tested, for example, by assessing binding of said panel of antibodies etc. under conventional conditions (see, e.g., Harlow and. Lane, (1988), loc. cit.) to the (poly)peptide of interest as well as to a number of more or less (structurally and/or functionally) closely related (poly)peptides. Only those antibodies that bind to the (poly)peptide/protein of interest but do not or do not essentially bind to any of the other (poly)peptides which are preferably expressed by the same tissue as the (poly)peptide of interest, are considered specific for the (poly)peptide/protein of interest and selected for further studies in accordance with the invention.

Described is also a method of treating depression comprising the administration of (a) geldanamycin or a geldanamycin derivative that can disrupt and inhibit the association between FKBP52 and Hsp90; and/or (b) an antibody or an aptamer specifically recognizing FKBP52 or a fragment or epitope thereof to a patient suffering from depression.

The Figures show:
**Figure 1****.** Representation of linkage disequilibrium (LD) structure using D' in the FKBP51 region. The 4 SNPs of the invention are labelled with a red arrow. The quantity D' (D prime) measures the amount of linkage disequilibrium between alleles at two loci. It is defined as the difference between an observed haplotype frequency and its expected value under the assumption of no disequilibrium. This difference is then divided by its theoretical maximum. The range of D' therefore goes from 0, indicating no linkage disequilibrium to 1, indicating maximum disequilibrium. D' is defined and first published by Lewontin in 1964²⁷.
**Figure 2****.** Association of SNPs in the FKBP51 gene region and response to antidepressant drugs at week 2. The -log p values (y axis) of the association were plotted against the physical location of the SNPs (x axis) on chromosome 6 according to version hg15 of the UCSC genome draft.

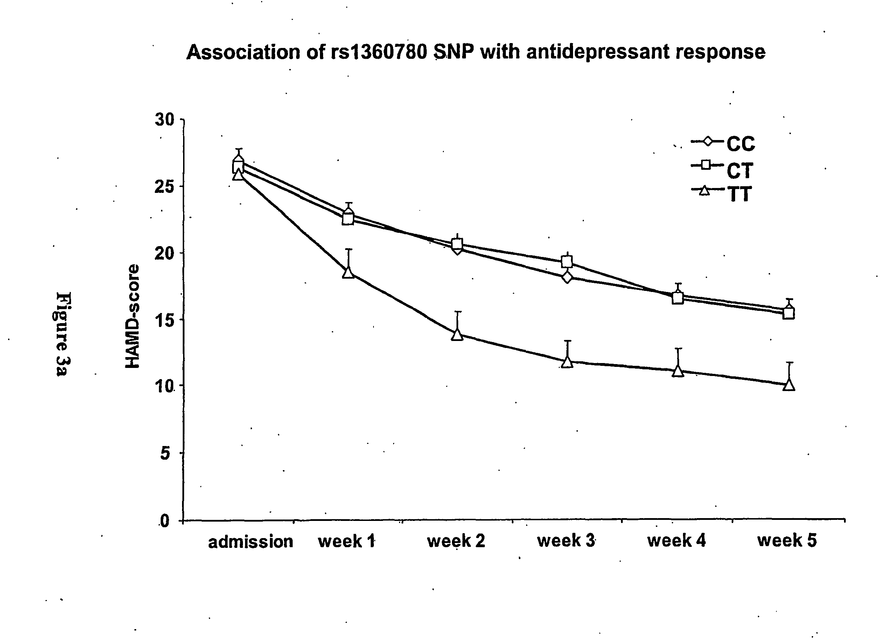

**Figure 3a****.** Hamilton Depression Rating Scale (HAM-D) scores over the first 5 weeks of hospitalisation according to the rs1360780 genotype with all patients. A repeated measures ANOVA showed an overall significant genotype effect: F_{2,197}= 5.3, p = 0.0058. Patients did not differ in disease severity at admission: mean HAM-D score at admission (SD) for CC = 26.2 (7.3); CT = 25.7 (8.1); TT = 26.2 (7.2). A one way ANOVA showed a non-significant genotype effect for differences in HAM-D scores at admission: F_{2,277} = 0.15, p = 0.86.

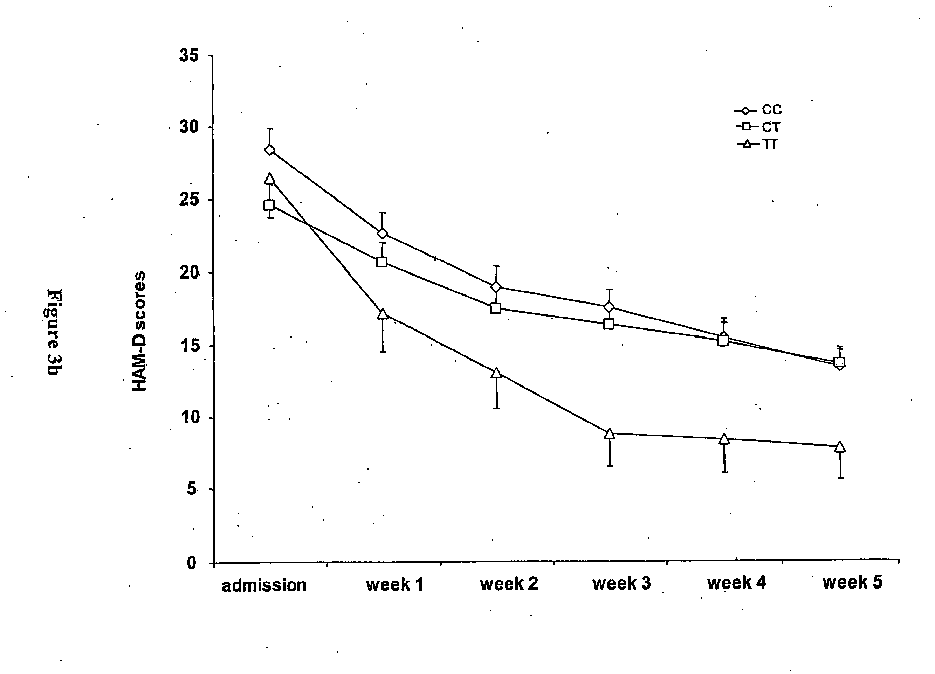

**Figure 3b****.** Hamilton Depression Rating Scale (HAM-D) scores over the first 5 weeks of hospitalisation according to the rs1360780 genotype with patients treated with selective serotonin reuptake inhibitors (N = 70). A repeated measures ANOVA shows a significant interaction of rs1360780 genotype and change in HAM-D scores, F_{4.3,170.5} (Greenhouse-Geisser) = 2.67; p = 0.03.

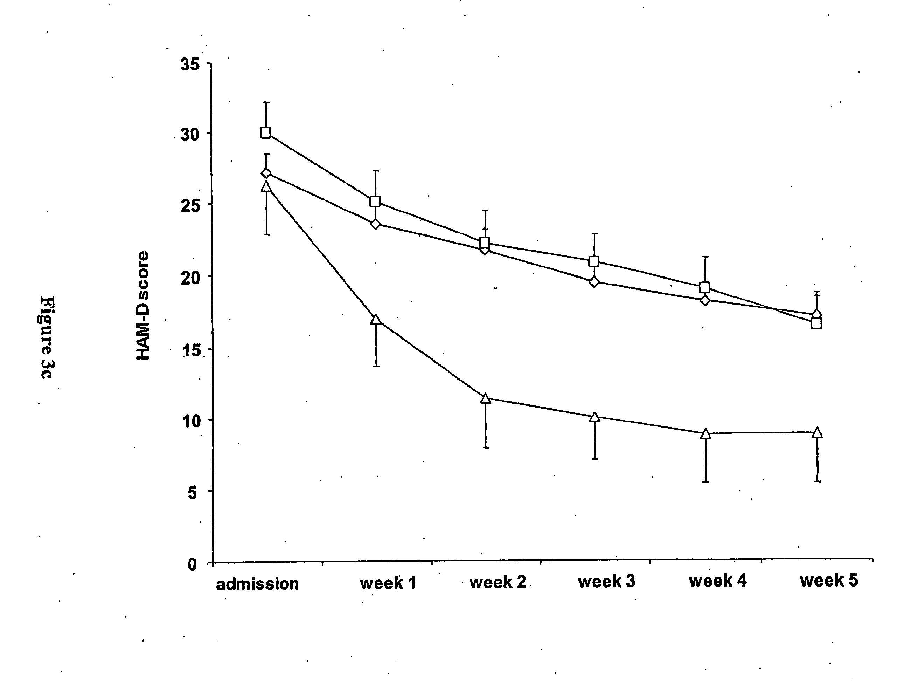

**Figure 3c****.** Hamilton Depression Rating Scale (HAM-D) scores over the first 5 weeks of hospitalisation according to the rs1360780 genotype with patients treated with tricyclic antidepressants (N = 48). A repeated measures ANOVA shows a significant effect of rs1360780 genotype on change in HAM-D scores, F_{2,45} = 3.43; p = 0.04.

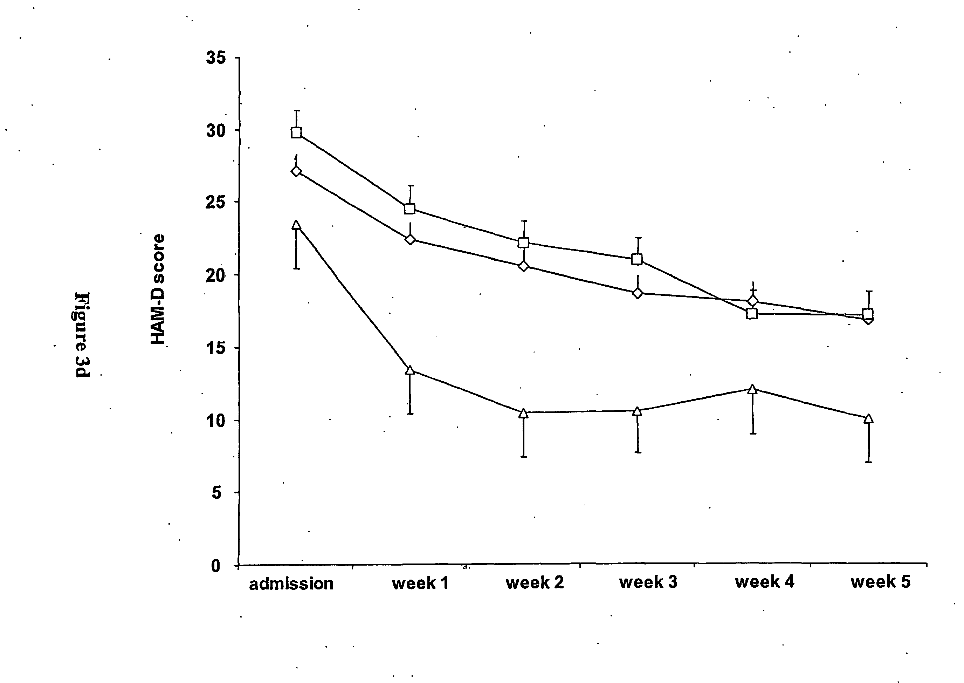

**Figure 3d****.** Hamilton Depression Rating Scale (HAM-D) scores over the first 5 weeks of hospitalisation according to the rs1360780 genotype with patients treated with mirtazapine (N = 55). A repeated measures ANOVA shows a significant effect of rs1360780 genotype on change in HAM-D scores, F_{2,52}= 4.2; p = 0.02.
**Figure 4****.** Effect of rs1360780 genotype on ACTH response in the Dex-CRH test at admission and discharge for all patients. A t-test showed that peak ACTH values (T = 3.48; df = 95.8; p = 0.00075) and area under the curve of the ACTH response (T = 1.92; df = 98.1; p = 0.056) were significantly lower in the TT genotype vs. the two other genotypes. The cortisol response at admission and the ACTH and cortisol responses at discharge were not significantly different between these two groups.
**Figure 5****.** Number of previous depressive episodes and rs1360780 genotype for all patients.

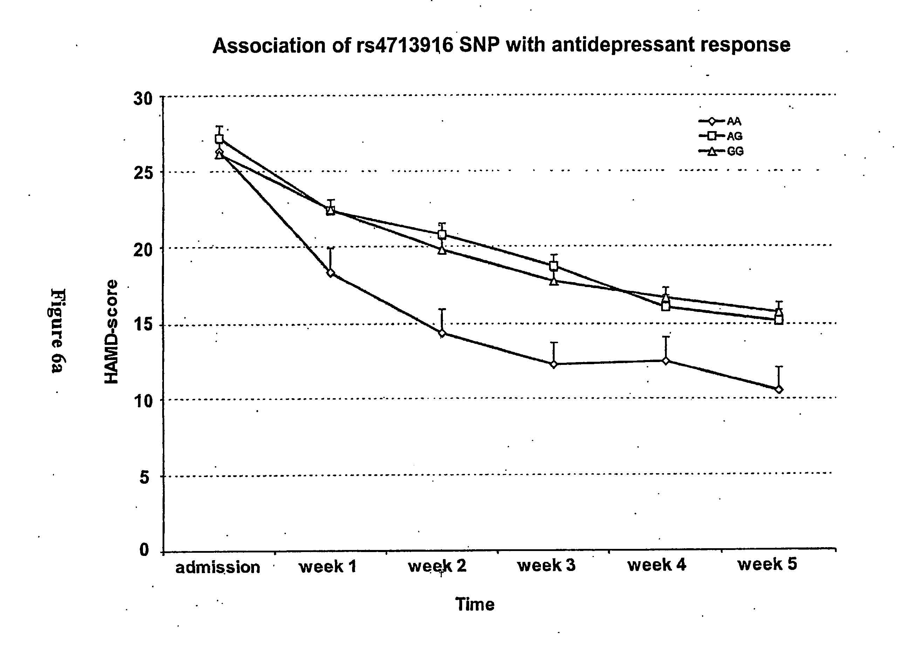

**Figure 6a****.** HAM-D scores over the first 5 weeks of hospitalisation against rs4713916 genotypes. A repeated measures ANOVA showed an overall significant genotype effect: F2,125 = 4.75, p = 0.012 and interaction between response and genotype: F4.761,511.812 = 2.6, p = 0.03. Patients did not differ in disease severity at admission: mean HAM-D score at admission (SD) for AA = 26.3 (7.0); AG = 27.4 (8.3); GG = 26.8 (7.6). A one-way ANOVA showed a non-significant genotype effect for differences in HAM-D scores at admission: F2,436, = 0.40, p = 0.67.

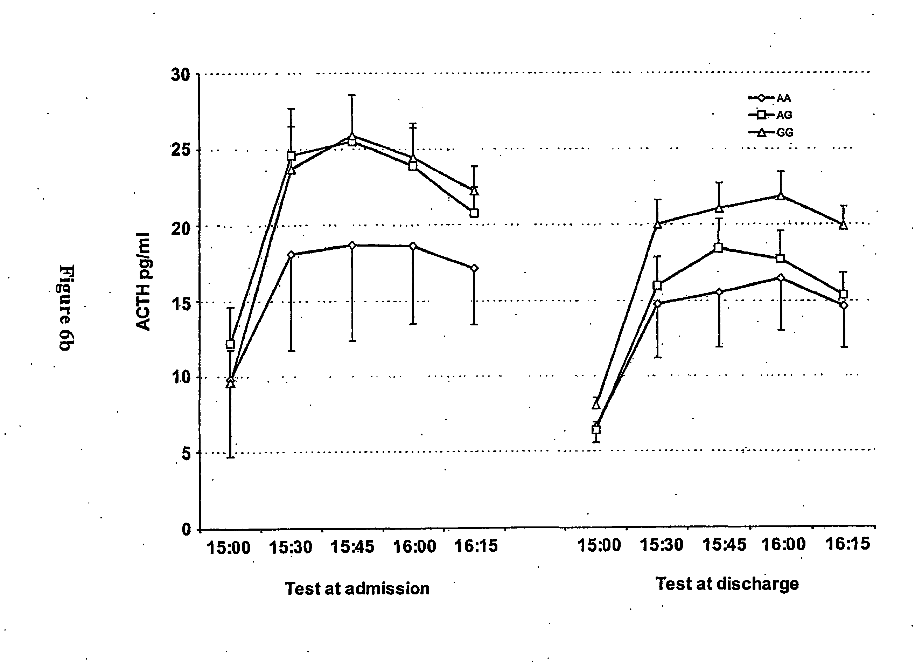

**Figure 6b****.** Effect of rs4713916 genotype on ACTH response in the Dex-CRH test at admission and discharge for all patients. Results show a tendency for lower ACTH response in the AA genotype vs. the two other genotypes.

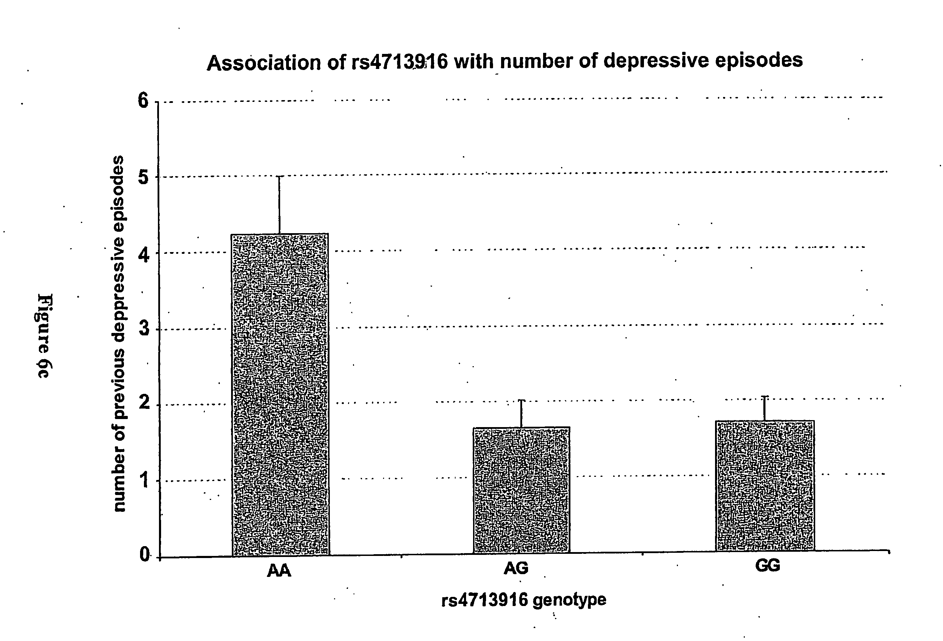

**Figure 6c****.** Number of previous depressive episodes and rs4713916 genotype for all patients.

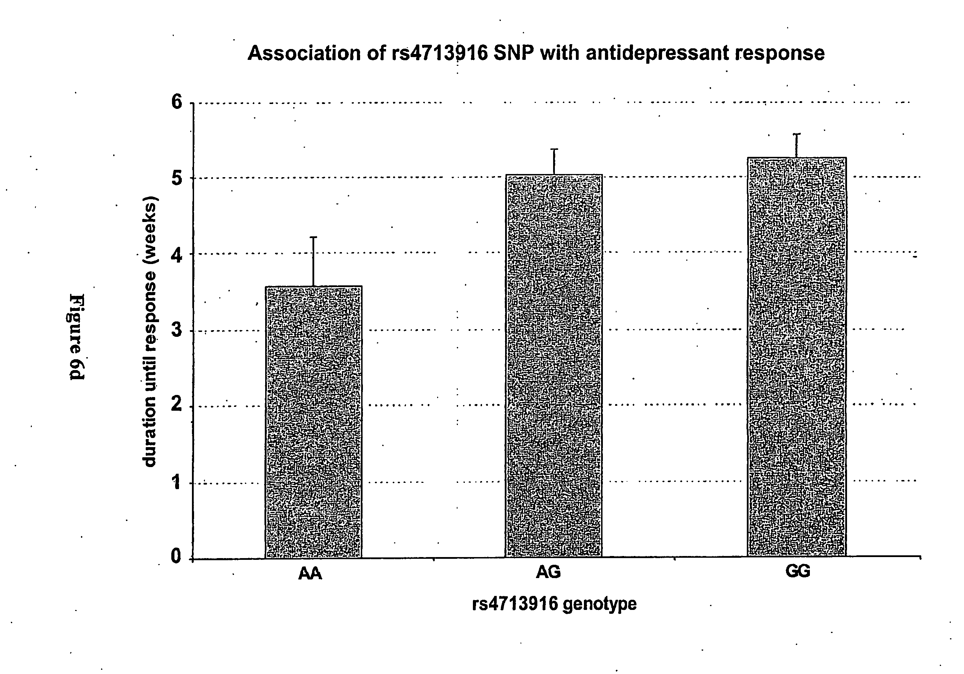

**Figure 6d****.** Duration of treatment with antidepressants until response for each rs4713916 genotype for all patients.

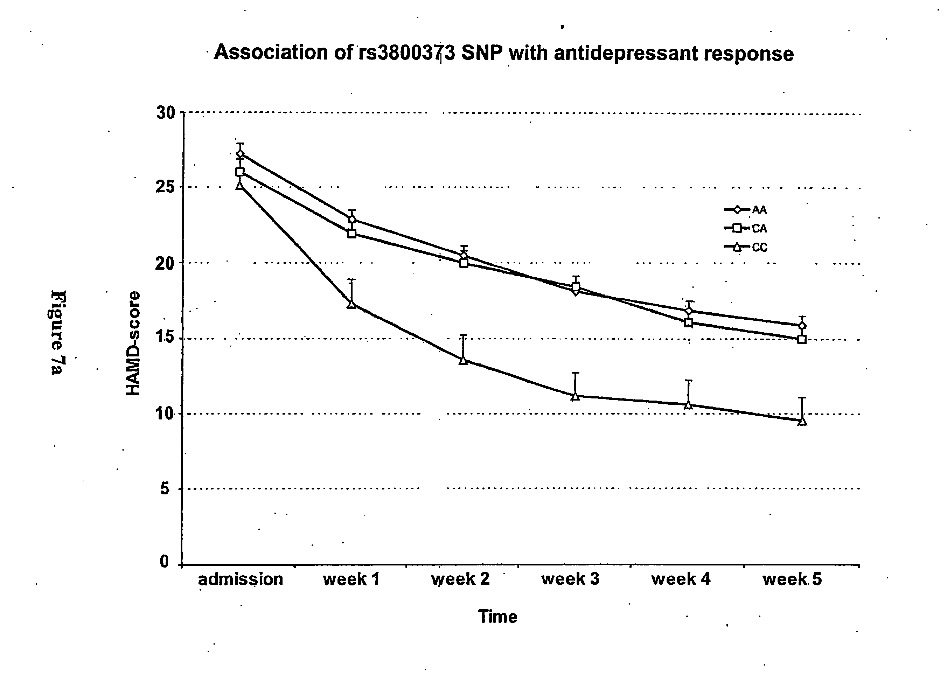

**Figure 7a****.** HAM-D scores over the first 5 weeks of hospitalisation against rs3800373 genotypes. A repeated measures ANOVA showed an overall significant genotype effect: F2,214= 7.76, p < 0.001 and interaction between response and genotype: 2,214 = 4.5, p = 0.012. Patients did not differ in disease severity at admission: mean HAM-D score at admission (SD) for CC = 25.6 (6.9); CA = 26.0 (8.2); AA = 27.9 (7.3). A one-way ANOVA showed a non-significant genotype effect for differences in HAM-D scores at admission: F2,435 = 0.38, p = 0.686.

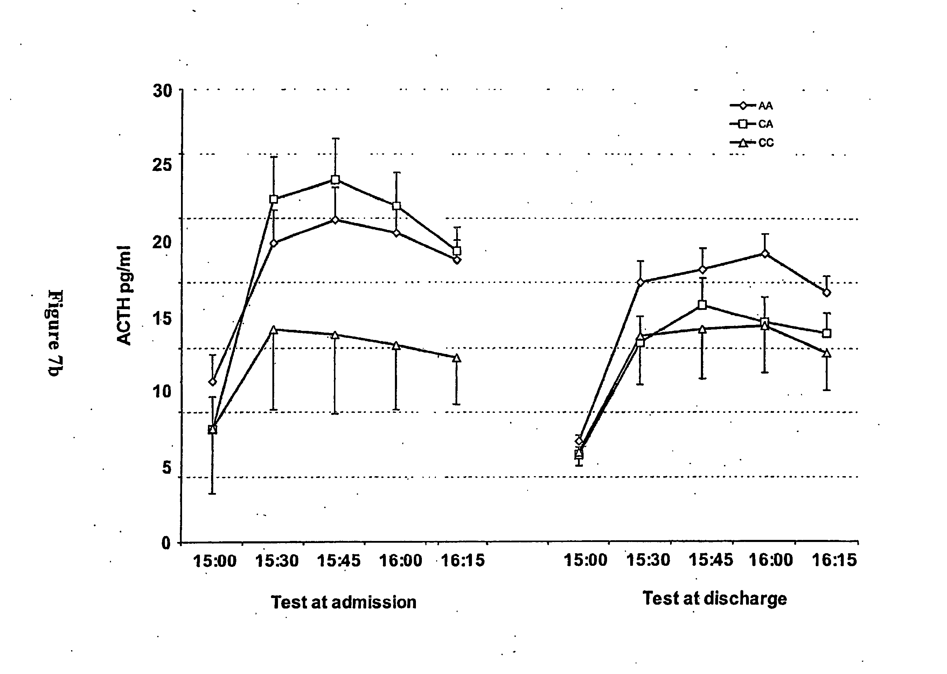

**Figure 7b****.** Effect of rs3800373 genotype on ACTH response in the Dex-CRH test at admission and discharge for all patients. A t-test showed that the area under the curve of the ACTH response (T = 2.273; df = 38.746; p = 0.029) was significantly lower in the AA genotype vs. the two other genotypes. Peak ACTH values were also lower in the AA genotype than the two other genotypes (p < 0.07). The cortisol response at admission and the ACTH and cortisol responses at discharge were not significantly different between these two groups.

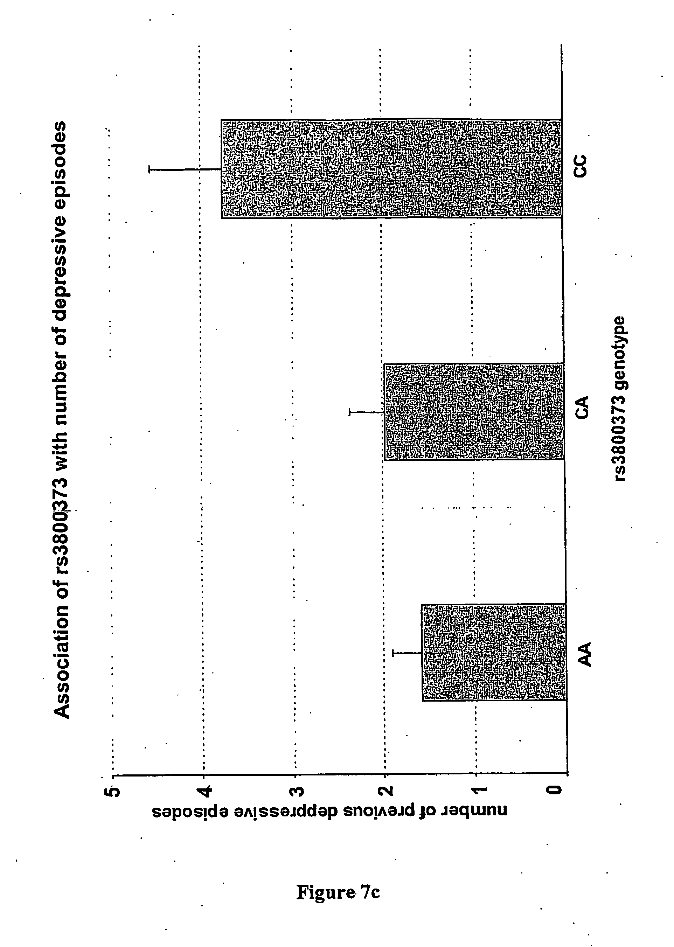

**Figure 7c****.** Number of previous depressive episodes and rs3800373 genotype for all patients.

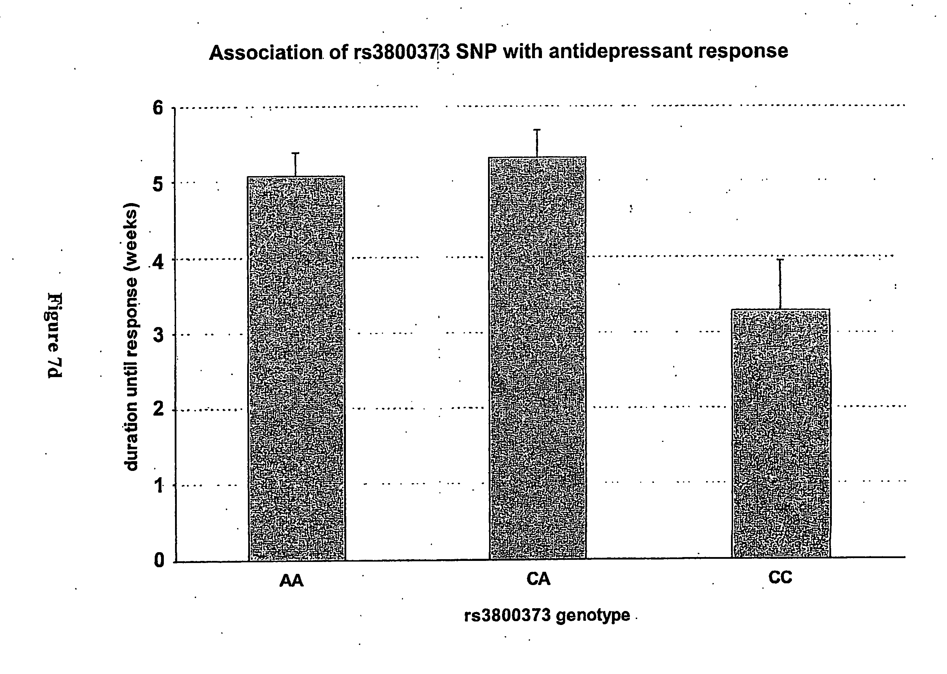

**Figure 7d****.** Duration of treatment with antidepressants until response for each rs3800373 genotype for all patients.

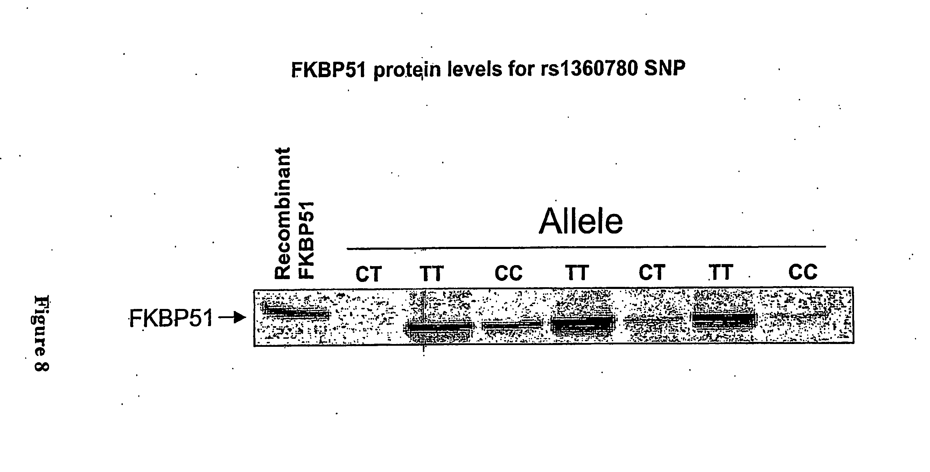

**Figure 8****.** Western blot of FKBP51 protein for the various genotype of the rs1360780 SNP.
**Figure 9****.** FKBP51 protein levels for the various genotype of the rs1360780 SNP. Quantitative difference in FKBP51 protein expression levels in lymphocytes according to rs1360780 genotypes (mean ± SEM). The data represents the average values of two independent replications of the western blot analysis. A one-way ANOVA with patient status and plasma cortisol levels as covariates showed a significant genotype effect (TT vs. two other genotypes) for differences in FKBP51 levels: F_{1,25} = 5.96; p = 0.024. Patient status (F_{1,25} = 0.35; p = 0.55) and plasma cortisol levels (F_{1,25} = 0.01; p = 0.95) had no significant influence on FKBP51 levels, Controls: N = 18, patients: N = 7; TT: N = 12, CT: N = 5, CC: N = 8. rec = recombinant FKBP51.

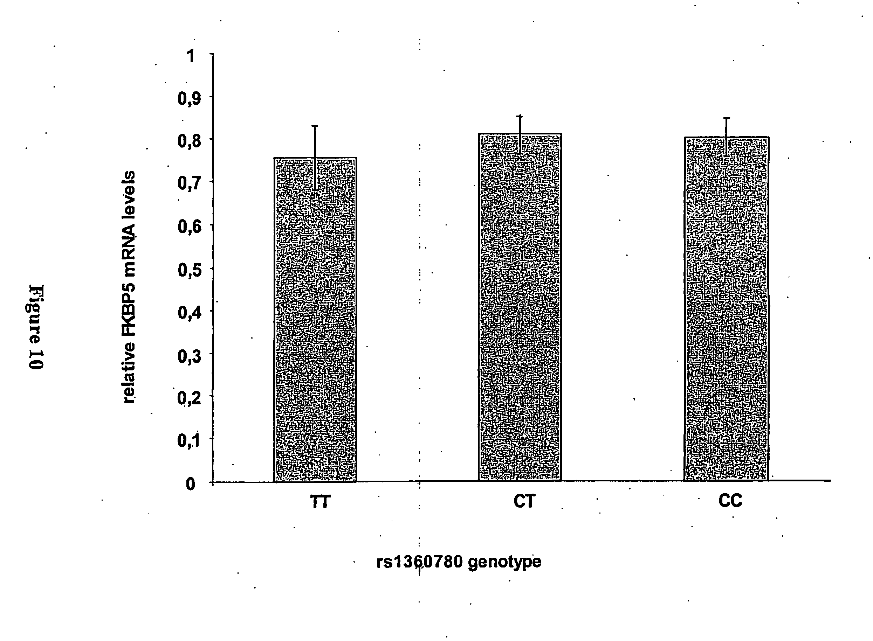

**Figure 10****.** Quantitative difference in FKBP51 mRNA expression in peripheral blood monocytes of healthy controls according to the rs1360780 genotypes (mean ± SEM). A one way ANOVA revealed no significant genotype effect (N = 124).

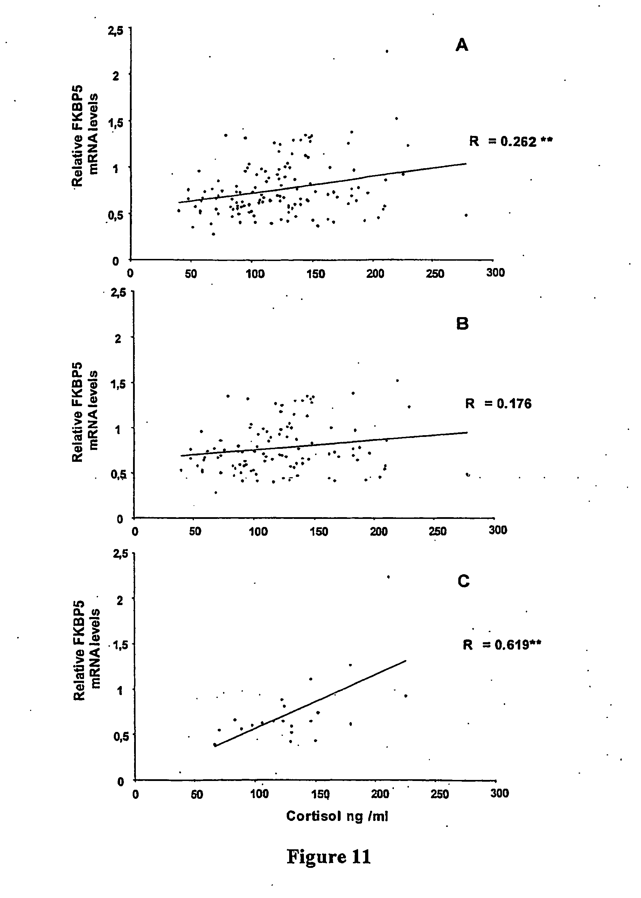

**Figure 11****. A.** Correlation of plasma cortisol levels (X axis) and relative FKBP51 mRNA levels in PBMCs in healthy controls, all rs1360780 genotypes combined (N = 124). **B**. Correlation of plasma cortisol levels (X axis) and relative FKBP51 mRNA levels in PBMCs in healthy controls with CC and TC as rs1360780 genotypes (N = 103). **C**. Correlation of plasma cortisol levels (X axis) and relative FKBP51 mRNA levels in PBMCs in healthy controls with TT as rs1360780 genotype (N = 21). For parts A-C ** = p values for correlation < 0.01.

The Examples illustrate the invention.

### Example 1

### Methods

### Patients:

Between 256 and 294 patients admitted to our psychiatric hospital for treatment of a depressive disorder presenting with a unipolar depressive episode (86.6%), bipolar disorder (12.0%, 7.5% bipolar I and 4.5% bipolar II) or dysthymia (1.2%) as their primary psychiatric diagnoses were recruited for the study. For 30.8% these patients the depressive episode could be specified as with melancholic features. Patients were included in the study within 1-3 days of admission to our hospital and the diagnosis was ascertained by trained psychiatrists according to the Diagnostic and Statistical Manual of Mental Disorders (DSM) IV criteria. Depressive disorders due to a medical or neurological condition were exclusion criteria. Ethnicity was recorded using a self-report sheet for perceived nationality, mother language and ethnicity of the subject itself and all 4 grandparents. All included patients were Caucasian and 92 % of German origin. An additional 50 patients were genotyped for the investigation of quantitative phenotypes. The study has been approved by the local ethics committee. Written informed consent was obtained from all subjects. Severity of psychopathology at admission was assessed using the 21 items Hamilton Depression Rating Scale (HAM-D) by trained raters, including residents in psychiatry and psychologists. Ratings were performed within 3 days of admission and then in weekly intervals until discharge. We used three types of response definition commonly used in psychiatric research, that define different aspects of antidepressant response. Early response at two weeks was defined as a greater than 25% decrease of HAM-D scores from the score obtained at admission. Patients with a reduction > 25% from their score at admission were considered as early responders, while patients whose HAM-D score decreased ≤ 25% from the score at admission were considered as early non-responders. For the definition of response at 5 weeks a reduction of over 50% in HAM-D scores at admission was required to be considered a responder for these time points. All patients with a reduction of HAM-D scores equal to or less than 50% were assigned to the group of non-responders. The five-week time point was chosen because this duration of treatment is considered sufficient for an antidepressant drug to display its clinical efficacy. Remission at discharge was defined as reaching a total HAM-D score equal or smaller than 10. Overall response at discharge may serve to investigate aspects of treatment resistance, which can occur in up to 10-15% of all patients. The study was designed as a naturalistic pharmacogenetic study (Munich Antidepressant Response Signature (MARS) project (Holsboer 2001)), all patients were treated according to the doctor's choice with antidepressant drugs within a few days of admission. Patients who were admitted on antidepressant medication (73%) were switched to another compound within a few days of admission. 21.6 % of patients received a mood stabilizer in addition to the antidepressant medication and 7.7% an antipsychotic drug. 48.7 % of patients were additionally treated with a benzodiazepine in the first few days after admission. For all patients plasma concentration of antidepressant medication was monitored to assure clinically efficient drug levels. Not all patients finished the weekly psychopathology ratings after being included in the study. Patients for the replication sample (N = 85) were recruited at the psychiatric hospital of the Ludwig Maximilian University in Munich, Germany (N = 57) and the psychiatric hospitals of Augsburg (N = 12) and Ingolstadt (N = 16), Germany. 89.8% had a unipolar affective disorder, 8.2 % a bipolar affective disorder (5.9% bipolar I and 2.3% bipolar II) and 2.0% dysthymia. 66.0% were female and 34.0% were male. The mean age was 50.5 years (SD:12.48). Patients from these hospitals were recruited for pharmacogenetic studies. Patients from the Ludwig Maximilian University were treated in monotherapy studies for 4 weeks, 18 patients with sertraline, 15 with reboxetine and 24 with mirtazapine. In Ingolstadt and Augsburg the studies also have a naturalistic design. The two most frequently used primary antidepressants were mirtazapine (45.9%) and citalopram (27.3%). In all three hospitals, diagnosis was assertained by trained psychiatrist according to DSMIV and patients were rated weekly from admission to discharge using the HAM-D rating scale: At the Ludwig Maximilian University patients were also rated using the Clinical Global Impression Scale (CGI) and the Montgomery Asperg Depression Rating Scale (MADRS). For the purpose of this study early response at two weeks was defined using the same criteria as for the Max-Planck Institute of Psychiatry sample (patients with a reduction > 25% from their score at admission were considered as early responders, while patients whose HAM-D score decreased ≤ 25% from the score at admission were considered as early non-responders).The studies had also been approved by the local ethics committee. Written informed consent was obtained from all subjects. 339 controls matched for ethnicity (using the same questionnaire as for patients), sex: 41.4% male in patients and 37.0% male in controls (p = 0.24) and age (mean age of 47.65, SD 14.5 in patients and mean age of 46.59, SD 15.4 in controls, p = 0.363) were recruited. Controls were selected randomly from a Munich-based community sample and screened for the presence of anxiety and affective disorders using the Composite International Diagnostic-Screener (Wittchen, H. et al. Screening for mental disorders: performance of the Composite International Diagnostic - Screener (CID-S). International Journal of Methods in Psychiatric Research 8, 59-70). Only individuals negative for the above-named disorders were included in the sample. Recruitment of controls was also approved by the local ethics committee and written informed consent was obtained from all subjects.

### DNA preparation:

On enrollment in the study, 40 ml of EDTA blood were drawn from each patient and DNA was extracted from fresh blood using the Puregene® whole blood DNA-extraction kit (Gentra Systems Inc; MN).

### Genotyping:

Up to 1000 bp of the promoter region and all exons including at least 50 bp of the exon/intron junctions were screened in 94 depressed patients, for potential genetic differences in all investigated genes except GR were screened using capillary electrophoresis single strand conformational polymorphism (CE-SSCP) analysis in 94 depressed patients. Subsequent sequencing of PCR products showing mobility differences revealed 9 SNPs with minor allele frequencies ranging from 0.022 to 0.364. Except for one intronic polymorphism in STUB1 (rs1046112) none of the other polymorphisms had been recorded in dbSNP as of March 2003 (see supplemental information on http//www.mpipsykl.mpg.de/AGBMM for sequences). CE-SSCP and subsequent sequencing were performed on an ABI PRISM® 3100 DNA sequencer (Applied Biosystems; CA) using standard procedures. SNPs in the FKBP51 locus were selected from the SNIPS identified by (CE-SSCP) analysis screening in 94 patients, from the public SNP database dbSNP (http://www.ncbi.nlm.nih.gov:80/). The SNP search tool at http://ihg.gsf.de/ihg/snps.html was used to download SNP sequences from public databases. All newly detected SNPs were selected for genotyping and supplemented by SNPs from dbSNP. Prior to genotyping, the relevant regions were amplified by PCR. Table 6 shows the PCR primers used. Genotyping was performed on a MALDI-TOF mass-spectrometer (MassArray® system) employing the Spectrodesigner software (Sequenom^{™}; CA) for primer selection and multiplexing and the homogeneous mass-extension (hMe) process for producing primer extension products. Table 7 shows the primers used for the primer extension assays.

**Table 6**

| SNP-ID | sense | antisense |
|---|---|---|
| rs1334894 | ACGTTGGATGCTTGTCATCCCAGCATC TTC | ACGTTGGATGCCCTATTTGGCCTCAAA AAG |
| rs1360780 | ACGTTGGATGAAGAGATCCAGGCACAG | ACGTTGGATGTGCCAGCAGTAGCAAGT AAG |
| rs15258 | ACGTTGGATGGTCAGTATGGCAAATGA GTG | ACGTTGGATGTTTTCAGCCCGGGTATA AGC |
| rs1546428 | ACGTTGGATGAAACGGAAGCAGAGTCA CTC | ACGTTGGATGGGCTGAAAATCCGGAAA AAG |
| rs1802039 | ACGTTGGATGGAGGTGTAGCAGAAAAA GGC | ACGTTGGATGTCAGGTAAATTCCGCTC CAG |
| rs1803817 | ACGTTGGATGTGGAGAGGGGCTATGCT TCTG | ACGTTGGATGCACAGAGATGAAGGAGG AGC |
| rs1903370 | ACGTTGGATGCATTGTGTATCCTTCCA GGC | ACGTTGGATGAGGGACTACAAATCAGT GTG |
| rs1981656 | ACGTTGGATGTACTCACTTTCTCCCCCT | ACGTTGGATGTAGTGGACAAAGACTCGGTC |
| rs2118727 | ACGTTGGATGAGCTCCAAAGTATGCTA CCG | ACGTTGGATGTCTAGCCCACAGTTGTG ATG |
| rs2290893 | ACGTTGGATGAGCCTTCTCCAAGATAG GTC | ACGTTGGATGGCTCTTCAGCGATTTCG TAG |
| rs258813 | ACGTTGGATGTTGATCATGGGAAGACA TGG | ACGTTGGATGTAGGATGCGCCTTTTTC TCC |
| rs2740204 | ACGTTGGATGTGTGTTTCTCTGGGTGT CTG | ACGTTGGATGAACTGCTAAGAACGTGT CCG |
| rs2958153 | ACGTTGGATGAAGGCCTAGGGTGAAGT TAC | ACGTTGGATGCCTACTTTTACCCCTTTG GC |
| rs2958154 | ACGTTGGATGCCAGCATCACTACATGA TAG | ACGTTGGATGGCATGTTTCAGAGTCCA GAG |
| rs3021522 | ACGTTGGATGACCCCAGGAGAGAAACA AAG | ACGTTGGATGCAGGGTCTTCCCATCTA AAG |
| rs3176921 | ACGTTGGATGTATGAGCCTGCAGAAGC AAG | ACGTTGGATGTGGGAGTAGCTCTTGTC ATC |
| rs3204090 | ACGTTGGATGTCCCCAACTTGGCTATG AAG | ACGTTGGATGAGGGAACCTCAGTAGTC CTC |
| rs33388 | ACGTTGGATGTCTGCCTGAATGAATGG GTG | ACGTTGGATGTGGGTGAAAGTCATGGA TGG |
| rs33391 | ACGTTGGATGCACTACAGGAGTCTCAC AAG | ACGTTGGATGGGTGAGTTGTGGTAACG TTG |
| rs5195 | ACGTTGGATGTGCGCTGCCAGGAGGA GAAC | ACGTTGGATGTTGCAGCAAACGCCGAA GGC |
| rs6156 | ACGTTGGATGGGGAAAGAGAGGAGAG AAAG | ACGTTGGATGTCTCTGCAGAGAGACGT CTC |
| rs6157 | ACGTTGGATGAAAGTTGGTGGCGTGTT CCG | ACGTTGGATGAAGGAAGACAACCTCCA GAG |
| rs6158 | ACGTTGGATGAAAGTTGGTGGCGTGTT CCG | ACGTTGGATGAGACGTCTCTCTGCAGA GAG |
| rs6159 | ACGTTGGATGTGGGAGAGGAGTACTTC CTC | ACGTTGGATGAAAAAGTTGGCGGTCGC CTG |
| rs6188 | ACGTTGGATGTAAACTGTGTAGCGCAG ACC | ACGTTGGATGTGTAGTGGCCTGCTGAA TTC |
| rs6191 | ACGTTGGATGTTTCCATCTTGGCTGGT CAC | ACGTTGGATGCCTTCTGACACTAAAAC CAG |
| rs6192 | ACGTTGGATGCCAAGCAGCGAAGACTT TTG | ACGTTGGATGACTGCTTTGGACAGATC TGG |
| rs6195 | ACGTTGGATGCATTCCACCAATTCCCG TTG | ACGTTGGATGTCCCCAGAGAAGTCAAG TTG |
| rs6196 | ACGTTGGATGGGCAGTCACTTTTGATG AAAC | ACGTTGGATGCCGAGATGTTAGCTGAA ATC |
| rs6597 | ACGTTGGATGCTGGCAAGCAGGAAATG TGG | ACGTTGGATGAGATGGACCTGGCCAGA TGTC |
| rs706118 | ACGTTGGATGAATGTCTGGGCCATAGG AAC | ACGTTGGATGTTTCCTGTCACAATGAG CCC |
| rs706120 | ACGTTGGATGAGACTGGAACTCAGGAC TTG | ACGTTGGATGTAGGTCTAGCAGCTTTC TGC |
| rs734369 | ACGTTGGATGATAAGGCTGCAAGACTG CAG | ACGTTGGATGATGTTGGCGTATATCCT GCG |
| rs737054 | ACGTTGGATGACAAGGTGACCGAGAAC ATG | ACGTTGGATGGGTATATTTGGTCAGGT GCC |
| rs755658 | ACGTTGGATGACTAGGATTTACCACAG CCC | ACGTTGGATGGGTGAGGGTTGGAGTAT TTG |
| rs2103681 | ACGTTGGATGCTTGAGCTGACATGAGC ATC | ACGTTGGATGAACAGCTGACCCACAGA ATC |
| rs1883636 | ACGTTGGATGAGTCTTGAGGGTTCACT CAG | ACGTTGGATGTCTTACTGGGCCCAAAC AAG |
| rs1540910 | ACGTTGGATGTGCTCCAAAGTCCCTAT TCC | ACGTTGGATGTTGCATGTCTGGAGATC TGG |
| rs4713878 | ACGTTGGATGCTAAACACTGGCTGTGT GAC | ACGTTGGATGTTCATCCTCACAGCACA CTC |
| rs1883637 | ACGTTGGATGAGAAGCTGGGCAGATTT CTC | ACGTTGGATGGGCCCTGAATCAGTCTT AAG |
| rs992105 | ACGTTGGATGGGCATGGCCTTAACTTT GTG | ACGTTGGATGGAACGTACTCTGGTAAG CAC |
| rs1051952 | ACGTTGGATGAACATCTGGAGTTGGAG CCG | ACGTTGGATGGGGAGAGGGGAGGTTA AA |
| rs2273000 | ACGTTGGATGTAGAATCCTCTGTCCTC CTC | ACGTTGGATGAATTTCTCCCCACGATG GTC |
| rs4713897 | ACGTTGGATGGCCCTGAGTATACTTTC TGG | ACGTTGGATGCACTGGGCATTTGCCCA TTT |
| rs734369 | ACGTTGGATGATAAGGCTGCAAGACTG CAG | ACGTTGGATGATGTTGGCGTATATCCT GCG |
| rs747411 | ACGTTGGATGGACGTGACACCACACTT GAC | ACGTTGGATGAGGCAGGAGGATCTCTT GAG |
| rs1320991 | ACGTTGGATGGTGTGCGTATGCATACT GTG | ACGTTGGATGTGCCTGCACTGAAAACA TGC |
| rs737054 | ACGTTGGATGACAAGGTGACCGAGAAC ATG | ACGTTGGATGGGTATATTTGGTCAGGT GCC |
| rs3777747 | ACGTTGGATGCCACTCTTACATTCCTCT CC | ACGTTGGATGTCCCTCTCCAAATCTCA CTG |
| rs4401662 | ACGTTGGATGAATTGCTTGAACCCAGG AGG | ACGTTGGATGAGTCTTGCTCTGTCATC CAG |
| rs4713908 | ACGTTGGATGATTCCGTTGTGTGTGTAT GC | ACGTTGGATGTCAGACATCTGTCATTCT TC |
| rs755658 | ACGTTGGATGACTAGGATTTACCACAG CCC | ACGTTGGATGGGTGAGGGTTGGAGTAT TTG |
| rs4713899 | ACGTTGGATGAGTGGAGCTATAGGAGC TAG | ACGTTGGATGACGGAAAGACTGCTGAT TGC |
| rs1360780 | ACGTTGGATGAAGAGATCCAGGCACAG AAG | ACGTTGGATGTGCCAGCAGTAGCAAGT AAG |
| rs4713907 | ACGTTGGATGTCCTGACTTTGTGATCC ACC | ACGTTGGATGATACCATACTCTAGGCT GGG |
| rs2092427 | ACGTTGGATGACCTGCAGTACTTTTGG CAG | ACGTTGGATGCAGACACTTTCTAAGTG CTG |
| rs4713905 | ACGTTGGATGTTCAATACCTCACCTGTC TC | ACGTTGGATGGTGTATTCTGCTTGTATT GGG |
| rs4520009 | ACGTTGGATGACGAACAGGAAACTGAA AGC | ACGTTGGATGGAGCATGGTTTTCAGTA AAG |
| rs4713906 | ACGTTGGATGAAGTATTTGTGGCTGGA GGC | ACGTTGGATGATCTCCTGACCTTGTGA TCC |
| rs4713916 | ACGTTGGATGTATCTGGCAACCCTAAC CTC | ACGTTGGATGCCTAACGAGATAGTGAG GAG |
| rs2143404 | ACGTTGGATGGGTTAGGTAGAGCTCAG TTC | ACGTTGGATGGTAGAGAACCTGGTAAG AAG |
| rs4711429 | ACGTTGGATGACACACGGCTCATCTGT AAC | ACGTTGGATGATGCCAGGCATTTGGGT TTC |
| rs9462104 | ACGTTGGATGTTTCCTCCCTAGATTCCC AG | ACGTTGGATGGTCCCAGTGGAACTTTA TGG |
| rs9394312 | ACGTTGGATGACAAGCTATCGGGCTTT CTC | ACGTTGGATGAGACAATCTCCCAGTTG CAG |
| rs943297 | ACGTTGGATGTCTGAAATCCAGCTGGA CAC | ACGTTGGATGAGTGAGACTCTGTCCAA AAG |
| rs9380528 | ACGTTGGATGTTGAGACCAGATGTTGG AGG | ACGTTGGATGGACAGAGTCTGGCTCTG TTG |
| FKBP5UT5A | ACGTTGGATGTACAGACCCTGCAAAGA ACC | ACGTTGGATGTGCGCTATGGCTGCAGA TAC |
| FKBP5UT5C | ACGTTGGATGTACAGACCCTGCAAAGA ACC | ACGTTGGATGTGCGCTATGGCTGCAGA TAC |
| rs9380526 | ACGTTGGATGAAACAGGGAAGCTTCTA GGC | ACGTTGGATGAAGCCAAGACTGAAACC GCC |
| rs10947563 | ACGTTGGATGTATTACAGGCGTGAGCC ATC | ACGTTGGATGATGGTCTTTAGAGGAAT TCT |
| rs9380525 | ACGTTGGATGGACAGAGTGAGACTCCT CTA | ACGTTGGATGTTCTCAAACCAGCACTC AGG |
| rs6912833 | ACGTTGGATGGGGAAAAGAATCAAGGG AAG | ACGTTGGATGCTGGAACTAAGTGATCT GTG |
| rs7748266 | ACGTTGGATGAGACCACACCACAGAAC AAG | ACGTTGGATGTTGCTTAACCCTCTCCA GAC |
| rs9470069 | ACGTTGGATGATATACATACAGGCTGG CCG | ACGTTGGATGACTCCTGACCTCGTGAT CTG |
| rs6926133 | ACGTTGGATGGATGACTGATACTTCAG TCT | ACGTTGGATGGAAAGCAAAGCTGAAAA GTAG |
| rs2395634 | ACGTTGGATGTCGAAGGGACTTATTCC TCC | ACGTTGGATGCAGCAGAAGGAAGACAT CAG |
| rs7753746 | ACGTTGGATGAGAATAAACCTGTGTTTC TG | ACGTTGGATGAGGAGCAACCATATTTC TAA |
| rs10807151 | ACGTTGGATGGGGTGAAAGTGGCAGAA CAA | ACGTTGGATGCCGCTGGCTAATGAAAA AAC |
| rs9348978 | ACGTTGGATGAATTTTTACCACTGAGCA GG | ACGTTGGATGGCTTTGGCTTTACGGAA GAG |
| rs11751447 | ACGTTGGATGATTCTCTCTTGAGTCGG TGC | ACGTTGGATGTAGAACCTTGCCACAGA GAC |
| rs2395631 | ACGTTGGATGTGCAAAAACTAACAAAA GCC | ACGTTGGATGAGGGTTATCAACCTTGA GGC |
| rs4713902 | ACGTTGGATGGGAGCCAAAACATGAAG AGC | ACGTTGGATGTAGGCAACCTGTATAAG CTG |
| rs1334894 | ACGTTGGATGCTTGTCATCCCAGCATC TTC | ACGTTGGATGCCCTATTTGGCCTCAAA AAG |
| rs1475774 | ACGTTGGATGCAAGTGAAAAACTCCAC ACC | ACGTTGGATGCCATAAGTCTTTGTCCA CAAG |
| rs2817035 | ACGTTGGATGCCTCTTTTCTCCTAGGAT CC | ACGTTGGATGGTTGCAAACAGAGGTAG GAG |
| rs3807050 | ACGTTGGATGAGAGGGAGGGMTAGTT CAG | ACGTTGGATGTGTGTCTCCAAGACTGT GTG |
| rs1977655 | ACGTTGGATGCCCGTCTCTGCTAAAAA TAC | ACGTTGGATGCCAAGTTCAAGCGATTC TTG |
| rs3800374 | ACGTTGGATGAGACGATGGACCCATTT TAC | ACGTTGGATGTCTTTTCCAAGTGGTGA ACC |
| rs4713912 | ACGTTGGATGAGGTTCAAGCGATTCTC CTG | ACGTTGGATGCAAAAATTAGCCGGGCT TGG |
| rs2817047 | ACGTTGGATGTCTGGGCTCAAGTGATT CTC | ACGTTGGATGAATTAGCCAGGCATGAT GGC |
| rs4713913 | ACGTTGGATGGAATTTAACTAGGAGTG CTG | ACGTTGGATGAGCGAGACTCCGTCTCA AAA |
| rs4713921 | ACGTTGGATGAAGCCCTGTGGTTTTAT GCC | ACGTTGGATGTGGAACAATTCTGTCCC CAG |
| rs2817041 | ACGTTGGATGGTACACACAGCGAGTGA TAC | ACGTTGGATGCTCCTCAACTCTTTGGA GTG |
| rs2766543 | ACGTTGGATGTCTGTGGTTGGCCTTTT CTC | ACGTTGGATGAAGCAGAGCTGCCCAAT AAG |
| rs5020575 | ACGTTGGATGTGATCTTGGCTCACTGC AAC | ACGTTGGATGAAATTAGCCAAGCGTGG TGG |
| rs1591365 | ACGTTGGATGGTGGCAAATAGGAGTTC TCC | ACGTTGGATGTTGGCAGGTGTTTTTCT GAG |
| rs2296662 | ACGTTGGATGAATTGGCCTATGACCAG CAC | ACGTTGGATGAAGAGGAGGAGAAACCA GAG |
| rs2817010 | ACGTTGGATGTGGTGTGTATGAGAAGC AGC | ACGTTGGATGTAAGTCTGTGCAGACGG TGG |
| rs873941 | ACGTTGGATGGGGTGATGCTTTTGCAA CTC | ACGTTGGATGGCTATGATACCTGGCTG ATC |
| rs3800373 | ACGTTGGATGAAACCCCTAGTGTAGAA GAG | ACGTTGGATGTTTACACTCCTCTATCAT GC |
| rs4713903 | ACGTTGGATGGAGTCTAAACAAAAACA TCC | ACGTTGGATGAAGTACTGGGATTACAG GTG |
| rs2766534 | ACGTTGGATGTCGAAGGGACTTATTCC TCC | ACGTTGGATGCAGCAGAAGGAAGACAT CAG |
| rs2766554 | ACGTTGGATGAGAACCTCCTACTACTG AGC | ACGTTGGATGGCTTAGAATCCATGACC CAC |
| rs2817054 | ACGTTGGATGAAGCTAAGGCCCAGGAC GTG | ACGTTGGATGACCTGTCAGCCCTCTGA GCT |
| rs2766597 | ACGTTGGATGTTATAGCCCCATCACCA CAG | ACGTTGGATGAGGTTGATAATGATCCC CCG |

**Table 7**

| SNP-ID | extension primer |
|---|---|
| rs1334894 | TTGATGACTTAGTCCTGTC |
| rs1360780 | GGCTTTCACATAAGCAAAGTTA |
| rs15258 | TTTAATACACTATTGGATTTTTT |
| rs1546428 | AATATGCCTCCTGGCGTT |
| rs1802039 | AGCAGAAAAAGGCTGTGCTGCC |
| rs1803817 | CTATGCTTCTGTCTCCAC |
| rs1903370 | TCCTTCCAGGCTTTTCTTTAG |
| rs1981656 | CAAGAGAGAGGGCACAGGT |
| rs2118727 | AAAGTATGCTACCGGAAACAC |
| rs2290893 | GTAAATTATGAGACAAACTTTT |
| rs258813 | CTAACTACAGTGATTTTGTC |
| rs2740204 | TGGGTGTCTGTGGCTCT |
| rs2958153 | GTTACCGAAAGAGGCGAGTA |
| rs2958154 | AGCTTGTCATTTCTCACCTTT |
| rs3021522 | GAGTCACGTACAGGGTG |
| rs3176921 | AGCCTGCAGAAGCAAGGCCAATAA |
| rs3204090 | GTTATTGACGCATTCATCTCTGA |
| rs33388 | ATGCTTCTCTAGGTGTGTGA |
| rs33391 | CCTCTGAAAATCCTGGTAA |
| rs5195 | GCTGCCAGGAGGAGAACTACCTGC |
| rs6156 | GGAAGACAACCTCCAGAG |
| rs6157 | CAGAGAGACGTCTCCGG |
| rs6158 | GCGCTTCGCAGGTGAGC |
| rs6159 | CTCCTCGCTCCTCGCCGG |
| rs6188 | TTACAGTTCATTTCTATGTATTT |
| rs6191 | CTGTAGGTGAATGTGTTTTT |
| rs6192 | GCGAAGACTTTTGGTTGAT |
| rs6195 | TTCCCGTTGGTTCCGAAA |
| rs6196 | ACAGAAGTTTTTTGATATTTCC |
| rs6597 | TGGGGAAGTGTGGATGTTAGC |
| rs706118 | TGGGCCATAGGAACTGATCT |
| rs706120 | TCAGGACTTGCCACAAAGAGAA |
| rs734369 | AAGACTGCAGATCTCCATGTGCCA |
| rs737054 | GACGCCCAGGCACAGCC |
| rs755658 | GCGCGTACATCTCACTG |
| rs2103681 | ATCTATTTCTGTAAAACTCAG |
| rs1883636 | CCCACAGTTGTCCTTCC |
| rs1540910 | CTTCCCTGGAAACCCCAAG |
| rs4713878 | GACAAGTTACTTAACTTCTCTGAG |
| rs1883637 | CCACTAGAGCCCCATAATTTCTC |
| rs992105 | TCACCTTGTATTTCTAAAGAT |
| rs1051952 | TCCGGGAGCAGTAGTCA |
| rs2273000 | GGGTGGAGGCATATATTGGTCT |
| rs4713897 | TTCTGGAACATAATGTGAGC |
| rs734369 | GCAGATCTCCATGTGCCA |
| rs747411 | CTATGCTGTCCTGGCTGGTCTC |
| rs1320991 | GCGTATGCATACTGTGTGTATA |
| rs737054 | GACGCCCAGGCACAGCC |
| rs3777747 | ATTCCTCTCCTTTCCAGC |
| rs4401662 | GAGCTGCGATAGCATCACT |
| rs4713908 | TGTTTTCTTTACTCATTTAACTTA |
| rs755658 | GCGCGTACATCTCACTG |
| rs4713899 | AGGCTCATCTGCATCTGTTAC |
| rs1360780 | AGGCTTTCACATAAGCAAAGTTA |
| rs4713907 | CTGTAGAATCCGTAGAATC |
| rs2092427 | AACCATCACTAAAGAAGTCAGCAA |
| rs4713905 | TTAGAAGATGTGAACTACATT |
| rs4520009 | AGGAAACTGAAAGCTAAAAGTTCA |
| rs4713906 | CCTGTAATCCCAGCATTTT |
| rs4713916 | GACTCCTACATTTTCCTCT |
| rs2143404 | GAGCTCAGTTCCCAGGG |
| rs4711429 | ATCTGTAACTTAGAGCCCTT |
| rs9462104 | CCTTAGGCATAACCACC |
| rs9394312 | TTCTCCCGAGGCTCCCA |
| rs943297 | CTGGACACAATTCCTTAGTTA |
| rs9380528 | AACTGAGATCATGCCACT |
| FKBP5UT5A | GGAAAGGGCTGCTCATCC |
| FKBP5UT5C | TCATTAACGCAGAAAAACAAA |
| rs9380526 | GAGGACAGACAAATGACT |
| rs10947563 | CAGTAATATGCTTGCTGTACC |
| rs9380525 | TTACTAGTGACATTCTAAAGGAG |
| rs6912833 | GATGCAAGAATAGCATGGATC |
| rs7748266 | GAACAAGAAACCAGTTGTATCA |
| rs9470069 | CTGTAATTCCAGCACTTTG |
| rs6926133 | AGTCTTTAAGTTTAATTGCAGGTC |
| rs2395634 | TAGAATGTTCTTACACAAATCTAG |
| rs7753746 | GTGTTTCTGGTTTGTTACC |
| rs10807151 | TGGCAGAACAATGCATC |
| rs9348978 | CTGAGCAGGTGAAAAAT |
| rs11751447 | GGTGCCTCGTGCAGAGC |
| rs2395631 | TTTCATCTGATAGGCCCAAC |
| rs4713902 | CATGAAGAGCTAATTCCTTTATCA |
| rs1334894 | GGAAGTTGATGACTTAGTCCTGTC |
| rs1475774 | CTTGTTTTCTAATGATTCAAG |
| rs2817035 | GGCTCCCTACTCCACCACTAC |
| rs3807050 | CCAGTAAAGACTTGCCTGA |
| rs1977655 | TCTGCTAAAAATACAAAAATAGCC |
| rs3800374 | CCCATTTTACAGTTATGGGCTC |
| rs4713912 | GCCACCCGAGAAGCTGGGATTA |
| rs2817047 | CCCCCCAAGTAGCTGGGACTACA |
| rs4713913 | GTGTTTTTGTTTGTTTGTTTGTTT |
| rs4713921 | CAGTTGAAAGAGCCTCAC |
| rs2817041 | GTGCAGGATCATGCTCTTGGC |
| rs2766543 | CTTTTCTCCCTGCTACGT |
| rs5020575 | AGTGATTCTCGTGCCTCA |
| rs1591365 | TGTCAGTTGTTTTTCCTTGAA |
| rs2296662 | CTGGGCTCGCTTCCCTCCC |
| rs2817010 | AGAAGCAGCCTATGTTGAGG |
| rs873941 | TTTTGCAACTCACTTTTTAAAA |
| rs3800373 | AAGAGCAACTATTTATTTGTCAAC |
| rs4713903 | CCAAACAACTAAATTGGGAAA |
| rs2766534 | CCGCCCTACACTTTCAC |
| rs2766554 | CTTTTGCCCTCACATTCTT |
| rs2817054 | AGCAGCGGCTCAGGCAG |
| rs2766597 | AGAAGATCCTGATCCTCC |

### Neuroendocrine assessment using the combined dexamethasone suppression/CRH stimulation (Dex-CRH) test:

For controls and remitted patients, Plasma was collected at the same timepoint as blood was drawn for protein or mRNA extraction to measure cortisol plasma concentrations. For depressed patients, the Dex-CRH test was administered to 241 patients of the Max-Planck Institute of Psychiatry on average within the first 7.2 days of admission (SD = 3.2). The Dex-CRH test was performed as described in detail in Heuser et al., 1994¹⁸. Patients were administered the test within the first ten days of admission (n= 225) and the last ten days of discharge (n = 150). Briefly, patients were pre-treated with 1.5 mg of dexamethasone per os at 23:00. The following day a venous catheter was placed at 14:30 and blood was drawn at 15:00, 15:30, 15:45, 16:00 and 16:15 into tubes containing EDTA and trasylol (Bayer Inc., Germany). At 15:02 100µg of human CRH (Ferring Inc., Kiel, Germany) was administered intravenously. For the area under the curve (AUC) of the cortisol and ACTH response, the area under the concentration-time course curve corrected for the baseline value at 15:00 was computed using a trapezoidal integration for the test at admission as well as at discharge. Hormone assays for the Dex-CRH test were identical to those described in detail in Zobel et al., 2001¹⁷. Briefly, for the measurement of plasma cortisol concentrations, a radioimmunoassay (RIA) kit from ICN Biomedicals, Carson, CA was used where the detection limit was 0.3 ng/ml. For plasma ACTH concentrations an immunometric assay without extraction (Nichols Institute, San Juan Capistrano, CA) was used, with a detection limit of 4.0 pg/ml.

### Quantification of FKBP51 protein levels in lymphocytes:

T cells were extracted from the whole blood of healthy controls using magnetic beads with antibodies against CD2 (Dynal Inc.). Cells were then lysed in a protein lysis buffer. Protein levels of FKBP51 were assessed using Western-blot analysis.

For protein extraction cells were solubilized in 1 ml lysis buffer (20 mM Tris/HCl pH 7.5; 130 mM NaCl; 20 mM Na₂MoO₄;1 mM EDTA; 10% glycerol; 0.5% Triton 100; 1:100 protease inhibitor cocktail P2714 from Sigma). The extract was incubated for 1 h on ice, centrifuged for 4 min at 13000 rpm and protein concentration was determined.

An alternate method for evaluating FKBP51 protein levels consists of extracting Lymphocytes from whole EDTA blood of healthy controls and remitted depressed patients using magnetic beads labeled with anti-CD2 antibodies according to standard protocols (Dynal Inc.). Briefly, EDTA blood was incubated with 100µl anti-CD2 Dynabeads for 20 minutes. Lymphocytes were then isolated using a magnetic device and 3 washes with PBS containing 2% fetal calf serum. Lymphocyte isolation and protein extraction occurred immediately following blood draw. For protein extraction cells were then dissolved in 250µl lysis buffer (20 mM Tris/HCl pH 7.5; 130 mM NaCl; 20 mM Na₂MO₄; 1 mM EDTA; 10% glycerol; 0.5 % Triton 100; 1:100 protease inhibitor cocktail P2714 from Sigma). The extract was incubated for 1h on ice, centrifuged for 4 min at 13000 rpm and protein concentration was determined using the BCA-kit (Pierce).

For immunoblot detection, 10 µg and, in an additional set of experiments, 20 µg of cell lysates' total protein were separated by SDS-PAGE under denaturing conditions. The proteins were transferred to polyvenylidene difluoride membrane (Schleicher & Schüll GmbH, Germany). Nonspecific binding to membrane was blocked by 5% nonfat milk in Tris-buffered saline/Tween buffer. FKBP51 was detected by a monoclonal anti-FKBP51 antibody (Stress Gen Biotech) followed by horseradish peroxidase-conjugated rabbit anti-mouse antibody (Sigma). Signals were visualized by ECL solution (Amersham Pharmacia Biotech) and monitored in an imaging system (Kodak Imaging Station 440). Light emission was normalized by the intensity of a recombinant FKBP51 standard that was loaded onto each gel to ensure accurate comparison of probes loaded on different gels.

### Statistical analysis:

All analyses for binary outcomes were performed using logistic regression using both R and SPSS (version 11), as well as by exact contingency table analyses using SPSS. Quantitative variables were analyzed using logistic regression analysis. Linkage dysequilibrium was calculated using the "Gold" software.

Appropriate correction for multiple testing in association studies with markers in linkage disequilibrium (LD) was done by performing different methods of correction and their results.
1. The classical Bonferroni correction. Here we have a total of 59 polymorphic SNPs and four phenotypes (depression per se, response at 2 weeks, at 5 weeks and at time of discharge). This gives a total of 59*4 = 236 tests.
2. A method recently described by Nyholt²⁸, which aims at estimating a number of effective tests by taking into account intermarker LD. This reduces the number of effective tests by roughly a factor 2, thus leading to a number of effective tests of 118.
3. permutation based methods, taking into account
   a. the correlation between the response phenotypes, effectively creating a joint response phenotype analysed.
   b. the LD between SNPs
   c. LD between the SNPs and the correlation between the phenotypes.

The p-values for methods a, b, and c were obtained using 50000 replicates each for methods b and c and 500,000 replicates for method a, using the method of Ge and colleagues (http://personales.unican.es/cofinoa/test/). Taking into account dependencies between SNPs and phenotypes was done using Fisher's product method.

For method a) the nominal p-value was 0.000036 for SNP rs3800373 with the joint response phenotype. This has to be Bonferroni-corrected by the fact that we are dealing with two phenotypes now (depression per se and the joint response phenotype) and have looked at 8 genes, with 32 SNPs typed in and around the FKBP51 gene, which also contains the rs3800373 SNP. Thus the corrected p-value for this SNP is 0.000036 * 2 * 8 * 32, which is equal to 0.018432.

For method b, we have a nominal p-value of 0.00018 for response at two weeks and the jointly considered SNPs in FKBP5, which needs to be multiplied by a factor of 3 to arrive at a p-value of 0.00054 to adjust for the three response phenotypes. This value of 0.0054 needs to be corrected again by a factor of 16 (8 genes and 2 phenotypes (depression per se and response) to arrive at a final p-value of 0.00864.

Finally for method c, which uses a Fisher product of p-values acroos all SNPs in the FKBP51 region and across all phenotypes, we arrive at a nominal p-value of 0.00014. This again has to be corrected for by a factor of 16 (8 genes and 2 phenotypes) to arrive at a corrected p-value of 0.00224.

Recent literature, advocating (haplotype-) block-wise significance tests²⁹ or gene-wide significance tests³⁰, which in our case are equivalent, that method c is actually the most appropriate method of analysis. However, we present all these results for the reader's perusal.

To control for stratification we used the genomic control approach of Devlin and Roeder (1999), with the estimation of the stratification parameter λ performed using the likelihood method of Freedman³¹. We used 100 unlinked SNPs to perform the analysis.

In addition we also performed one-way and repeated measures analysis of variance (ANOVA), a stepwise (forward inclusion) multiple regression analysis and correlation analysis using SPSS.

Individual haplotype assignments were determined using SNPHAP (http://www-gene.cimr.cam.ac.uk/clayton/software/snphap.txt ). Only haplotype assignments with a remaining uncertainty of less than five percent and haplotypes with a frequency over five percent were included in the analyses. Removing these restrictions turned out not to alter our conclusions, with p-values showing only minimal changes. To test for the simultaneous involvement of several polymorphisms we also applied the method of Cordell and Clayton³². For the analysis of the LD pattern and haplotype block' delineation we used haplotypes estimated by PHASE (hftp://www.stat.washington.edu/stephens/phase.html, ³³) in our sample of controls as input to HAPLOBLOCKFINDER (http://cgi.uc.edu/cgi-bin/kzhang/haploBlockFinder.cgi/, ³⁴). Haplotype block definition was done using the |D'| method³⁵ with a threshold of 0.75. SNPs with a frequency of the minor allele less than 0.1 were omitted from the analysis. We also used the R package "genetics" (http://lib.stat.cmu.edu/R/CRAN/) to compute pairwise D' values from genotype data for a graphical depiction of LD via GOLD (http://www.sph.umich.edu/csg/abecasis/publications/10842743.html, ³⁶).

### Example 2

### Association study of polymorphisms in FKBP51 and response to antidepressant drugs

30 SNPs in FKBP51 (see table 1) were genotyped in 327 patients with major depression. 103 of them showed an improvement of over 25% in disease severity as measured by the Hamilton Depression Rating Scale (HAM-D) after two weeks of treatment with an antidepressant (response), while 156 (non-response) showed less than 25% improvement. The patient population used for the studies comprises of individuals that were treated with various antidepressant drugs including, Amitriptylin, Amitriptylinoxid, Clomipramin, Doxepin, Imipramin, Nortriptylin, Trimipramin, Citalopram, Fluoxetin, Fluvoxamin, Paroxetin, Sertralin, Tianeptin, Mirtazapin, Venlafaxin, Reboxetin, Moclobemid, Tranylcypromin, Bupoprion, Buspiron, Dibenzepin, Nefazodon, Opipramol, Sibutramin, and Trazodon. Most patients received only one of these antidepressants as monotherapy regiment, but some individuals received combination therapy (i.e. more than one antidepressant).

We could show a significant association of 30 SNPs in FKBP51 with response after 2 weeks of treatment.

**Table 8: Association of SNPs in FKBP51 with response at 2 weeks.**

| SNP | Non-responders (n = 156) | Responders (n = 103) | p-value |
|---|---|---|---|
| rs2766534 | GG:4.6%, GT:44.5%, TT:50.8% | GG:2.2%, GT:28.9%, TT:68.9% | 0,0057 |
| rs4711429 | AA:3.4%, AG:40.7%. AA:3.4%, AG:40.7%, GG:55.9% | AA:14.0%, AG:35.5%, GG:50.6% | 0,0076 |
| rs4713921 | CC:54.7%, TC:42.2%, TT:3.1% | CC:49.4%, TC:36.7%, TT:13.9% | 0,0036 |
| rs9462104 | CC:2.6%, TC:39.5%, TT:57.9% | CC:13.5%, TC:35.6%, TT:50.9% | 0,0043 |
| rs9394312 | CC:24.6%, GC:61.0%, GG:14.4% | CC:27.3%, GC:42.4%, GG:30.2% | 0,0016 |
| rs4713916 | AA:1.0%, AG:41.0%, GG:58.0% | AA:16.1%, AG:33.6%, GG:50.8% | 5.5 x 10⁻⁵ |
| rs943297 | AA:1.7%, AG:42.0%, GG:56.3% | AA:14.5%, AG:35.8%, GG:49.7% | 3.1 x 10⁻⁴ |
| rs9380528 | AA:21.8%, AG:61.3%, GG:16.8% | AA:24.6%, AG:42.7%, GG:32.7% | 0,0034 |
| FKBP5UT5A | GG:99.99%, GC:0.01 %, CC:0.0% | GG:99.69%, GC:0.4%, CC:0.01% | - |
| FKBP5UT5C | AA:0.0%, AG:0.0%, GG:100.0% | AA:0.0%, AG:0.01 %, GG:99.99% | - |
| rs9380526 | CC:3.4%, TC:45.8% TT:50.8% | CC:17.4%, TC:36.0% TT:46.5% | 4.8 x 10⁻⁴ |
| rs10947563 | AA:61.5%, AG:36.5%, GG:2.1% | AA:54.2%, AG:29.9%, GG:16.0% | 0,0013 |
| rs9380525 | CC:50.8, GC:46.6, GG:2.3% | CC:47.6, GC:35.9, GG:16.5% | 2.7 x 10⁻⁴ |
| rs6912833 | AA:1.9%, TA:47.7%, TT 50.5% | AA:16.2%, TA:42.6%, TT 41.2% | 3.5 x 10⁻⁴ |
| rs2143404 | CC:74.8%, TC:25.2% TT:0.0% | CC:71.1%, TC:25.4% TT:3.5% | 0,046 |
| rs1360780 | TT:1.0%, CT:40.8%, CC:58.2% | TT:14.7%, CT:35.9%, CC:49.3% | 1.2 x 10⁻⁴ |
| rs1591365 | AA:55.5, AG:42.2%, GG:2.3% | AA:51.5, AG:34.4%, GG:14.4% | 6.2 x 10⁻⁴ |
| rs7748266 | CC:75.2%, TC:24.8% TT:0.0% | CC:71.7%, TC:24.3% TT:4.0% | 0,0348 |
| rs9470069 | CC:0.8%, GC:25.2%, GG:73.9% | CC:0.0%, GC:15.6%, GG:84.4% | 0,045 |
| rs6926133 | AA:0.0%, AC:27.4%, CC:72.9% | AA:5.3%, AC:24.6%, CC:70.2% | 0,0135 |
| rs3777747 | AA:18.8%, AG:61.7%, AA:18.8%, AG:61.7%, GG:19.5% | AA:33.9%, AG:43.9%, GG:22.2% | 0,0061 |
| rs4713899 | AA:0.0%, AG:24.2%, GGC | AA:3.3%, AG:26.7%, GG:70.0% | 0,0244 |
| rs2395634 | AA:2.5%, AG:41.5%, GG:55.9% | AA:13.3%, AG:34.7%, GG:52.0% | 0,0036 |
| rs7753746 | AA:73.9%, AG:26.1 %, GG:0.0% | AA:72.7%, AG:23.3%, GG:4.1% | 0,0328 |
| rs3800373 | CC:0.0%, CA:37.9%, AA:62.1% | CC:13.5%, CA:33.8%, AA:52.7% | 2.8 x 10⁻⁵ |
| rs10807151 | CC:0.0%, TC:23.5% TT:76.5% | CC:5.2%, TC:23.1% TT:71.7% | 0,0125 |
| rs3800374 | CC:75.0%, TC:24.2% TT:0.8% | CC:65.4%, TC:29.6% TT:5.0% | 0,0354 |
| rs9348978 | AA :11.3, GA :58.3, GG :30.4 | AA :30.1, GA :48.0, GG :22.0 | 5.8 x 10⁻⁴ |
| rs11751447 | CC:18.6%, TC:55.9% TT:25.4% | CC:33.3%, TC:46.8% TT:19.9% | 0,0179 |
| rs2395631 | AA:17.9%, AG:57.3%, GG:24.8% | AA:33.5%, AG:47.6%, GG:18.8% | 0,0114 |

This effect, however, was not limited to response at 2 weeks, but could be observed during the whole in-patient treatment course. This is illustrated with rs1360780 in Figure 3a, b, c, d, with rs4713916 in figure 6a and with rs 3800373 in Figure 7a. All 30 SNPs genotyped in FKBP51 are in strong linkage dysequilibrium (see figures 1 and 2). This implies that the causal variant could lie anywhere within the linkage disequlibrium block defined by these SNPs.

We first analyzed 57 SNPs in these genes in 294 depressed patients and 339 healthy controls, matched for age, sex and ethnicity. Haplotypes were reconstructed for all SNPs within a gene. After correcting for multiple testing, we could not detect any significant differences in frequencies of single SNPs or haplotypes between our patient and our control sample. We then tested all single SNPs and haplotypes for an association with parameters related to the response to antidepressant drug treatment after 2 weeks and 5 weeks of hospitalization and to remission of symptoms at discharge (N = 233 depressed patients). There were significant associations between three SNPs in the FKBP51 gene and response at at least two of the three time points. For rs1360780 the nominal p-values for association with response at 2 weeks and 5 weeks and remission at discharge were 0.00048, 0.0027 and 0.092 respectively. For rs1334894 they were 0.050, 0.0054 and 0.030; and for rs755658 they were 0.036, 0.010 and 0.043.

We therefore focused on the region of FKBP51 and genotyped an additional 27 SNPs in 288 kb around this gene to investigate whether this result could indeed be attributed to FKBP51 and not adjacent genes. The region investigated included the neighboring genes TULP1 (3' of FKBP5), the hypothetical protein FLJ25390 and CLPS (both 5' of FKBP5), with an average distance of 9.6 kb between SNPs. Using genotype data from the control group, we constructed a linkage disequilibirium (LD) block map based on D' for this region. We detected a single large block of LD that encompasses most of the FKBP51 gene plus a region 5' of the gene, with the block ending before FLJ25390. In addition, four minor blocks of LD are detected by HAPLOBLOCKFINDER in our data (see figure 1). We then tested all SNPs in this region for association with response to antdepressant treatment. For the association with response at 2 weeks, the smallest p-values were found with SNPs within the major block of LD containing FKBP51 (see figure 2). Here we observed 3 peaks of strong association, one at the 5' end of the gene with rs4713916 (5' of FKBP5, potential promoter region, nominal p = 0.00031), the second in intron 2 (rs1360780, nominal p = 0.00048) and the third in the 3' untranslated region of FKBP51 (rs3800373, nominal p = 0.00003). A similar pattern of association was found with response at 5 weeks and remission. Adjustment for multiple tesing was performed using various methods, all showing that the observed association was significant after adjusting for multiple testing. The classical Bonferroni correction gave a corrected p-value of 0.00708, the method recently described by Nyholt²⁸ a corrected p-value of 0.00354, both for rs3800373 and response at two weeks. Permutation based methods, taking into account the correlation between the response phenotypes, effectively creating a joint response phenotype analysed (a), the LD between SNPs (b) and LD between the SNPs and the correlation between the phenotypes (c) gave corrected p-values of 0.018432 for rs3800373 and the joint response phenotype (a), 0.00864 for an association of all the genetic variation in and around FKBP51 considered and response at two weeks (b), and 0.00224 for an association of all the genetic variation in and around FKBP51 considered and the joint reponse phenotype (c), respectively.

The clustering of significant associations within the LD block containing FKBP51 indicated that the observed associations can be attributed to this gene. It is, however, not clear which one of the highly associated SNPs could be the causal variant or might be closest to it as all three of them are in strong LD (D' > 0.8). Both haplotype analysis and the stepwise procedure of Cordell and Clayton³² did not hint at untyped causal polymorphisms, most likely because of the high degree of LD across all of FKBP5.

### Example 3

### Association of rs1360780 genotype with HPA-axis activity

To investigate changes of HPA-axis activity from admission to discharge, we used the Dex-CRH test, which sensitively identifies GR-signaling impairment¹⁸. By comparing the ACTH and cortisol responses in this test of patients with CC/CT or TT genotypes of rs1360780, we observed that at admission T homozygotes displayed a significantly lower ACTH response following CRH stimulation than patients with the CC or CT genotypes (figure 4). Similar results were obtained with rs4713916 and, rs3800373 (figures 6b, 7b). An elevated ACTH response in this test indicates an impaired negative feedback through GR. Our finding thus suggests that, even though T homozygotes are as severely depressed as patients with the two other rs1360780 genotypes, their GR sensitivity is not as much impaired, allowing a faster restoration of normal HPA-axis function by antidepressants and thus an accelerated clinical response.

We plotted the weekly scores of the Hamilton Depression Rating Scale (HAM-D), measuring the severity of depressive symptoms for the first five weeks of treatment against the rs1360780 (figure 3a), rs4713916 (figure 6a, d) and, rs3800373 (figure 7a, d) genotypes. For rs1360780, repeated measures analysis of variance (ANOVA) showed T homozygotes to have an accelerated response over the whole time course (p = 0.00015 for genotype effect), while the response of patients with the CT and CC genotypes was significantly slower and almost identical. Overall, duration to response, as defined by a reduction of the HAM-D scores from admission by more than 50%, was significantly shorter for T homozygotes with 3.18 weeks (SEM=0.62) against 5.03 weeks (SEM=0.244) for heterozygotes and C homozygotes combined (p = 0.006).

Differences in response to antidepressant drugs among groups of patients can be due to a series of confounding variables, including population stratification. To exclude the latter possibility, we estimated the amount of stratification in responders and non-responders. To this end we genotyped a sample of 100 unlinked SNPs. We analysed the data using the likelihood method of Freedman³¹. The estimate of 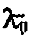 , the parameter measuring the population stratification, is 1.000, with the upper bound of the 95% confidence interval being at 2.081. It should be noted that this upper bound is the upper bound for 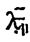 . For the most significant p-value of 0.00003 obtained with rs3800373 and response at two weeks (obtained with 96 non-responders and 146 responders), this upper bound translates into an upper bound for λ₉₆₁₄₆ of 1.125, owing to the linear relationship between sample size and λ described by Freedman et al.
(2004). Therefore, to take an example, the p-value obtained using the classical Bonferroni correction (0.00708) cannot be reasonably due to population stratification. Taking this p-value as an example and applying the correction described by Freedman³¹ (for which we transform the p-value obtained by logistic regression into a chisquared variable with one degree of freedom (7.253)), the p-value for a chisquared variable with one degree of freedom of 7.253/1.125 is 0.011, clearly significant.

Besides population stratification, differences in treatment regimen, in sex or age, in disease subtypes or the presence of psychiatric and somatic comorbidities could confound our results. To exclude the possibility that stratification in these variables (age, sex, type of antidepressant medication, presence of adjunct mood stabilizer, benzodiazepine or antipsychotic treatment, length of antidepressant treatment before admission, melancholic vs. non-melancholic, bipolar vs. unipolar and psychotic vs. non-psychotic depression or the number of psychiatric and somatic comorbidities) was responsible for the observed effects, we included these 12 variables together with the genotype of rs1360780 in a regression analysis with response at 2 weeks as the dependent variable. For antidepressant medication, we grouped patients according to the primary class of antidepressant in patients treated with selective serotonin reuptake inhibitors (N = 70), tricyclic antidepressants (N = 48) and patients treated with mirtazapine (N = 55), an antidepressant mainly targeting serotonin 2C and alpha2A-adrenergic receptors. Patients receiving a combination of antidepressants from more than one class of antidepressants were included in a fourth group. In this stepwise regression analysis containing the above-named 13 variables, only the rs1360780 genotype and length of antidepressant treatment before admission were included as significant in the model while the remaining nine variables were not found to contribute significantly to the phenotype. The longer patients had been treated in an out-patient setting before admission, the less likely they were to be in the early responder group. The inclusion of this variable did, however, not influence the significant association of rs1360780 with response. This suggests that the observed association of FKBP51 polymorphisms with response to antidepressant treatment is not likely to be due to stratification in these other clinical variables, including differences in the class of administered antidepressant. Almost identical results were obtained in the three primary antidepressant treatment classes for the association of rs1360780 with response to antidepressant treatment (see figures 3b, c, d), suggesting that this effect is independent of the primary pharmacological profile of antidepressants. This supports the notion that FKBP51 is involved in the common final signaling pathway of antidepressant drugs.

We then investigated whether the rs1360780 genotype was associated with other disease-associated variables than response to antidepressant treatment. No genotype-dependent differences in sex, age, age of onset or the diagnostic subgroup (unipolar, bipolar, psychotic, presence of melancholic features) of depressed patients were observed. There was, however, a significant difference in the number of previously experienced depressive episodes (F_{282,2} = 5.3; p = 0.005; ANOVA with age as covariate). Patients with the TT genotype of rs1360780 experienced over twice as many depressive episodes (see figure 5) before the index episode suggesting these patients have more lifetime depressive episodes in addition to their faster response to antidepressant drugs. Similar results were observed with rs4713916 and, rs3800373 (figures 6c, 7c)

### Example 4

### Association of rs1360780 genotype and FKBP51 protein expression in lymphocytes

Polymorphisms in *FKBP51* could influence GR sensitivity by several means. Polymorphisms in regulatory regions could lead to an altered expression level of FKBP51, varying the stoichiometric proportions of FKBP51 and FKBP52. Polymorphisms leading to amino acid exchanges may change the affinity of FKBP51, modifying its exchange rate against FKBP52. Both types of mutations could alter the rate of nuclear translocation of the activated GR. Glucocorticoids induce FKBP51 expression via an ultra-short negative feedback loop for GR activity²⁴. Polymorphisms decreasing the induction of FKBP51 expression by glucocorticoids could thus lead to a maintained GR sensitivity despite high circulating cortisol levels, resembling somewhat the neuroendocrine situation observed in T homozygotes. In order to investigate whether the genotype of rs1360780 was associated with different levels of FKBP51 protein, we quantified this protein in lymphocytes (T-cells) of healthy probands (Figures 8, 9). Lymphocytes of individuals homozygous for the T allele of the rs1360780 SNP, the genotype associated with a fast response to antidepressant drugs, have FKBP51 levels that are twice as high as those of individuals with the CC or CT genotypes (p < 0.05). Semi-quantitative analysis of Western blot results (see Figure 8) revealed that probands homozygous for the CC or CT allele of rs1360780 showed the lowest FKBP51 protein levels in lymphocytes (see Figures 8, 9). Higher levels of FKBP51 thus could be associated with an enhanced GR sensitivity that would prevent long-lasting dysregulations of the HPA-axis (see Figure 4) and in consequence a faster response to antidepressant drugs (see Figure 3a,b,c,d).

To identify the mechanism of increased FKBP51 protein levels, we quantified FKBP51 mRNA levels in peripheral blood monocytes (PBMCs) of these probands. There were no direct effects of the rs1360780 genotype on mRNA levels (figure 10), suggesting that not increased transcription but enhanced translation or protein stability contribute to the increased FKBP51 levels. We did, however, observe genotype-dependent differences in the positive correlation of plasma cortisol and FKBP51 mRNA levels. As noted above, glucocorticoids have been shown to induce FKBP51 expression *in vitro*²⁴. This finding could be confirmed in the overall sample *in vivo* (figure 11A), where plasma cortisol and FKBP51 mRNA levels showed a significant positive correlation (R = 0.262, p = 0.003). Intriguingly, when this analysis was repeated with the sample split in probands with the TT vs. the CC and CT genotypes of rs1360780, a much stronger correlation was observed in T homozygotes (R = 0.619; p = 0.003) that was significantly different (p = 0.012) from the correlation observed in the other genotypes (figures 11B and 11C).

### References

1. Holsboer, F. The Corticosteroid Receptor Hypothesis of Depression. Neuropsychopharmacology 23, 477-501 (2000).
2. Nemeroff, C. B. & Owens, M. J. Treatment of mood disorders. Nat Neurosci 5, 1068-70. (2002).
3. Holsboer, F. & Barden, N. Antidepressants and hypothalamic-pituitary-adrenocortical regulation. Endocrine Reviews 17, 187-205 (1996).
4. Nemeroff, C. B. The role of corticotropin-releasing factor in the pathogenesis of major depression. Pharmacopsychiatry 21, 76-82. (1988).
5. Nemeroff, C. B. et al. Elevated concentrations of CSF corticotropin-releasing factor-like immunoreactivity in depressed patients. Science 226, 1342-4. (1984).
6. Nemeroff, C. B., Owens, M. J., Bissette, G., Andom, A. C. & Stanley, M. Reduced corticotropin releasing factor binding sites in the frontal cortex of suicide victims. Arch Gen Psychiatry 45, 577-9. (1988).
7. Purba, J. S., Hoogendijk, W. J., Hofman, M. A. & Swaab, D. F. Increased number of vasopressin- and oxytocin-expressing neurons in the paraventricular nucleus of the hypothalamus in depression. Archives of General Psychiatry 53, 137-43 (1996).
8. Timpl, P. et al. Impaired stress response and reduced anxiety in mice lacking a functional corticotropin-releasing hormone receptor. Nature Genetics 19, 162-6 (1998).
9. Pariante, C. M. & Miller, A. H. Glucocorticoid receptors in major depression: relevance to pathophysiology and treatment. Biol Psychiatry 49, 391-404. (2001).
10. Reul, J. M., Stec, I., Soder, M. & Holsboer, F. Chronic treatment of rats with the antidepressant amitriptyline attenuates the activity of the hypothalamicpituitary-adrenocortical system. Endocrinology 133, 312-20. (1993).
11. Reul, J. M., Labeur, M. S., Grigoriadis, D. E., De Souza, E. B. & Holsboer, F. Hypothalamic-pituitary-adrenocortical axis changes in the rat after long-term treatment with the reversible monoamine oxidase-A inhibitor moclobemide. Neuroendocrinology 60, 509-19. (1994).
12. Keck, M. et al. The anxiolytic effect of the CRH1 receptor antagonist R121919 depends on innate emotionality in rats. European Journal of Neuroscience 13, 1-10 (2001).
13. Nickel, T. et al. Clinical and neurobiological effects of tianeptine and paroxetine in major depression. J Clin Psychopharmacol 23, 155-68. (2003).
14. Barden, N., Stec, I. S., Montkowski, A., Holsboer, F. & Reul, J. M. Endocrine profile and neuroendocrine challenge tests in transgenic mice expressing antisense RNA against the glucocorticoid receptor. Neuroendocrinology 66, 212-20 (1997).
15. Pratt, W. B. & Toft, D. O. Steroid receptor interactions with heat shock protein and immunophilin chaperones. Endocr Rev 18, 306-60. (1997).
16. Cheung, J. & Smith, D. F. Molecular chaperone interactions with steroid receptors: an update. Mol Endocrinol 14, 939-46. (2000).
17. Zobel AW, Nickel T, Sonntag A, Uhr M, Holsboer F, Ising M (2001): Cortisol response in the combined dexamethasone/CRH test as predictor of relapse in patients with remitted depression a prospective study. J Psychiatr Res 35:83-94.
18. Heuser, I., Yassouridis, A. & Holsboer, F. The combined dexamethasone/CRH test: a refined laboratory test for psychiatric disorders. Journal of Psychiatric Research 28, 341-56 (1994).
19. Schiene-Fischer, C. & Yu, C. Receptor accessory folding helper enzymes: the functional role of peptidyl prolyl cis/trans isomerases. FEBS Lett 495, 1-6. (2001).
20. Davies, T. H., Ning, Y. M. & Sanchez, E. R. A new first step in activation of steroid receptors: hormone-induced switching of FKBP51 and FKBP52 immunophilins. J Biol Chem 277, 4597-600. (2002).
21. Denny, W. B., Valentine, D. L., Reynolds, P. D., Smith, D. F. & Scammell, J. G. Squirrel monkey immunophilin FKBP51 is a potent inhibitor of glucocorticoid receptor binding. Endocrinology 141, 4107-13. (2000).
22. Reynolds, P. D. et al. Glucocorticoid-resistant B-lymphoblast cell line derived from the Bolivian squirrel monkey (Saimiri boliviensis boliviensis). Lab Anim Sci 48, 364-70. (1998).
23. Scammell, J. G., Denny, W. B., Valentine, D. L. & Smith, D. F. Overexpression of the FK506-binding immunophilin FKBP51 is the common cause of glucocorticoid resistance in three New World primates. Gen Comp Endocrinol 124, 152-65. (2001).
24. Vermeer, H., Hendriks-Stegeman, B. I., van der Burg, B., van Buul-Offers, S. C. & Jansen, M. Glucocorticoid-induced increase in lymphocytic FKBP51 messenger ribonucleic acid expression: a potential marker for glucocorticoid sensitivity, potency, and bioavailability. J Clin Endocrinol Metab 88, 277-84. (2003).
25. Zhang, K., and, Jin L., HaploBlockFinder: haplotype block analyses. Bioinformatics 19, 1300-1301 (2003)
26. Reynolds, P. D., Ruan, Y., Smith, D. F., and Scammel, J. G. Glucocorticoid resistance in the squirrel monkey is associated with overexpression of the immunophilin FKBP51. J Clin Endocrinol Metab 84, 663-669. (1999).
27. Lewontin, R. C. The interaction of selection and linkage. I. General considerations; heterotic models. Genetics 49, 49-67 (1964).
28. Nyholt DR. A simple correction for multiple testing for single-nucleotide polymorphisms in linkage disequilibrium with each other. Am J Hum Genet. 74:765-469 (2004).
29. Dudbridge F, Koeleman BP. Efficient computation of significance levels for multiple associations in large studies of correlated data, including genomewide association studies. Am J Hum Genet. 75:424-35 (2004).
30. Neale BM, Sham PC.The future of association studies: gene-based analysis and replication. Am J Hum Genet. 75:353-62 (2004).
31. Freedman ML, Reich D, Penney KL, McDonald GJ, Mignault AA, Patterson N, Gabriel SB, Topol EJ, Smoller JW, Pato CN, Pato MT, Petryshen TL, Kolonel LN, Lander ES, Sklar P, Henderson B, Hirschhom JN, Altshuler D.Assessing the impact of population stratification on genetic association studies. Nat Genet. 36:388-93 (2004).
32. Cordell, H.J., Clayton D.G. A unified stepwise regression procedure for evaluating the relative effects of polymorphisms within a gene using case/control or family data: application to HLA in type 1 diabetes. Am J Hum Genet. 70:124-41 (2002).
33.Stephens, M., Smith, N., and Donnelly, P. A new statistical method for haplotype reconstruction from population data. American Journal of Human Genetics 68: 978-989 (2001).
34. Kun Z., Li J. HaploBlockFinder: Haplotype Block Analyses. Bioinformatics 19: 1300-1301 (2003).
35. Lewontin R.C., On Measures of Gametic Disequilibrium, Genetics 120: 849-852(1988)
36. Abecasis G.R. and Cookson W.O.C. GOLD - Graphical Overview of Linkage Disequilibrium. Bioinformatics 16:182-3 (2000).

## Claims

1. A method of classifying an individual comprising analyzing the nucleic acid of a sample taken from said individual for nucleotide polymorphisms in the gene encoding FKBP51 or in a haplotype block comprising the gene encoding FKBP51 and optionally determining the expression level of FKBP51 in said sample, wherein said patient is suffering from depression and said classifying
(a) consists of or comprises predicting the response to therapy, wherein the haplotype block comprises at least one SNP selected from a first group wherein the respective alleles of each SNP are recognised by the sequences SEQ ID 102 or SEQ ID 103,
SEQ ID 104 or SEQ ID 105,
SEQ ID 106 or SEQ ID 107,
SEQ ID 108 or SEQ ID 109,
SEQ ID 110 or SEQ ID 111,
SEQ ID 112 or SEQ ID 113,
SEQ ID 114 or SEQ ID 115,
SEQ ID 13 or SEQ ID 14,
SEQ ID 120 or SEQ ID 121,
SEQ ID 122 or SEQ ID 123,
SEQ ID 124 or SEQ ID 125,
SEQ ID 126 or SEQ ID 127,
SEQ ID 128 or SEQ ID 129,
SEQ ID 17 or SEQ ID 18,
SEQ ID 130 or SEQ ID 131,
SEQ ID 132 or SEQ ID 133,
SEQ ID 134 or SEQ ID 135,
SEQ ID 136 or SEQ ID 137,
SEQ ID 138 or SEQ ID 139,
SEQ ID 140 or SEQ ID 141,
SEQ ID 142 or SEQ ID 143,
SEQ ID 144 or SEQ ID 145,
SEQ ID 19 or SEQ ID 20,
SEQ ID 146 or SEQ ID 147,
SEQ ID 148 or SEQ ID 149,
SEQ ID 150 or SEQ ID 151,
SEQ ID 152 or SEQ ID 153, and
SEQ ID 154 or SEQ ID 155; and/or
wherein the polymorphism is at least one SNP selected from said first group; or
(b) consists of or comprises predicting the predisposition for an elevated number of episodes of depression,
wherein the haplotype block comprises at least one SNP selected from a second group and wherein the respective alleles of each SNP are recognised by the sequences SEQ ID 13 or SEQ ID 14, SEQ ID 18 or SEQ ID 17, and SEQ ID 19 or SEQ ID 20; and / or
wherein the polymorphism is at least one SNP selected from said second group.

2. The method of claim 1, wherein said classifying comprises selecting said individual for a clinical trial.

3. The method according to claim 2, wherein said clinical trail is a clinical trial for an antidepressant drug.

4. The method of any one of claims 1 to 3, wherein the nucleic acid is gDNA.

5. The method of any one of claims 1 to 4, wherein analyzing the nucleic acid comprises:
(a) a primer extension assay;
(b) a differential hybridization assay; and/or
(c) an assay which detects allele-specific enzyme cleavage.

6. The method of any one of claims 1 to 5 further comprising, prior to analyzing, amplification of at least a portion of said gene or haplotype block.

7. The method of claims 6, wherein said amplification is effected by or said amplification is the polymerase chain reaction (PCR).

8. The method of claim 6 or 7, wherein said amplification reaction uses primers which hybridize specifically with a portion of said gene or haplotype block.

9. The method of claim 8, wherein the primers to be used for said amplification reaction have the sequence as set forth in SEQ ID NOs: 21 and 22; 23 and 24; 25 and 26; 27 and 28; 29 and 30; 1 and 2; 31 and 32; 33 and 34; 39 and 40; 41 and 42; 43 and 44; 45 and 46; 47 and 48; 5 and 6; 49 and 50; 51 and 52; 53 and 54; 55 and 56; 57 and 58; 59 and 60; 61 and 62; 63 and 64; 7 and 8; 65 and 66; 67 and 68; 69 and 70; 71 and 72; or 73 and 74.

10. The method of any one of claims 5 to 9, wherein said primer extension assay uses a primer which hybridizes specifically with a portion of said gene or haplotype block which is adjacent to a polymorphism.

11. The method of claim 10, wherein the primer to be used for said primer extension assay has the sequence as set forth in SEQ ID NO: 75, 76, 77, 78, 79, 9, 80, 81, 84, 85, 86, 87, 88, 11, 89, 90, 91, 92, 93, 94, 95, 96, 12, 97, 98, 99, 100, or 101.

12. The method of any one of claims 5 to 9, wherein said differential hybridization assay or said assay detecting allele-specific enzyme cleavage uses probes which hybridize specifically with a portion of said gene or haplotype block which comprises a polymorphism.

13. The method of claim 12, wherein said probes have the sequence as set forth in SEQ ID NO: 102 and 103; 104 and 105; 106 and 107; 108 and 109; 110 and 111; 13 and 14; 112 and 113; 114 and 115; 120 and 121; 122 and 123; 124 and 125; 126 and 127; 128 and 129; 17 and 18; 130 and 131; 132 and 133; 134 and 135; 136 and 137; 138 and 139; 140 and 141; 142 and 143; 144 and 145; 19 and 20; 146 and 147; 148 and 149; 150 and 151; 152 and 153; or 154 and 155.

14. The method of any one of claims 1 to 3, wherein the expression level to be determined is the mRNA expression level.

15. The method of any one of claims 1 to 3, wherein the expression level to be determined is the protein expression level.

## Patentansprüche

1. Verfahren zum Klassifizieren eines Individuums, umfassend das Analysieren der Nukleinsäure einer diesem Individuum entnommenen Probe im Hinblick auf Nukleotidpolymorphismen im FKBP51 kodierenden Gen oder in einem Haplotypblock umfassend das FKBP51 kodierende Gen, und gegebenenfalls Bestimmen des Expressionsspiegels von FKBP51 in der Probe, wobei der Patient unter Depression leidet und das Klassifizieren
(a) das Vorhersagen des Antwortverhaltens auf Therapie umfasst oder daraus besteht, wobei der Haplotypblock mindestens einen SNP umfasst, der aus einer ersten Gruppe ausgewählt ist, wobei die jeweiligen Allele jedes SNPs von den Sequenzen
SEQ ID 102 oder SEQ ID 103,
SEQ ID 104 oder SEQ ID 105,
SEQ ID 106 oder SEQ ID 107,
SEQ ID 108 oder SEQ ID 109,
SEQ ID 110 oder SEQ ID 111,
SEQ ID 112 oder SEQ ID 113,
SEQ ID 114 oder SEQ ID 115,
SEQ ID 13 oder SEQ ID 14,
SEQ ID 120 oder SEQ ID 121,
SEQ ID 122 oder SEQ ID 123,
SEQ ID 124 oder SEQ ID 125,
SEQ ID 126 oder SEQ ID 127,
SEQ ID 128 oder SEQ ID 129,
SEQ ID 17 oder SEQ ID 18,
SEQ ID 130 oder SEQ ID 131,
SEQ ID 132 oder SEQ ID 133,
SEQ ID 134 oder SEQ ID 135,
SEQ ID 136 oder SEQ ID 137,
SEQ ID 138 oder SEQ ID 139,
SEQ ID 140 oder SEQ ID 141,
SEQ ID 142 oder SEQ ID 143,
SEQ ID 144 oder SEQ ID 145,
SEQ ID 19 oder SEQ ID 20,
SEQ ID 146 oder SEQ ID 147,
SEQ ID 148 oder SEQ ID 149,
SEQ ID 150 oder SEQ ID 151,
SEQ ID 152 oder SEQ ID 153, und
SEQ ID 154 oder SEQ ID 155 erkannt werden; und/oder wobei der Polymorphismus mindestens ein SNP ist, der aus der ersten Gruppe ausgewählt ist; oder
(b) das Vorhersagen der Veranlagung für eine erhöhte Anzahl von Depressionsepisoden umfasst oder daraus besteht,
wobei der Haplotypblock mindestens einen SNP umfasst, der aus einer zweiten Gruppe ausgewählt ist, wobei die jeweiligen Allele jedes SNPs von den Sequenzen SEQ ID 13 oder SEQ ID 14, SEQ ID 18 oder SEQ ID 17, und SEQ ID 19 oder SEQ ID 20 erkannt werden; und/oder wobei der Polymorphismus mindestens ein SNP ist, der aus der zweiten Gruppe ausgewählt ist.

2. Verfahren nach Anspruch 1, wobei das Klassifizieren das Auswählen des Individuums für eine klinische Studie umfasst.

3. Verfahren nach Anspruch 2, wobei die klinische Studie eine klinische Studie eines Antidepressivums ist.

4. Verfahren nach einem der Ansprüche 1 bis 3, wobei die Nukleinsäure gDNA ist.

5. Verfahren nach einem der Ansprüche 1 bis 4, wobei das Analysieren der Nukleinsäure umfasst:
(a) einen Primer-Verlängerungstest;
(b) einen differenziellen Hybridisierungstest; und/oder
(c) einen Test, der allelspezifische Enzymspaltung nachweist.

6. Verfahren nach einem der Ansprüche 1 bis 5, ferner umfassend, vor dem Analysieren, die Amplifikation mindestens eines Teils des Gens oder Haplotypblocks.

7. Verfahren nach Anspruch 6, wobei das Amplifizieren mittels Polymerasekettenreaktion (PCR) durchgeführt wird oder die Amplifikation die Polymerasekettenreaktion (PCR) ist.

8. Verfahren nach Anspruch 6 oder 7, wobei die Amplifikationsreaktion Primer verwendet, die spezifisch mit einem Teil des Gens oder Haplotypblocks hybridisieren.

9. Verfahren nach Anspruch 8, wobei die Primer, die für die Amplifikationsreaktion zu verwenden sind, die Sequenz gemäß SEQ ID NOs: 21 und 22; 23 und 24; 25 und 26; 27 und 28; 29 und 30; 1 und 2; 31 und 32; 33 und 34; 39 und 40; 41 und 42; 43 und 44; 45 und 46; 47 und 48; 5 und 6; 49 und 50; 51 und 52; 53 und 54; 55 und 56; 57 und 58; 59 und 60; 61 und 62; 63 und 64; 7 und 8; 65 und 66; 67 und 68; 69 und 70; 71 und 72; oder 73 und 74 haben.

10. Verfahren nach einem der Ansprüche 5 bis 9, wobei der Primer-Verlängerungstest einen Primer verwendet, der spezifisch mit einem Teil des Gens oder Haplotypblocks hybridisiert, der einem Polymorphismus benachbart ist.

11. Verfahren nach Anspruch 10, wobei der für den Primer-Verlängerungstest zu verwendende Primer die Sequenz hat, wie sie in SEQ ID NO: 75, 76, 77, 78, 79, 9, 80, 81, 84, 85, 86, 87, 88, 11, 89, 90, 91, 92, 93, 94, 95, 96, 12, 97, 98, 99, 100 oder 101 dargestellt ist.

12. Verfahren nach einem der Ansprüche 5 bis 9, wobei der differenzielle Hybridisierungstest oder der Test, der allelspezifische Enzymspaltung nachweist, Sonden verwendet, die mit einem Teil des Gens oder Haplotypblocks spezifisch hybridisieren, der einen Polymorphismus umfasst.

13. Verfahren nach Anspruch 12, wobei die Sonden die Sequenz haben, wie sie in SEQ ID NO: 102 und 103; 104 und 105; 106 und 107; 108 und 109; 110 und 111; 13 und 14; 112 und 113; 114 und 115; 120 und 121; 122 und 123; 124 und 125; 126 und 127; 128 und 129; 17 und 18; 130 und 131; 132 und 133; 134 und 135; 136 und 137; 138 und 139; 140 und 141; 142 und 143; 144 und 145; 19 und 20; 146 und 147; 148 und 149; 150 und 151; 152 und 153; oder 154 und 155 dargestellt ist.

14. Verfahren nach einem der Ansprüche 1 bis 3, wobei der zu bestimmende Expressionsspiegel der mRNA-Expressionsspiegel ist.

15. Verfahren nach einem der Ansprüche 1 bis 3, wobei der zu bestimmende Expressionsspiegel der Proteinexpressionsspiegel ist.

## Revendications

1. Procédé de classement d'un individu comprenant l'analyse de l'acide nucléique d'un échantillon prélevé à partir dudit individu pour des polymorphismes nucléotidiques dans le gène codant FKBP51 ou dans un bloc haplotypique comprenant le gène codant FKBP51 et éventuellement la détermination du niveau d'expression de FKBP51 dans ledit échantillon, où ledit patient souffre de dépression et ledit classement
(a) consiste en ou comprend la prédiction de la réponse à une thérapie, où le bloc haplotypique comprend au moins un SNP choisi dans un premier groupe dans lequel les allèles respectifs de chaque SNP sont reconnus par les séquences
SEQ ID : 102 ou SEQ ID : 103,
SEQ ID : 104 ou SEQ ID : 105,
SEQ ID : 106 ou SEQ ID : 107,
SEQ ID : 108 ou SEQ ID : 109,
SEQ ID : 110 ou SEQ ID : 111,
SEQ ID : 112 ou SEQ ID : 113,
SEQ ID : 114 ou SEQ ID : 115,
SEQ ID : 13 ou SEQ ID : 14,
SEQ ID : 120 ou SEQ ID : 121,
SEQ ID : 122 ou SEQ ID : 123,
SEQ ID : 124 ou SEQ ID : 125,
SEQ ID : 126 ou SEQ ID : 127,
SEQ ID : 128 ou SEQ ID : 129,
SEQ ID : 17 ou SEQ ID : 18,
SEQ ID : 130 ou SEQ ID : 131,
SEQ ID : 132 ou SEQ ID : 133,
SEQ ID : 134 ou SEQ ID : 135,
SEQ ID : 136 ou SEQ ID : 137,
SEQ ID : 138 ou SEQ ID : 139,
SEQ ID : 140 ou SEQ ID : 141,
SEQ ID : 142 ou SEQ ID : 143,
SEQ ID : 144 ou SEQ ID : 145,
SEQ ID : 19 ou SEQ ID : 20,
SEQ ID : 146 ou SEQ ID : 147,
SEQ ID : 148 ou SEQ ID : 149,
SEQ ID : 150 ou SEQ ID : 151,
SEQ ID: 152 ou SEO ID : 153, et
SEQ ID : 154 ou SEQ ID : 155 ; et/ou
où le polymorphisme est au moins un SNP choisi dans ledit premier groupe ; ou
(b) consiste en ou comprend la prédiction de la prédisposition à un nombre élevé d'épisodes de dépression,
où le bloc haplotypique comprend au moins un SNP choisi dans un second groupe et dans lequel les allèles respectifs de chaque SNP sont reconnus par les séquences SEQ ID : 13 ou SEQ ID : 14, SEQ ID : 18 ou SEQ ID : 17, et SEQ ID : 19 ou SEQ ID : 20 ; et/ou où le polymorphisme est au moins un SNP choisi dans ledit second groupe.

2. Procédé selon la revendication 1, dans lequel ledit classement comprend la sélection dudit individu pour une étude clinique.

3. Procédé selon la revendication 2, dans lequel ladite étude clinique est une étude clinique pour un médicament antidépresseur.

4. Procédé selon l'une quelconque des revendications 1 à 3, dans lequel l'acide nucléique est de l'ADNg.

5. Procédé selon l'une quelconque des revendications 1 à 4, dans lequel l'analyse de l'acide nucléique comprend :
(a) une analyse d'extension d'amorce ;
(b) une analyse d'hybridation différentielle ; et/ou
(c) une analyse qui détecte un clivage enzymatique spécifique d'un allèle.

6. Procédé selon l'une quelconque des revendications 1 à 5, comprenant en outre, avant l'analyse, l'amplification d'au moins une partie dudit gène ou bloc haplotypique.

7. Procédé selon la revendication 6, dans lequel ladite amplification est effectuée par ou ladite amplification est la réaction en chaîne par polymérase (PCR).

8. Procédé selon la revendication 6 ou 7, dans lequel ladite réaction d'amplification utilise des amorces qui s'hybrident spécifiquement à une partie dudit gène ou bloc haplotypique.

9. Procédé selon la revendication 8, dans lequel les amorces à utiliser pour ladite réaction d'amplification ont une séquence telle que présentée dans SEQ ID NO : 21 et 22 ; 23 et 24 ; 25 et 26 ; 27 et 28 ; 29 et 30 ; 1 et 2 ; 31 et 32 ; 33 et 34 ; 39 et 40 ; 41 et 42 ; 43 et 44 ; 45 et 46 ; 47 et 48 ; 5 et 6 ; 49 et 50 ; 51 et 52 ; 53 et 54 ; 55 et 56 ; 57 et 58 ; 59 et 60 ; 61 et 62 ; 63 et 64 ; 7 et 8 ; 65 et 66 ; 67 et 68 ; 69 et 70 ; 71 et 72 ; ou 73 et 74.

10. Procédé selon l'une quelconque des revendications 5 à 9, dans lequel ladite analyse d'extension d'amorce utilise une amorce qui s'hybride spécifiquement à une partie dudit gène ou bloc haplotypique qui est adjacent à un polymorphisme.

11. Procédé selon la revendication 10, dans lequel l'amorce à utiliser pour ladite analyse d'extension d'amorce a la séquence telle que présentée dans SEQ ID NO : 75, 76, 77, 78, 79, 9, 80, 81, 84, 85, 86, 87, 88, 11, 89, 90, 91, 92, 93, 94, 95, 96, 12, 97, 98, 99, 100 ou 101.

12. Procédé selon l'une quelconque des revendications 5 à 9, dans lequel ladite analyse d'hybridation différentielle ou ladite analyse de détection d'un clivage enzymatique spécifique d'allèle utilise des sondes qui s'hybrident spécifiquement à une partie dudit gène ou bloc haplotypique qui comprend un polymorphisme.

13. Procédé selon la revendication 12, dans lequel lesdites sondes ont la séquence telle que présentée dans SEQ ID NO : 102 et 103 ; 104 et 105 ; 106 et 107 ; 108 et 109 ; 110 et 111 ; 13 et 14 ; 112 et 113 ; 114 et 115 ; 120 et 121 ; 122 et 123 ; 124 et 125 ; 126 et 127 ; 128 et 129 ; 17 et 18 ; 130 et 131 ; 132 et 133 ; 134 et 135 ; 136 et 137 ; 138 et 139 ; 140 et 141 ; 142 et 143 ; 144 et 145 ; 19 et 20 ; 146 et 147 ; 148 et 149 ; 150 et 151 ; 152 et 153 ; ou 154 et 155.

14. Procédé selon l'une quelconque des revendications 1 à 3, dans lequel le niveau d'expression à déterminer est le niveau d'expression de l'ARNm.

15. Procédé selon l'une quelconque des revendications 1 à 3, dans lequel le niveau d'expression à déterminer est le niveau d'expression de la protéine.
